# EUROPÄISCHE PATENTSCHRIFT

(11) **EP 2 132 193 B1**
(45) Veröffentlichungstag und Bekanntmachung des Hinweises auf die Patenterteilung: **24.10.2012**
(21) Anmeldenummer: 08718234.1
(22) Anmeldetag: 26.03.2008
(51) Int. Cl.: C07D 401/06, C07D 401/14, C07D 417/14, C07D 419/14, C07D 495/04, A61K 31/42, A61P 7/02

(54) **SUBSTITUIERTE PYRROLIDINAMIDE, DEREN HERSTELLUNG UND DEREN VERWENDUNG ALS ARZNEIMITTEL**
SUBSTITUTED PYRROLIDINE AMIDES, THE PRODUCTION THEREOF, AND THE USE THEREOF AS MEDICATIONS
PYRROLIDINAMIDES SUBSTITUÉS, LEUR FABRICATION ET LEUR UTILISATION EN TANT QUE MÉDICAMENT

(30) Priorität: 27.03.2007 EP 07105053
(43) Veröffentlichungstag der Anmeldung: 16.12.2009
(73) Patentinhaber: Boehringer Ingelheim International GmbH, 55216 Ingelheim am Rhein (DE)
(72) Erfinder: GERLACH, Kai, 55216 Ingelheim am Rhein (DE); PRIEPKE, Henning, 55216 Ingelheim am Rhein (DE); WIENEN, Wolfgang, 55216 Ingelheim am Rhein (DE); SCHULER-METZ, Annette, 55216 Ingelheim am Rhein (DE); DAHMANN, Georg, 55216 Ingelheim am Rhein (DE); NAR, Herbert, 55216 Ingelheim am Rhein (DE)
(74) Vertreter: Simon, Elke Anna Maria
(86) Internationale Anmeldenummer: PCT/EP2008/053568
(87) Internationale Veröffentlichungsnummer: WO 2008/116881

(56) Entgegenhaltungen:
- WO-A-2004/017908
- WO-A-2005/032472

## Beschreibung

WO 2004/017908 beschreibt substituierte Pyrazole-Pyrimidine Verbindungen un deren Verwendung als Kalzium-Rezeptor-Modufatoren.

WO 2005/032472 beschreibt Pyrrolidin und Piperidin-Derivate als Faktor Xa Hemmer.

Gegenstand der vorliegenden Erfindung sind neue substituierte Pyrrolidinamide der allgemeinen Formel (I) 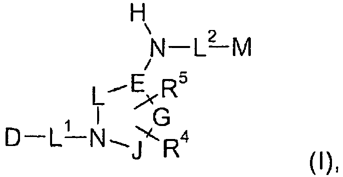 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
D ein substituiertes bicyclisches Ringsystem der Formel (IIa) , (IIb) oder (IIc) - 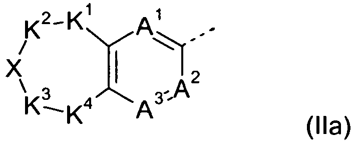 oder 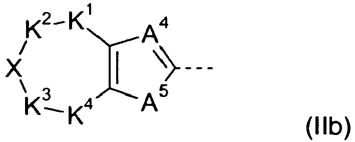 oder 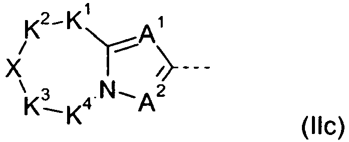 darstellt, in dem
K¹ und K⁴
jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³
jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c}
jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (IIa) oder (IIb) oder (IIc) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonylgruppe substituiert sein können, oder durch eine Hydroxy-, C₁₋₅-Alkyloxy-, Amino-, C₁₋₅-Alkylamino-, C₁₋₅-Dialkylamino- oder C₄₋₇Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
- A¹: entweder N oder CR¹⁰ bedeutet,
- A²: entweder N oder CR¹¹ bedeutet,
- A³: entweder N oder CR¹² bedeutet,
- A⁴: entweder N oder CR¹² bedeutet,
- A⁵: NH, Schwefel oder Sauerstoff bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Phenyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
-L-E-G-J- eine -C-C-C-C-Gruppe bedeutet, die durch R⁴ und R⁵ substituiert sein kann, und
L¹ eine -C(O)-Gruppe bedeutet, und
R⁴ ein Wasserstoffatom oder
eine geradkettige oder verzweigte C₁₋₆-Alkyl-, C₂₋₆-Alkenyl- oder
C₂₋₆-Alkinylgruppe bedeutet, wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkylaminocarbonyloxy-, Di-(C₁₋₅-alkyl)-aminocarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₃-Alkyloxy-C₁₋₂alkylcarbonylamino-, C₁₋₃-Alkyloxycarbonylamino-, C₁₋₃-Alkylaminocarbonylamino-, C₁₋₅-Alkylsulfonylamino-, N-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet,
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₅-Alkyloxycarbonyl-, Carboxy-, Methyl-, Hydroxy-, Methoxy- oder Aminosubstituiert sein können, und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O- Bindung bilden, ausgeschlossen ist, und
R⁵ ein Wasserstoffatom, eine C₁₋₅Alkyl-, C₂₋₅Alkenyl- oder C₂₋₅Alkinyl- oder eine Phenyl-C₀₋₅Alkylgruppe bedeutet, wobei die Alkylgruppe durch eine Hydroxy-, Methoxy-, Hydroxycarbonyl- oder C₁₋₅Alkoxycarbonyl-gruppe substituiert sein kann,
oder sofern R⁵ mit G verknüpft ist auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O Gruppe, oder eine -CF₂ Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder
C₄₋₇-Cycloalkyleniminocarbonylgruppe substituiert sein können, mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O- Bindung bilden,
ausgeschlossen ist, und
L² eine -C(O)-Gruppe bedeutet, und
M einen gegebenenfalls durch R² und R³ substituierten Phenyl-, Pyridyl-, Thienyl- oder Furyl-Ring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R³ ein Wasserstoff-, Fluor-, Chlor-, Brom- oder Jod-atom oder eine Hydroxy-, Methoxy-, Trifluormethoxy-gruppe, oder eine gegebenenfalls durch Fluoratome substituierte C₁₋₃-Alkyl-gruppe, oder eine Cyano-, Amino- oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und lod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.
Beispiele für monocyclische Heteroarylgruppen sind die Pyridyl-, N-Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thienyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[c]furanyl-, Benzothiophenyl-, Benzo[c]thiophenyl-, Benzothiazolyl-, Benzo[c]isothiazolyl-, Benzo[d]isothiazolyl-, Benzooxazolyl-, Benzo[c]isoxazolyl-, Benzo[d]isoxazolyl-, Benzo[1,2,5]oxadiazolyl-, Benzo[1,2,5]thiadiazolyl-, Benzo[1,2,3]thiadiazolyl-, Benzo[d][1,2,3]triazinyl-, Benzo[1,2,4]triazinyl-, Benzotriazolyl-, Cinnolinyl-, Chinolinyl-, N-Oxy-chinolinyl-, Isochinolinyl-, Chinazolinyl-, N-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, n-Butyl-, sec-Butyl-, tert-Butyl-, 1-Pentyl-, 2-Pentyl-, 3-Pentyl-, neo-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-, n-Butyloxy-, sec-Butyloxy-, tert-Butyloxy-, 1-Pentyloxy-, 2-Pentyloxy-, 3-Pentyloxy- oder neo-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I) in denen D, E, G, J, L, L¹, L² und M wie in Ausführungsform 1 beschrieben definiert sind, und in der
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, C₁₋₅-Alkylaminocarbonyloxy-, Di-(C₁₋₅-alkyl)-aminocarbonyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₃-Alkyloxy-C₁₋₂alkylcarbonylamino-, C₁₋₃-Alkyloxycarbonylamino-, C₁₋₃-Alkylaminocarbonylamino-, C₁₋₅-Alkylsulfonylamino-, N-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅-Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkyl-, Allyl-, Propargyl- oder Benzylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
deren Tautomere, deren Enantiomere, deren Diastereomere, deren Gemische und deren Salze.

Eine 3. Ausführungsform der vorliegenden Erfindung umfasst diejenigen Verbindungen der allgemeinen Formel (I), in denen E, G, J, L, L¹, L², M, R⁴ und R⁵ wie in den Ausführungsformen 1 oder 2 beschrieben definiert sind, und in der
D ein substituiertes bicyclisches Ringsystem der Formel (IIa) oder (IIb) 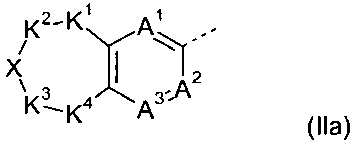 oder 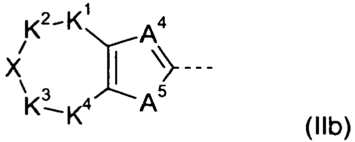 darstellt, in dem
K¹ und K⁴
jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe, oder
zwei Reste R^{7b}/R^{7c} zusammen mit dem Ringkohlenstoffatom einen 3-gliedrigen Carbocyclus bilden können,
mit der Maßgabe, dass K¹ und K⁴ gleichzeitig eine Bindung bedeuten, ausgeschlossen ist, und
K² und K³
jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c}
jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können und
insgesamt in den Formeln (IIa) oder (IIb) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine -CF₂-
oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
- A¹: entweder N oder CR¹⁰ bedeutet,
- A²: entweder N oder CR¹¹ bedeutet,
- A³: entweder N oder CR¹² bedeutet,
- A⁴: entweder N oder CR¹² bedeutet,
- A⁵: NH, Schwefel oder Sauerstoff bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten,
deren Tautomere, deren Enantiomere, deren Diastereomere, deren Gemische und deren Salze.

Eine 4. Ausführungsform der vorliegenden Erfindung umfasst diejenigen Verbindungen der Ausführungsformen 1, 2 oder 3, in denen
- X: eine NR¹-Gruppe bedeutet, in der
- R¹: ein Wasserstoffatom oder eine C₁₋₅-Alkyl-, Allyl- oder Cyclopropylgruppe bedeutet, und
- A¹: CR¹⁰ bedeutet,
- A²: CR¹¹ bedeutet,
- A³: CR¹² bedeutet,
- A⁴: entweder N oder CR¹² bedeutet,
- A⁵: Schwefel bedeutet,
- wobei R¹⁰, R¹¹ und R¹²: jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

Eine 5. Ausführungsform der vorliegenden Erfindung umfasst diejenigen Verbindungen der Ausführungsformen 1, 2, 3 oder 4, in denen
D ein substituiertes bicyclisches Ringsystem der Formel 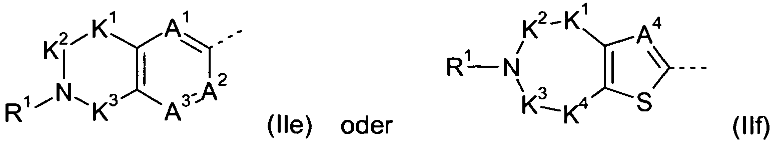 darstellt, in dem
K¹ eine -CH₂-, -CHR^{7a}-, oder eine -CR^{7b}R^{7c}- Gruppe bedeutet, und
K² und K³
jeweils unabhängig voneinander eine -CH₂-, -CHR^{8a}-, oder eine - CR^{8b}R^{8c}- Gruppe bedeuten, wobei
R^{8a}/R^{8b}/R^{8c}
jeweils unabhängig voneinander eine C₁₋₅-Alkylgruppe bedeutet, und
K⁴ eine Bindung, eine -CH₂-, -CHR^{7a}-, -CR^{7b}R^{7c}- oder eine -C(O)-Gruppe bedeutet,
wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet,
wobei nicht gleichzeitig beide Reste R^{7b}/R^{7c} über ein Sauerstoffatom an das Ringkohlenstoffatom gebunden sein können, und
insgesamt in den Formeln (IIe) oder (IIf) maximal vier Reste ausgewählt aus R^{7a}, R^{7b}, R^{7c}, R^{8a}, R^{8b} und R^{8c} vorhanden sein dürfen, und
R¹ ein Wasserstoffatom oder eine C₁₋₃-Alkyl-, Allyl- oder Cyclopropylgruppe bedeutet, und in denen
- A¹: CR¹⁰ bedeutet,
- A²: CR¹¹ bedeutet,
- A³: CR¹² bedeutet,
- A⁴: entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine -C-C-C-C-Gruppe bedeutet, die durch R⁴ und R⁵ substituiert sein kann, und
R⁴ ein Wasserstoffatom oder
eine geradkettige oder verzweigte C₁₋₃-Alkylgruppe bedeutet,
wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, C₁₋₅-Alkylaminocarbonyloxy-, Di-(C₁₋₅-alkyl)-aminocarbonyloxy-, Carboxy-, C₁₋₅-Alkyloxycarbonyl-, C₁₋₃-Alkyloxy-C₁₋₂alkylcarbonylamino-, C₁₋₃-Alkyloxycarbonylamino-, C₁₋₃-Alkylaminocarbonylamino-, C₁₋₅-Alkylcarbonylamino-, C₁₋₅-Alkylsulfonylamino-gruppe substituiert sein können, oder
sofern R⁴ mit G verknüpft ist, auch ein Fluoratom oder eine Hydroxy-, Methoxy-, C₃₋₅-Alkenyl-oxy-, C₂₋₅-Alkyl-oxy-, C₃₋₆-Cycloalkyl-oxy-, Benzyloxy-, C₁₋₅-Alkylaminocarbonyloxy-, Di(C₁₋₅-alkyl)aminocarbonyloxy- oder eine C₄₋₇-Cycloalkyleniminocarbonyloxygruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
R⁵ ein Wasserstoffatom oder eine C₁₋₅Alkyl-, Allyl-, Benzyl- oder Phenylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
deren Tautomere, deren Enantiomere, deren Diastereomere, deren Gemische und deren Salze.

Eine 6. Ausführungsform der vorliegenden Erfindung umfasst diejenigen Verbindungen der Ausführungsformen 1, 2, 3, 4 oder 5, in denen
D ein substituiertes bicyclisches Ringsystem der Formel 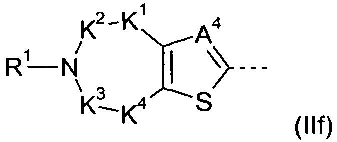 darstellt, in dem
K¹ eine -CH₂-, -CHR^{7a}-, oder eine -CR^{7b}R^{7c}- Gruppe bedeutet, und
K² und K³
jeweils unabhängig voneinander eine -CH₂-, -CHR^{8a}-, oder eine - CR^{8b}R^{8c}- Gruppe bedeuten, wobei
R^{8a}/R^{8b}/R^{8c}
jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe bedeutet,
wobei
R^{7a} eine C₁₋₅-Alkylgruppe bedeutet, und
R^{7b}/R^{7c} jeweils unabhängig voneinander eine Hydroxy-, C₁₋₅-Alkyloxy- oder eine C₁₋₅-Alkylgruppe bedeutet,
wobei nicht gleichzeitig beide Reste R^{7b}/R^{7c} über ein Sauerstoffatom an das Ringkohlenstoffatom gebunden sein können,
und
insgesamt in der Formel (IIf) maximal vier Reste ausgewählt aus R^{7a}, R^{7b}, R^{7c}, R^{8a}, R^{8b} und R^{8c} vorhanden sein dürfen, und
- R¹: ein Wasserstoffatom oder eine C₁₋₃-Alkyl- oder Cyclopropylgruppe bedeutet, und in denen
- A¹: CR¹⁰ bedeutet,
- A²: CR¹¹ bedeutet,
- A³: CR¹² bedeutet,
- A⁴: entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

Eine 7. Ausführungsform der vorliegenden Erfindung umfasst diejenigen Verbindungen der Ausführungsformen 1, 2, 3, 4, 5 oder 6, in denen
M einen Thiophen-2-yl-Ring der Formel 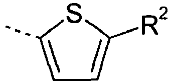 bedeutet, in dem
R² ein Chlor-, Bromatom oder eine Ethinyl-Gruppe darstellt,
deren Tautomere, deren Enantiomere, deren Diastereomere, deren Gemische und deren Salze.

Eine 8. Ausführungsform der vorliegenden Erfindung umfaßt die folgenden Verbindungen:
(3R,5S)-5-Chlor-thiophen-2-carbonsäure-[4-hydroxymethyl-1-(6-methyl-4,5,6,7-tetrahydro-thieno[2,3-c]pyridin-2-carbonyl)-pyrrolidin-3-yl]-amid
(3R,5S)-5-Chlor-thiophen-2-carbonsäure-[5-methoxymethyl-1-(6-methyl-5,6,7,8-tetrahydro-4H-thieno[2,3-d]azepin-2-carbonyl)-pyrrolidin-3-yl]-amid
(2S,4R)-4-[(5-Chlor-thiophen-2-carbonyl)-amino]-1-(6-methyl-5,6,7,8-tetrahydro-4H-thieno[2,3-d]azepin-2-carbonyl)-pyrrolidin-2-carbonsäure-methylester
(2S,4R)-4-[(5-Chlor-thiophen-2-carbonyl)-amino]-1-(4,6-dimethyl-5,6,7,8-tetrahydro-4H-thieno[2,3-d]azepin-2-carbonyl)-pyrrolidin-2-carbonsäure-methylester
(2S,4R)-5-Chlor-thiophen-2-carbonsäure-[5-hydroxymethyl-1-(6-methyl-5,6,7,8-tetrahydro-4H-thieno[2,3-d]azepin-2-carbonyl)-pyrrolidin-3-yl]-amid
(3*R*,5*S*)-5-Chlor-thiophen-2-carbonsäure-{1-((4*SR*)-4-methoxy-6-methyl-5,6,7,8-tetrahydro-4H-thieno[2,3-d]azepin-2-carbonyl]-4-methoxy-pyrrolidin-3-yl}-amid
(2S,4R)-4-[(5-Chlor-thiophen-2-carbonyl)-amino]-1-(6-methyl-5,6,7,8-tetraydro-4H-thieno[2,3-d]azepin-2-carbonyl)-pyrrolidin-2-carbonsäure-methylamid
(2S,4R)-Ethyl-carbaminsäure-4-[(5-chlor-thiophen-2-carbonyl)-amino]-1-(6-methyl-5,6,7,8-tetrahydro-4H-thieno[2,3-d]azepin-2-carbonyl)-pyrrolidin-2-yl-methylester
(3R,5S)- 5-Chlor-thiophen-2-carbonsäure-[5-(methansulfonylamino-methyl)-1-(6-methyl-5,6,7,8-tetrahydro-4H-thieno[2.3-d]azepin-2-carbonyl)-pyrrolidin-3-yl]-ami
(3*R*,5*S*)- 5-Chlor-thiophen-2-carbonsäure-[5-[(3-ethyl-ureido)-methyl]-1-(6-methyl-5,6,7,8-tetrahydro-4H-thieno[2.3-d]azepin-2-carbonyl)-pyrrolidin-3-yl]-amid
(2*S*,4*R*)- 4-[(5-Chlor-thiophen-2-carbonyl)-amino]-1-(6-methyl-5,6,7,8-tetrahydro-4H-thieno[2,3-d]azepin-2-carbonyl)-pyrrolidin-2-ylmethyl]-carbaminsäure-methylester
(3R,5S)-5-Chlor-thiophen-2-carbonsäure-[5-[(2-methoxy-acetylamino)-methyl]-1-(6-methyl-5,6,7,8-tetrahydro-4H-thieno{[2,3-d]azepin-2-carbonyl}-pyrrolidin-3-yl)-amid
(2S,4R)-4-[(5-Chlor-thiophen-2-carbonyl)-amino]-1-((S)-4-methoxy-6-methyl-5,6,7,8-tetrahydro-4H-thieno[2,3-d]azepin-2-carbonyl)-pyrrolidin-2-carbonsäuremethylamid
(3*R*,5*S*)-5-Chlor-thiophen-2-carbonsäure-[5-methoxymethyl-1-[(4S)-6-methyl-5,6,7,8-tetrahydro-4H-4-methoxy-thieno[2,3-d]azepin-2-carbonyl]-pyrrolidin-3-yl]-amid
(3*R*,5*S*)-5-Chlor-thiophen-2-carbonsäure-[5-(acetylamino-methyl)-1-(6-methyl-5,6,7,8-tetrahydro-4H-thieno[2,3-d]azepin-2-carbonyl)-pyrrolidin-3-yl]-amid

Erfindungsgemäß erhält man die Verbindungen der allgemeinen Formel (I) nach an sich bekannten Verfahren, beispielsweise nach folgenden Verfahren:
(a) Die Herstellung einer Verbindung der allgemeinen Formel (III) 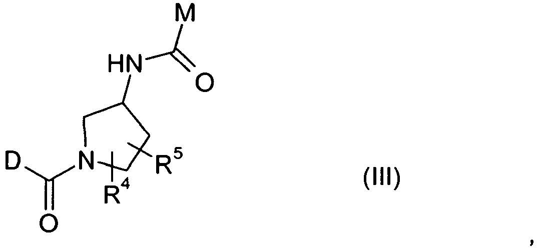 in der D, M und R¹ bis R⁵ wie in Ausführungsform 1 erwähnt definiert sind,
   und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein kann, und deren Schutzgruppen in
   Literatur nach bekannter Methode abgespalten werden können, wird in den Ausführungsbeispielen beschrieben oder kann beispielsweise nach einem der folgenden Formelschemata 1 und 2 oder in Analogie zu den Syntheseverfahren, die in WO2002/14308 oder WO2006/114402 beschrieben sind, durchgeführt werden. 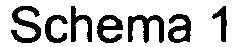 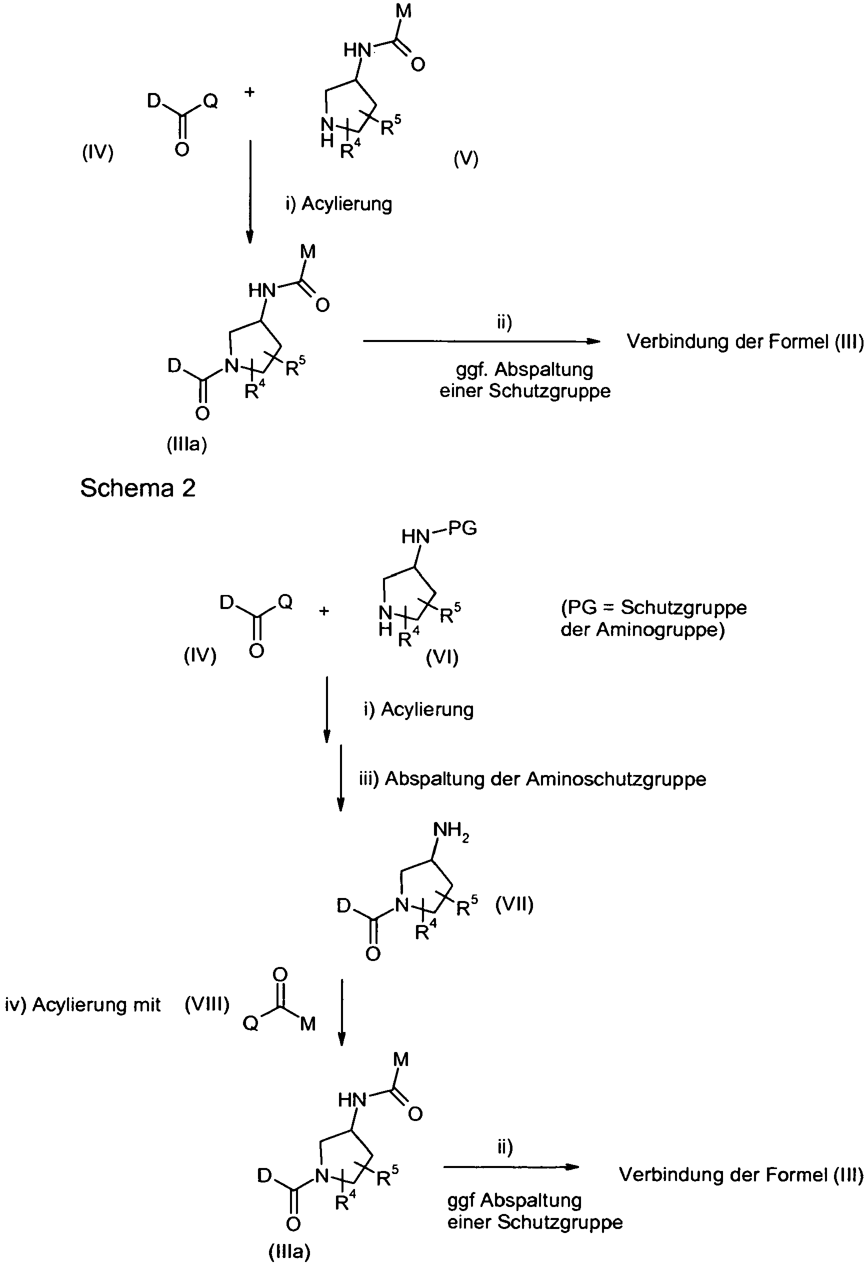 wobei
   Q eine Austrittsgruppe oder eine *in-situ* in eine Austrittsgruppe überführbare Gruppe wie beispielsweise ein Halogenatom, eine Hydroxy-, C₁₋₄-Alkyloxy-, Alkyloxycarbonyloxy-, Pentafluorphenyloxy-, 4-Nitrophenyloxy-, eine Trichlormethyl- oder Acyloxy-gruppe darstellt, und PG eine in Literatur bekannte Schutzgruppe der Aminofunktion wie beispielsweise eine tert.-Butoxycarbonyl-, Benzyloxycarbonyl- oder eine Trifluoracetyl-gruppe darstellt.
   Die in Schema 1 und 2 beschriebenen Reaktionsstufen i) -iv) können auf die in den Beispielen beschriebene Weise oder nach in Literatur bekannter Bedingungen beispielsweise wie folgt durchgeführt werden:
   i) Acylierung eines Amins (V) mit einer gegebenenfalls aktivierten Carbonsäure (IV) oder (VIII) :
      Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Dimethylsulfoxid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 100°C, durchgeführt.
      Die Acylierung kann jedoch auch mit der freien Säure gegebenenfalls in Gegenwart eines die Säure aktivierenden Mittels oder eines wasserentziehenden Mittels, beispielsweise in Gegenwart von Ethyl-1-ethoxy-1,2-dihydrochinolin-1-carboxylat, Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid, Propanphosphonsäurecycloanhydrid, *N,N'*-Dicyclohexylcarbodiimid, *N,N*'-Dicyclohexylcarbodiimid/Camphersulfonsäure, *N,N'*-Dicyclohexylcarbodiimid/*N*-Hydroxysuccinimid oder 1-Hydroxy-benztriazol, *N,N*'-Carbonyldiimidazol, *O*-(Benzotriazol-1-yl)-*N,N,N',N'*-tetramethyl-uroniumtetrafluorborat/N-Methylmorpholin, *O*-(Benzotriazol-1-yl)-*N,N,N',N'*-tetramethyl-uroniumtetrafluorborat/N-Ethyldiisopropylamin, *O*-(7-Azabenzotriazol-1-yl)-*N,N,N',N'-*tetramethyluronium-hexafluorphosphat/*N*-Methylmorpholin, *O*-Pentafluorophenyl-*N,N,N',N'*-tetramethyluronium-hexafluorophosphat/Triethylamin, *N,N'*-Thionyldiimidazol oder Triphenylphosphin/Tetrachlorkohlenstoff, gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
      Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995, oder auch im Houben-Weyl Ergänzungsband 22, Thieme Verlag, 2003 und der dort zitierten Literatur beschrieben.
   ii) bzw. iii) Abspaltung einer Schutzgruppe
      Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.
      Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Tetrahydrofuran, Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.
      Die Abspaltung einer Schutzgruppe kann aber auch nach den in T.W. Greene, P.G.M. Wuts in "Protective Groups in Organic Synthesis", Wiley, 1991 und 1999, beschriebenen Verfahren durchgeführt werden.
(b) Die Bausteine der allgemeinen Formel 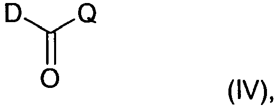 wobei Q eine Hydroxy- oder Alkyloxy-Gruppe darstellt, und in denen D wie in Ausführungsform 1 erwähnt definiert sind, und die gegebenenfalls an vorhandenen Amino-, Hydroxy-, Carboxy- oder Thiol-gruppen durch gängige Schutzgruppen, wie beispielsweise denen in T.W. Greene, P.G.M. Wuts in "Protective Groups in Organic Synthesis", Wiley, 1991 und 1999, beschriebenen geschützt sein können, und deren Schutzgruppen in Literatur nach bekannter Methode im Verlauf der Synthesesequenz zu Verbindungen der Formel (I) abgespalten werden können,
   sind aus der Literatur bekannt, oder deren Synthese wird in den Ausführungsbeispielen beschrieben, oder sie können beispielsweise nach in Literatur bekannter Syntheseverfahren oder in Analogie zu in Literatur bekannten Syntheseverfahren wie beispielsweise in DE3105858, JP04046139 bzw. in N. Haginoya et al. J. Med. Chem. 2004, 47(21), 5167, S. Komoriya et al. Bioorg. Med. Chem. 2006, 14, 1309, Ortar et al. Tetrahedron Lett. 1986, 3931 oder in J.M. Herbert et al., Tetrahedron. Lett 1998, 2421, beschrieben, hergestellt werden.
   Beispielsweise kann eine Verbindung der allgemeinen Formel (IV), in der D wie in Ausführungsform 1 erwähnt definiert sind, durch palladiumvermittelte Carboxylierung in Alkoholen oder Wasser aus Verbindungen der allgemeinen Formeln (X) oder (Xa) 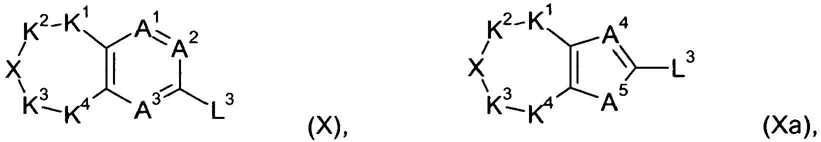 wobei
   L³ eine Austrittsgruppe oder eine *in-situ* in eine Austrittsgruppe überführbare Gruppe wie beispielsweise ein Halogenatom oder ein Trifluormethansulfonat darstellt, und
   in der A¹, A², A³, A⁴, A⁵, K¹, K², K³, K⁴ und X wie in Ausführungsform 1 erwähnt definiert sind, wie folgt hergestellt werden.
   Die Einführung einer Estergruppe aus Verbindungen der allgemeinen Formeln (X) oder (Xa) wird beispielsweise zweckmäßigerweise mit einem Alkohol durch katalytische Carbonylierung mit Kohlenmonoxid, beispielsweise unter einem Druck zwischen 0.5 und 100 bar, vorzugsweise jedoch zwischen 1 und 50 bar, zweckmäßigerweise in Gegenwart eines Katalysators wie beispielsweise Palladium(II)acetat,Tetrakis(triphenylphosphin)palladium(0) oder Dichlorbis(triphenylphosphin)palladium(II), zweckmäßigerweise in einem Lösungsmittel oder Lösungsmittelgemisch wie Wasser, Methanol, Ethanol, Isopropanol, Butanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, beispielsweise bei Temperaturen zwischen -30 und 250°C, vorzugsweise jedoch zwischen 0 und 150°C, durchgeführt.
(c) Die Bausteine der allgemeinen Formel 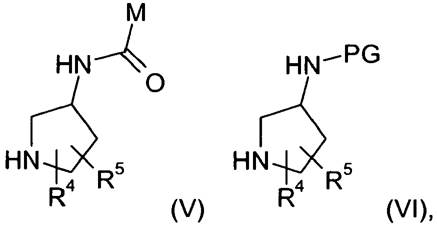 in denen M, R⁴ und R⁵ wie in Ausführungsform 1 erwähnt definiert sind, sind in der Literatur bekannt, oder deren Synthese wird in den Ausführungsbeispielen beschrieben, oder sie können beispielsweise nach in der Literatur bekannten Syntheseverfahren oder in Analogie zu in der Literatur bekannten Syntheseverfahren wie beispielsweise in WO2006/114402 beschrieben, hergestellt werden.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, tert.-Butyl-, Trityl-, Benzyl- oder Tetrahydropyranyl-gruppe in Betracht.

Beispielsweise kommt als Schutzrest für eine Carboxylgruppe die Trimethylsilyl-, Methyl-, Ethyl-, tert.-Butyl-, Benzyl- oder Tetrahydropyranyl-gruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, tert.-Butoxycarbonyl-, Benzyloxycarbonyl-, Benzyl-, Methoxybenzyl- oder 2,4-Dimethoxybenzyl-gruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch chromatographische Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor Vlla, Faktor IX, Faktor XI und Faktor XII.

Die im Experimentellen Teil angeführten Verbindungen können auf ihre Wirkung auf die Hemmung des Faktors Xa wie folgt untersucht werden.

### Methodik

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material

Tris(hydroxymethyl)-aminomethan-Puffer (100 mMol) und Natriumchlorid (150 mMol), pH 8.0 plus 1 mg/ml Human Albumin Fraction V, Proteasefrei.

Faktor Xa (Calbiochem), Spez. Aktivität: 217 IU/mg, Endkonzentration: 7 IU/ml pro Reaktionsansatz.

Substrat S 2765 (Chromogenix), Endkonzentration: 0.3 mM/l (1 KM) pro Reaktionsansatz.

Testsubstanz: Endkonzentration 100, 30, 10, 3, 1, 0.3, 0.1, 0.03, 0.01, 0.003, 0.001 µMol/l.

### Durchführung

10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigen IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, Thrombophlebitis, der Verhinderung von Reokklusionen nach Bypass-Operationen oder Angioplastie (PT(C)A), sowie der Okklusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulzerativen Colitis, der Prophylaxe und Behandlung der Koronarthrombose, der Prophylaxe des Schlaganfalls und der Verhinderung der Okklusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von fibrin-abhängigen Gewebsadhäsionen und/oder Narbengewebebildung sowie zur Förderung von Wundheilungsprozessen geeignet.

Die genannten Verbindungen können auch als Antikoagulantien in Zusammenhang mit der Herstellung, Lagerung, Fraktionierung oder Verwendung von Vollblut oder bei invasiven Therapieverfahren, zum Beispiel zum Beschichten von Prothesen, künstlichen Herzklappen und Kathetern zur Reduktion des Thromboserisikos eingesetzt werden.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Hirntraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging, 2004, 25(6), 783-793). Darüberhinaus deuten verschiedene in vitro Studien an Gehirnen von Patienten mit Alzheimer-Krankheit darauf hin, dass Thrombin in der Pathogenese dieser Krankheit eine Rolle spielt (Neurosci. Lett., 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci., 2003, 23, 5877-86).

Die neuen Verbindungen und ihre physiologisch verträglichen Salze sind auch für die Prophylaxe und Behandlug von arteriellen vaskulären Erkrankungen in der Kombinationstherapie mit lipidsenkenden Wirkstoffen wie HMG-CoA Reduktase Inhibitoren und Vasodilatatoren, insbesondere ACE-Inhibitoren, Angiotensin II-Antagonisten, Renin-Inhibitoren, ß-Rezeptor-Antagonisten, α-Rezeptor-Antagonisten, Diuretika, Ca-Kanalblockern, oder Stimulatoren der löslichen Guanylatzyklase einsetzbar.

Durch Erhöhung der antithrombotischen Wirkung sind die neuen Verbindungen und ihre physiologisch verträglichen Salze auch in der Kombinationstherapie mit anderen Antikoagulantien wie beispielsweise unfraktioniertem Heparin, niedermolekularem Heparin, Fondaparinux oder direkten Thrombinhemmern, zum Beispiel rekombinantem Hirudin oder "active-site"-Thrombinhemmern, einsetzbar

Die neuen Verbindungen und deren physiologisch verträgliche Salze können therapeutisch in Kombination mit Azetylsalizylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Prasugrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX*^{™}*, 35-70 µm) verwendet. Falls nähere Angaben zur Konfiguration fehlen, bleibt offen, ob es sich um reine Stereoisomere oder um Enantiomeren-/Diastereomerengemische handelt.

Die HPLC-MS Daten wurden unter den folgenden Bedingungen erzeugt.

### Methode A:

Waters Alliance 2695, Waters Micromass ZQ Massen Spektrometer mit Diodenarraydetektor 2996.

Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 6.00 |
| 0.01 | 95 | 5 | 6.00 |
| 0.89 | 2 | 98 | 6.00 |
| 0.90 | 2 | 98 | 6.00 |
| 0.95 | 95 | 5 | 6.00 |
| 1.05 | 95 | 5 | 6.00 |
| 1.10 | 95 | 5 | 0.10 |

Als stationäre Phase diente eine Säule Varian MS 100 C18, 3 µm, 4.6 mm x 30 mm.

### Methode B:

Waters Alliance 2695, Waters Micromass ZQ Massen Spektrometer mit Diodenarraydetektor 2996.

Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 3.50 |
| 0.18 | 95 | 5 | 3.50 |
| 2.00 | 2 | 98 | 3.50 |
| 2.20 | 2 | 98 | 3.50 |
| 2.30 | 95 | 5 | 3.50 |
| 2.50 | 95 | 5 | 3.50 |
| 2.60 | 95 | 5 | 0.10 |

Als stationäre Phase diente eine Säule Varian MS 100 C18, 3 µm, 4.6 mm x 30 mm.

Die Diodenarraydetektion erfolgte im Wellenlängenbereich 210-380 nm.

### Methode C:

Waters Alliance 2695, Waters Micromass ZQ Massen Spektrometer mit Diodenarraydetektor 2996.

Als mobile Phase wurde eingesetzt:
A: Wasser mit 0.1 % Ammoniak
B: Acetonitril

| Zeit in min | %A | %B | Flussrate in ml/min |
|---|---|---|---|
| 0.00 | 95 | 5 | 4.00 |
| 0.01 | 95 | 5 | 4.00 |
| 0.89 | 2 | 98 | 4.00 |
| 0.90 | 2 | 98 | 4.00 |
| 0.95 | 95 | 5 | 4.00 |
| 1.05 | 95 | 5 | 4.00 |
| 1.10 | 95 | 5 | 0.10 |

Als stationäre Phase diente eine Säule Waters Xbridge C18, 3.5 µm, 4.6 mm x 20 mm.

Die Diodenarraydetektion erfolgte im Wellenlängenbereich 210-380 nm.

### Methode D:

Waters Alliance 2695, Waters Micromass ZQ Massen Spektrometer mit Diodenarraydetektor 2996.

Als mobile Phase wurde eingesetzt:
A: Wasser mit 0.13% Trifluoressigsäure
B: Acetonitril

| Zeit in min | %A | %B | Flussrate in ml/min |
|---|---|---|---|
| 0.00 | 100 | 0 | 5.00 |
| 0.08 | 100 | 0 | 5.00 |
| 1.70 | 0 | 100 | 5.00 |
| 1.75 | 0 | 100 | 5.00 |
| 1.80 | 100 | 0 | 5.00 |
| 1.85 | 100 | 0 | 5.00 |
| 1.90 | 100 | 0 | 0.10 |

Als stationäre Phase diente eine Varian Polaris C18, 3 µm, 4.6 mm x 30 mm.

Die Diodenarraydetektion erfolgte im Wellenlängenbereich 210-380 nm.

### Methode E:

Waters Alliance 2695, Waters Micromass ZQ Massen Spektrometer mit Diodenarraydetektor 2996.

Als mobile Phase wurde eingesetzt:
A: Wasser mit 0.1% Ammoniak
B: Acetonitril

| Zeit in min | %A | %B | Flussrate in ml/min |
|---|---|---|---|
| 0.00 | 95 | 5 | 4.00 |
| 0.01 | 95 | 5 | 4.00 |
| 0.89 | 2 | 98 | 4.00 |
| 0.90 | 2 | 98 | 4.00 |
| 0.95 | 95 | 5 | 4.00 |
| 1.05 | 95 | 5 | 4.00 |
| 1.10 | 95 | 5 | 0.50 |

Als stationäre Phase diente eine Säule Waters Xbridge C18, 3.5 µm, 4.6 mm x 20 mm.

Die Diodenarraydetektion erfolgte im Wellenlängenbereich 210-380 nm.

### Methode F:

Waters Alliance 2695, Waters Micromass ZQ Massen Spektrometer mit Diodenarraydetektor 2996.

Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 3.50 |
| 0.18 | 95 | 5 | 3.50 |
| 2.00 | 2 | 98 | 3.50 |
| 2.20 | 2 | 98 | 3.50 |
| 2.30 | 95 | 5 | 3.50 |
| 2.50 | 95 | 5 | 3.50 |
| 2.60 | 95 | 5 | 0.50 |

Als stationäre Phase diente eine Säule Varian MS 100 C18, 3 µm, 4.6 mm x 30 mm.

Die Diodenarraydetektion erfolgte im Wellenlängenbereich 210-380 nm.

### Methode G:

Waters Alliance 2695, Waters Micromass ZQ Massen Spektrometer mit Diodenarraydetektor 2996.

Als mobile Phase wurde eingesetzt:
A: Wasser mit 0.1% Ammoniak
B: Acetonitril

| Zeit in min | %A | %B | Flussrate in ml/min |
|---|---|---|---|
| 0.00 | 95 | 5 | 4.00 |
| 0.01 | 95 | 5 | 4.00 |
| 0.89 | 2 | 98 | 4.00 |
| 0.90 | 2 | 98 | 4.00 |
| 0.95 | 95 | 5 | 4.00 |
| 1.05 | 95 | 5 | 4.00 |
| 1.10 | 95 | 5 | 0.50 |

Als stationäre Phase diente eine Säule Waters Xbridge C18, 3.5 µm, 4.6 mm x 20 mm.

Die Diodenarraydetektion erfolgte im Wellenlängenbereich 210-380 nm.

### Methode H:

Waters Alliance 2695, Waters Micromass ZQ Massen Spektrometer mit Diodenarraydetektor 2996.

Als mobile Phase wurde eingesetzt:
A: Wasser mit 0.13% Ammoniak
B: Acetonitril

| Zeit in min | %A | %B | Flussrate in ml/min |
|---|---|---|---|
| | 95 | 5 | 5.00 |
| 0.01 | 95 | 5 | 5.00 |
| 1.80 | 2 | 98 | 5.00 |
| 1.90 | 2 | 98 | 5.00 |
| 2.00 | 95 | 5 | 5.00 |
| 2.10 | 95 | 5 | 5.00 |
| 2.15 | 95 | 5 | 0.50 |

Als stationäre Phase diente eine Säule Varian Pursuit XRS 5 C18, 3 µm, 4.6 mm x 30 mm.

Die Diodenarraydetektion erfolgte im Wellenlängenbereich 210-380 nm.

In den Versuchsbeschreibungen werden die folgenden Abkürzungen verwendet.
- DCM: Dichlormethan
- DIPEA: *N*-Ethyl-diisopropylamin
- DMF: *N,N*-Dimethylformamid
- EtOH: Ethanol
- ges.: gesättigt
- h: Stunde(n)
- HATU: *O*-(7-Azabenzotriazol-1-yl)-*N,N,N'N*'-tetramethyluronium-hexafluorphosphat
- i. Vak.: im Vakuum
- konz.: konzentriert
- min: Minute(n)
- NMM: *N*-Methyl-morpholin
- R_{f}: Retentionsfaktor
- Rₜ: Retentionszeit
- TBTU: *O*-(Benzotriazol-1-yl)-*N,N,N',N*'-tetramethyluroniumtetrafluorborat
- TEA: Triethylamin
- TFA: Trifluoressigsäure
- THF: Tetrahydrofuran

### Beispiel 1

### 5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz)

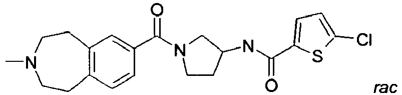

### (a) 3-[(5-Chlor-thiophen-2-carbonyl)-amino]-pyrrolidin-1-carbonsäure-tert.-butylester

0.5 g (2.7 mmol) 3-Amino-pyrrolidin-1-carbonsäure-tert.-butylester werden in 7 ml DCM gelöst, mit 1.4 ml (10.1 mmol) TEA und 0.5 g (2.7 mmol) 5-Chlorthiophen-2-carbonsäurechlorid versetzt und eine Stunde bei RT gerührt. Das Reaktionsgemisch wird mit DCM verdünnt und nacheinander mit verd. wässriger KHSO₄-Lösung ges. wässriger NaHCO3-Lösung und Wasser gewaschen. Die vereinigten organischen Phasen werden über Natriumsulfat getrocknet und i. Vak. bis zur Trockene eingeengt.
Rₜ-Wert: 0.65 min (Methode A)
C₁₄H₁₉CiN₂O₃S (330.83)
Massenspektrum: (M+H)⁺ = 329/331 (Chlorisotope)

### (b) 5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz)

150 mg (453 mmol) 3-[(5-Chlor-thiophen-2-carbonyl)-amino]-pyrrolidin-1-carbonsäure-tert.-butylester werden in einem Gemisch aus DCM/TFA (v/v 1:1) bei RT für 30 min gerührt. Dann wird 1 ml (76 mmol) TEA zugetropft, so dass die Mischung alkalisch reagiert.

In einem weiteren Reaktionsgefäß werden 110 mg (455 mmol) 3-Methyl-2,3,4,5-tetrahydro-1H-benzo[d]azepin-7-carbonsäure-Hydrochlorid in 5 ml DCM vorgelegt und nacheinander mit 0.3 ml (2.3 mmol) TEA und 0.2 g (0.5 mmol) TBTU versetzt. Diese Reaktionsmischung wird für 20 min bei RT gerührt, dann zu der zuvor hergestellten Amin-Lösung gegeben und für 72 Stunden gerührt. Das Reaktionsgemisch wird i. Vak. eingeengt, und der Rückstand wird mit TFA sauer gestellt und mittels RP-HPLC gereinigt.
Rₜ-Wert: 1.16 min (Methode B)
C₂₁H₂₄CiN₂O₃S x CF₃CO₂H (417.96)
Massenspektrum: (M+H)⁺ = 418/420 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **2** | 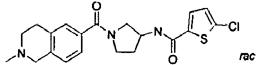 | (M+H)⁺ = 404/406 (Chlorisotope) | Rₜ-Wert = 0.44 min (Methode A) |
| | 5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **3** | 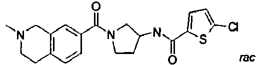 | (M+H)⁺ = 404/406 (Chlorisotope) | Rₜ-Wert = 0.44 min (Methode A) |
| | 5-Chlor-thiophen-2-carbonsäure-[1-(2-methyl-1,2,3,4-tetrahydro-isochinolin-7-carbonyl)-pyrrolidin-3-yl]-amid (als Trifluoracetat-Salz) | | |
| **4** | 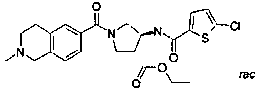 | (M+H)⁺ = 476/478 (Chlorisotope) | Rₜ-Wert = 1.25 min (Methode B) |
| | (3RS,4RS)-4-[(5-Chlor-thiophen-2-carbonyl)-amino]-1-(2-methyl-1,2,3,4-tetrahydro-isochinolin-6-carbonyl)-pyrrolidin-3-carbonsäure-ethylester (als Trifluoracetat-Salz) | | |
| **5** | 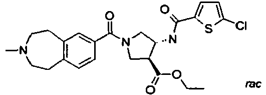 | (M+H)⁺ = 490/492 (Chlorisotope) | Rₜ-Wert: 1.29 min (Methode B) |
| | (3RS,4RS)-4[(5-Chlor-thiophen-2-carbonyl)-amino]-1-(3-methyl-2,3,4,5-tetrahydro-1H-benzo[d]azepin-7-carbonyl)-pyrrolidin-3-carbonsäure-ethylester (als Trifluoracetat-Salz) | | |
| **8** | 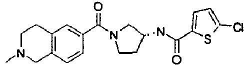 | (M+H)⁺ = 404/406 (Chlorisotope) | Rₜ-Wert = 1.14 min (Methode B) |
| | (R)-5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **9** | 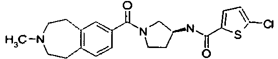 | (M+H)⁺ = 418/420 (Chlorisotope) | Rₜ-Wert = 1.14 min (Methode B) |
| | (S)-5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz) | | |
| **10** | 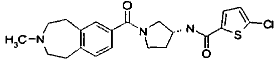 | (M+H)⁺ = 418/420 (Chlorisotope) | Rₜ-Wert = 1.17 min (Methode B) |
| | (R)-5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz) | | |
| **11** | 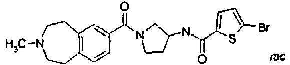 | (M+H)⁺ = 462/464 (Bromisotope) | Rₜ-Wert = 1.15 min (Methode B) |
| | 5-Brom-thiophen-2-carbonsäure-[1-(3-methyl-2,3,4,5-tetrahydro- | | |
| | 1H-benzo[d]azepin-7-carbonyl)-pyn-olidin-3-yl]-amid (als Trifluoracetat-Salz) | | |
| **12** | 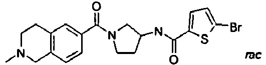 | (M+H)⁺ = 448/450 (Bromsotope) | Rₜ-Wert = 1.13 min (Methode B) |
| | 5-Brom-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **13** | 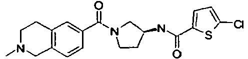 | (M+H)⁺ = 404/406 (Chlorisotope) | Rₜ-Wert=1.14 min (Methode B) |
| | (S)-5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **23** | 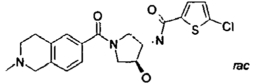 | (M+H)⁺ = 420/422 (Chlorisotope) | Rₜ-Wert = 0.99 min (Methode B) |
| | (3SR,4SR)-5-Chlor-thiophen-2-carbonsäure-[4-hydroxy-1-(2-metyl-1,2,3,4-tetrahydro-isochinolin-6-carbonyl)-pyrrolidin-3-yl]-amid (als Trifluoracetat-Salz) | | |
| **25** | 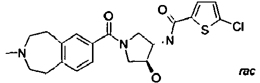 | (M+H)⁺ = 434/436 (Chlorisotope) | Rₜ-Wert: 1.02 min (Methode B) |
| | (3SR,4SR)-5-Chlor-thiophen-2-carbonsäure-[4-hydroxy-1-(3-metyl-2,3,4,5-tetrahydro-1H-benzo[d]azepin-7-carbonyl)-pyrrolidin-3-yl]-amid (als Trifluoracetat-Salz) | | |
| **28** | 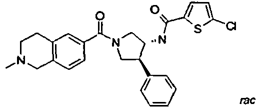 | (M+H)⁺ = 480/482 (Chlorisotope) | Rₜ-Wert = 1.32 min (Methode B) |
| | (3RS,4SR)-5-Chlor-thiophen-2-carbonsäure-[1-(2-methyl-1,2,3,4-tetrahydro-isochinolin-6-carbonyl)-4-phenyl-pyrrolidin-3-yl]-amid (als Trifluoracetat-Salz) | | |
| **29** | 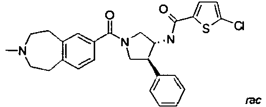 | (M+H)⁺ = 494/496 (Chlorisotope) | Rₜ-Wert: 1.36 min (Methode B) |
| | (3RS,4SR)-5-Chlor-thiophen-2-carbonsäure-[1-(3-methyl-2,3,4,5-tetrahydro-1H-benzo[d]azepin-7-carbonyl)-4-phenyl-pyrrolidin-3-yl]-amid (als Trifluoracetat-Salz) | | |

### Beispiel 6

### (3RS,4RS)-4-[(Chlor-thiophen-2-carbonyl)-amino]-1-(2-methyl-1,2,3,4-tetrahydro-isochinolin-6-carbonyl)-pyrrolidin-2-carbonsäure (als Trifluoracetat-Salz)

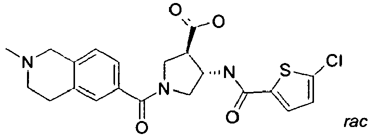

### (a) (3SR,4RS)-4-[(Chlor-thiophen-2-carbonyl)-amino]-1-(2-methyl-1,2,3,4-tetrahydro-isochinolin-6-carbonyl)-pyrrolidin-2-carbonsäure (als Trifluoracetat-Salz)

14 mg (24 µmol) (3SR,4RS)-4-[(5-Chlor-thiophen-2-carbonyl)-amino]-1-(2-methyl-1,2,3,4-tetrahydro-isochinolin-6-carbonyl)-pyrrolidin-3-carbonsäure-ethylester (als Trifluoracetat-Salz) werden in 500 µL Methanol gelöst, mit 120 µL Lithiumhydroxid-Lösung (8%ig in Wasser) versetzt und 16 Stunden bei RT gerührt. Man konzentriert die Mischung i.Vak. und reinigt den Rückstand mittels
RP-HPLC.
Rₜ-Wert: 1.07 min (Methode B)
C₂₁H₂₂ClN₃O₄S x CF₃CO₂H (447.94)
Massenspektrum: (M+H)⁺ = 448/450 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **7** | 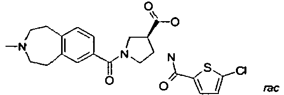 | (M+H)⁺ = 462/464 (Chlorisotope) | Rₜ-Wert: 1.10 min (Methode B) |
| | (3SR,4RS)-4-[(5-Chlor-thiophen-2-carbonyl)-amino]-1-(3-methyl-2,3,4,5-tetrahydro-1H-benzo[d]azepin-7-carbonyl)-pyrrolidin-3-carbonsäure (als Trifluoracetat-Salz) | | |
| **21** | 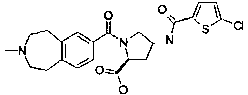 | (M+H)⁺ = 462/464 (Chlorisotope) | Rₜ-Wert: 1.11 min (Methode B) |
| | (2S,4R)-4-[(5-Chlor-thiophen-2-carbonyl)-amino]-1-(3-methyl-2,3,4,5-tetrahydro-1H-benzo[d]azepin-7-carbonyl)-pyrrolidin-2-carbonsäure (als Trifluoracetat-Salz) | | |
| **62** | 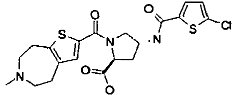 | (M+H)⁺ = 468/470 (Chlorisotope) | Rₜ-Wert: 1.11 min (Methode B) |
| | (2*S*,4*R*)-4-[(5-Chlor-thiophen-2-carbonyl)-amino]-1-(6-methyl-5,6,7,8-tetrahydro-4H-thieno[2,3-d]azepin-2-carbonyl)-pyrrolidin-2-carbonsäure (als Trifluoracetat-Salz) | | |
| **88** | 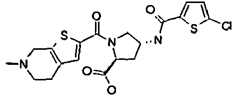 | (M+H)⁺ = 454/456 (Chlorisotope) | Rₜ-Wert: 0.47 min (Methode G) |
| | (2*S*,4*R*)-4-[(5-Chlor-thiophen-2-carbonyl)-amino]-1-(6-methyl-4,5,6,7-tetrahydro-thieno[2,3-c]pyridin-2-carbonyl)-pyrrolidin-2-carbonsäure | | |

### Beispiel 14

### (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-methylester (als Trifluoracetat-Salz)

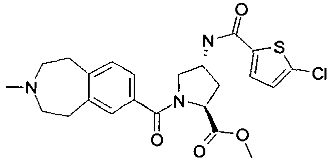

### (a) (2S,4S)-Methansulfonyloxy-pyrrolidin-1,2-dicarbonsäure-1-tert.-butylester-2-methylester

6.5 g (26.3 mmol) (2S,4S)-4-Hydroxy-pyrrolidin-1,2-dicarbonsäure-1-tert.-butylester-2-methylester werden in 40 ml DCM gelöst und bei 0°C mit 4.4 ml (31.7 mmol) TEA und 2.5 ml (32.2 mmol) Methansulfonsäurechlorid versetzt. Die Mischung wird 30 Minuten bei 0°C und zwei Stunden bei RT gerührt. Anschließend gießt man die Mischung auf Wasser und extrahiert die wässrige Phase dreimal mit DCM. Die vereinigten organischen Phasen werden über Natriumsulfat getrocknet und i.Vak. konzentriert.
Rₜ-Wert: 1.27 min (Methode B)
C₁₂H₂₁NO₇S (323.36)
Massenspektrum: (M+H)⁺ = 324

### (b) (2S,4R)-Azido-pyrrolidin-1,2-dicarbonsäure-1-tert.-butylester-2-methylester

8.5 g (26.3 mmol) (2S,4S)-Methansulfonyloxy-pyrrolidin-1,2-dicarbonsäure-1-tert.-butylester-2-methylester werden in 25 ml DMF gelöst und bei RT mit 6.0 g (92.3 mmol) Natriumazid versetzt. Die Mischung wird 20 Stunden bei 50°C gerührt. Anschließend konzentriert man das Reaktionsgemisch i.Vak. und versetzt den Rückstand mit Ethylacetat und Wasser. Die wässrige Phase wird dreimal mit Ethylacetat extrahiert. Die vereinigten organischen Phasen werden über Natriumsulfat getrocknet und i.Vak. konzentriert.
Rₜ-Wert: 1.38 min (Methode B)
C₁₁H₁₈N₄O₄ (270.29)
Massenspektrum: (M+H)⁺ = 271

### (c) (2S,4R)-Amino-pyrrolidin-1,2-dicarbonsäure-1-tert.-butylester-2-methylester

7.5 g (27.8 mmol) (2S,4R)-Azido-pyrrolidin-1,2-dicarbonsäure-1-tert.-butylester-2-methylester werden in 15 ml Methanol gelöst, mit 500 mg Palladium/Kohle (10%ig) versetzt und zwei Tage mit 3 bar Wasserstoff hydriert. Anschließend wird die Mischung filtriert und i. Vak. eingeengt.
Rₜ-Wert: 0.91 min (Methode B)
C₁₁H₂₀N₂O₄ (244.29)
Massenspektrum: (M+H)⁺ = 245

### (d) (2S,4R)-4-[(5-Chlor-thiophen-2-carbonyl)-amino]-pyrrolidin-1,2-dicarbonsäure-1-tert.-butylester-2-methylester

Herstellung analog Beispiel 1 a aus (2S,4R)-Amino-pyrrolidin-1,2-dicarbonsäure-1-tert.-butylester-2-methylester und 5-Chlor-thiophen-2-carbonylchlorid.
Rₜ-Wert: 1.56 min (Methode B)
C₁₁H₂₁CIN₂O₅S (388.87)
Massenspektrum: (M-H)⁻ = 387/389 (Chlorisotope)

### (e) (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-methylester (als Trifluoracetat-Salz)

Herstellung analog Beispiel 1 b aus (2S,4R)-4-[(5-Chlor-thiophen-2-carbonyl)-amino]-pyrrolidin-1,2-dicarbonsäure-1-tert.-butylester-2-methylester und 3-Methyl-2,3,4,5-tetrahydro-1H-benzo[d]azepin-7-carbonsäure-Hydrochlorid.
Rₜ-Wert: 1.29 min (Methode B)
C₂₃H₂₆CIN₃O₄S x CF₃CO₂H (476.00)
Massenspektrum: (M+H)⁺ = 476/478 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **15** | 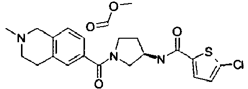 | (M+H)⁺ = 462/464 (Chlorisotope) | Rₜ-Wert: 1.01 min (Methode B) |
| | (2S,4R)-4-[(5-Chlor-thiophen-2-carbonyl)-amino]-1-(2-methyl-1,2,3,4-tetrahydro-isochinolin-6-carbonyl)-pyrrolidin-2-carbonsäure-methylester (als Trifluoracetat-Salz) | | |
| **59** | 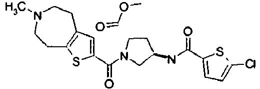 | (M+H)⁺ = 482/484 (Chlorisotope) | Rₜ-Wert: 1.14 min (Methode F) |
| | (2S,4R)-4-[(5-Chlor-thiophen-2-carbonyl)-amino]-1-(6-methyl-5,6,7,8-tetrahydro-4H-thieno[2,3-d]azepin-2-carbonyl)-pyrrolidin-2-carbonsäure-methylester | | |
| **87** | 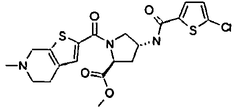 | (M+H)⁺ = 468/470 (Chlorisotope) | Rₜ-Wert: 0.68 min (Methode G) |
| | (2*S*,4*R*)-4-[(5-Chlor-thiophen-2-carbonyl)-amino]-1-(6-methyl-4,5,6,7-tetrahydro-thieno[2,3-c]pyridin-2-carbonyl)-pyrrolidin-2-carbonsäure-methylester | | |

### Beispiel 16

### (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-dimethylamid (als Trifluoracetat-Salz)

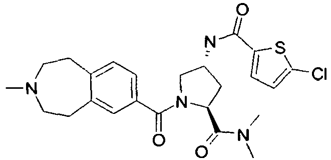

### (a) (2S,4R)-4-[(5-Chlor-thiophen-2-carbonyl)-amino]-2-dimethylcarbamoyl-pyrrolidin-1-carbonsäure-tert.-butylester

210 mg (2.6 mmol) Dimethylamin-Hydrochlorid werden in 5 ml DCM gelöst und mit 3 ml Trimethylaluminium-Lösung (2M in Toluol, 6 mmol) versetzt. Man rührt diese Mischung für 30 Minuten, gibt dann eine Lösung aus 1.0 g (2.6 mmol) (2S,4R)-4-[(5-Chlor-thiophen-2-carbonyl)-amino]-pyrrolidin-1,2-dicarbonsäure-1-tert.-butylester-2-methylester in 5 ml DCM hinzu und rührt 16 Stunden bei RT. Dann werden weitere 3 mmol Dimethylaluminium-dimethylamid-Lösung in DCM/Toluol (hergestellt analog) zugegeben und weitere drei Tage bei RT gerührt. Man verdünnt die Mischung mit 20 ml DCM und versetzt mit wenig Wasser. Diese Mischung wird i. Vak. eingeengt. Der Rückstand wird mit DCM versetzt und nacheinander mit Wasser und 0.5 N Natronlauge gewaschen. Die organische Phase wird einmal mit 0.5 N Salzsäure gewaschen, über Natriumsulfat getrocknet und i.Vak. eingeengt.
Rₜ-Wert: 1.32 min (Methode B)
C₁₇H₂₄CIN₃O₄S (401.91)
Massenspektrum: (M+H)⁺ = 402/404 (Chlorisotope)

### (b) (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-methylester (als Trifluoracetat-Salz)

Herstellung analog Beispiel 1 b aus (2S,4R)-4-[(5-Chlor-thiophen-2-carbonyl)-amino]-2-dimethylcarbamoyl-pyrrolidin-1-carbonsäure-tert.-butylester und 3-Methyl-2,3,4,5-tetrahydro-1H-benzo[d]azepin-7-carbonsäure-Hydrochlorid.
Rₜ-Wert: 1.07 min (Methode B)
C₂₄H₂₉CIN₄O₃S x CF₃CO₂H (489.04)
Massenspektrum: (M+H)⁺ = 489/491 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **17** | 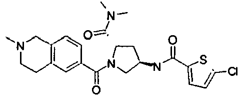 | (M+H)⁺ = 475/477 (Chlorisotope) | Rₜ-Wert: 1.05 min (Methode B) |
| | (2S,4R)-4-[(5-Chlor-thiophen-2-carbonyl)-amino]-1-(2-methyl-1,2,3,4-tetrahydro-isochinolin-6-carbonyl)-pyrrolidin-2-carbonsäure-dimethylamid (als Trifluoracetat-Salz) | | |
| **33** | 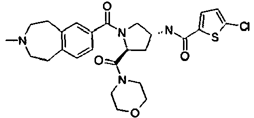 | (M+H)⁺ = 531/533 (Chlorisotope) | Rₜ-Wert: 1.09 min (Methode B) |
| | (2S,4R)-5-Chlor-thiophen-2-carbonsäure-[1-(3-methyl-2,3,4,5-tetrahydro-1H-benzo[d]azepin-7-carbonyl)-5-(rnorpholin-4-carbonyl)-pyrrolidin-3-yl]-amid (als Trifluoracetat-Salz) | | |
| **34** | 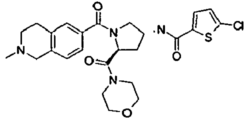 | (M+H)⁺ = 517/519 (Chlorisotope) | Rₜ-Wert: 1.05 min (Methode B) |
| | (2S,4R)-5-Chlor-thiophen-2-carbonsäure-[2-methyl-1,2,3,4-tetrahydro-isochinolin-6-carbonyl)-5-(morpholin-4-carbonyl)-pyrrolidin-3-yl]-amid (als Trifluoracetat-Salz) | | |
| **75** | 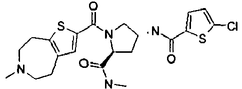 | (M+H)⁺ = 481/483 (Chlorisotope) | Rₜ-Wert: 1.02 min (Methode B) |
| | (2S,4R)-4-[(5-Chlor-thiophen-2-carbonyl)-amino]-1-(6-methyl-5,6,7,8-tetraydro-4H-thieno[2,3-d]azepin-2-carbonyl)-pyrrolidin-2-carbonsäure-methylamid (als Trifluoracetat-Salz) | | |
| **57** | 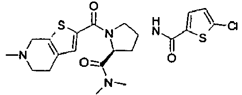 | (M-H)⁻ = 479/481 (Chlorisotope) | Rₜ-Wert: 1.05 min (Methode F) |
| | (2S,4R)-4-[(5-Chlor-thiophen-2-carbonyl)-amino]-1-[6-methyl-4,5,6,7,-tetrahydro-thieno[2,3-c]pyridin-2-carbonyl]-pyrrolidin-2-carbonsäure-dimethylamid | | |
| **58** | 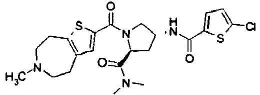 | (M+H)⁺ = 495/497 (Chlorisotope) | |
| | (2S,4R)-4-[(5-Chlor-thiophen-2-carbonyl)-amino]-1-(6-methyl-5,6,7,8-tetraydro-4H-thieno[2,3-d]azepin-2-carbonyl)-pyrrolidin-2-carbonsäure-dimethylamid | | |
| **78** | 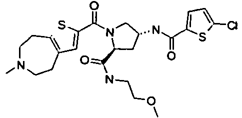 | (M+H)⁺ = 525/527 (Chlorisotope) | Rₜ-Wert: 0.61 min (Methode G) |
| | (2S,4R)-4-[(5-Chlor-thiophen-2-carbonyl)-amino]-1-(6-methyl-5,6,7,8-tetraydro-4H-thieno[2,3-d]azepin-2-carbonyl)-pyrrolidin-2-carbonsäure-(2-methoxy-ethyl)-amid | | |
| **79** | 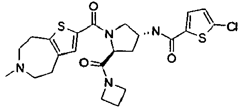 | (M+H)⁺ = 507/509 (Chlorisotope) | Rₜ-Wert: 0.63 min (Methode G) |
| | (3*S*,5*R*)-5-Chlor-thiophen-2-carbonsäure-[5-(azetidin-1-carbonyl)-1-(6-methyl-5,6,7,8-tetrahydro-4H-thieno[2,3-d]azepin-2-carbonyl)-pyrrolidin-3-yl]-amid | | |
| **80** | 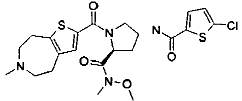 | (M+H)⁺ = 511/513 (Chlorisotope) | Rₜ-Wert: 1.16 min (Methode F) |
| | (2*S*,4*R*)-4-[(5-Chlor-thiophen-2-carbonyl)-amino]-1-(6-methyl-5,6,7,8-tetraydro-4H-thieno[2,3-d]azepin-2-carbonyl)-pyrrolidin-2-carbonsäure-methoxy-methyl-amid (als Hydrochlorid-Salz) | | |
| **83** | 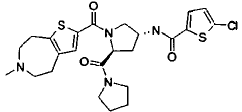 | (M+H)⁺ = 521/523 (Chlorisotope) | Rₜ-Wert: 1.15 min (Methode F) |
| | (3*S*,5*R*)-5-Chlor-thiophen-2-carbonsäure-[1-(6-methyl-5,6,7,8-tetrahydro-4H-thieno[2,3-d]azepin-2-carbonyl)-5-pyrrolidin-1-carbonyl)-pyrrolidin-3-yl]-amid | | |

### Beispiel 19

(3RS,4SR)-5-Chlor-thiophen-2-carbonsäure-[4-hydroxymethyl-1-(2-methyl-1,2,3,4-tetrahydro-isochinolin-6-carbonyl)-pyrrolidin-3-yl]-amid (als Trifluoracetat-Salz)

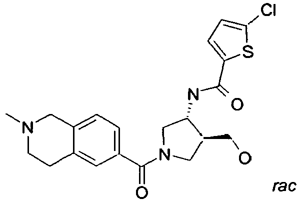

### (a) (3RS,4SR)-3-[(5-Chlor-thiophen-2-carbonyl)-amino]-4-hydroxymethyl-pyrrolidin-1-carbonsäure-tert.-butylester

168 mg (417 µmol) (3RS,4RS)-4-[(5-Chlor-thiophen-2-carbonyl)-amino]-pyrrolidin-1,3-dicarbonsäure-1-tert.-butylester-3-ethylester werden in 4 ml THF gelöst, portionsweise mit insgesamt 20 mg (872 µmol) Lithiumborhydrid versetzt und für eine Stunde bei RT gerührt. Anschließend wird die Reaktionsmischung in ges. Natriumchlorid-Lösung gegossen und gerührt. Man extrahiert die wässrige Phase dreimal mit Ethylacetat, trocknet die vereinigten organischen Phasen über Natriumsulfat und engt i.Vak. zur Trockene ein.
Rₜ-Wert: 1.36 min (Methode B)
C₁₅H₂₁CIN₂O₄S (360.86)
Massenspektrum: (M+H)⁺ = 361/363 (Chlorisotope)

### (b) (3RS,4SR)-5-Chlor-thiophen-2-carbonsäure-[4-hydroxymethyl-1-(2-methyl-1,2,3,4-tetrahydro-isochinolin-6-carbonyl)-pyrrolidin-3-yl]-amid

Herstellung analog Beispiel 1 b aus (3RS,4RS)-3-[(5-Chlor-thiophen-2-carbonyl)-amino]-4-hydroxymethyl-pyrrolidin-1-carbonsäure-tert.-butylester und 2-Methyl-1,2,3,4-tetrahydro-isochinolin-6-carbonsäure-Hydrochlorid.
Rₜ-Wert: 1.00 min (Methode B)
C₂₁H₂₄CIN₃O₃S (433.95)
Massenspektrum: (M+H)⁺ = 434/436 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **18** | 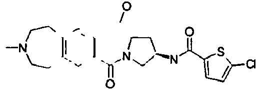 | (M+H)⁺ = 448/450 (Chlorisotope) | Rₜ-Wert: 1.03 min (Methode B) |
| | (2S,4R)-5-Chlor-thiophen-2-carbonsäure-[5-hydroxymethyl-1-(3-methyl-2,3,4,5-tetrahydro-1H-benzo[d]azepin-7-carbonyl)-pyrrolidin-3-yl]-amid | | |
| **20** | 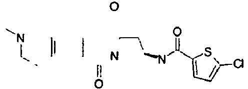 | (M+H)⁺ = 434/436 (Chlorisotope) | Rₜ-Wert: 1.00 min (Methode B) |
| | (2S,4R)-5-Chlor-thiophen-2-carbonsäure-[5-hydroxymethyl-1-(2-methyl-1,2,3,4-tetrahydro-isochinolin-6-carbonyl)-pyrrolidin-3-yl]-amid (als Trifluoracetat-Salz) | | |
| **63** | 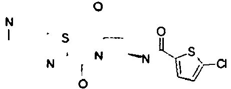 | (M+H)⁺ = 455/457 (Chlorisotope) | Rₜ-Wert: 1.01 min (Methode F) |
| | (2S,4R)-5-Chlor-thiophen-2-carbonsäure-[5-hydroxymethyl-1-(6-methyl-5,6,7,8-tetrahydro-4H-thiazolo[4,5-d]azepin-2-carbonyl)-pyrrolidin-3-yl]-amid (als Trifluoracetat-Salz) | | |
| **64** | 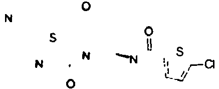 | (M+H)⁺ = 441/443 (Chlorisotope) | Rₜ-Wert: 1.01 min (Methode F) |
| | (2S,4R)-5-Chlor-th iophen-2-carbonsäure-[5-hydroxymethyl-1-(5-methyl-4,5,6,7-tetrahydro-thiazolo[4,5-c]pyridin-2-carbonyl)-pyrrolidin-3-yl]-amid (als Trifluoracetat-Salz) | | |
| **66** | 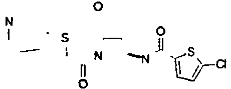 | (M-H)⁻ = 452/454 (Chlorisotope) | Rₜ-Wert: 0.60 min (Methode G) |
| | (2S,4R)-5-Chlor-thiophen-2-carbonsäure-[5-hydroxymethyl-1-(6-methyl-5,6,7,8-tetrahydro-4H-thieno[2,3-d]azepin-2-carbonyl)-pyrrolidin-3-yl]-amid (als Trifluoracetat-Salz) | | |

### Beispiel 22

### (3RS,4RS)-5-Chlor-thiophen-2-carbonsäure-[4-methoxy-1-(2-methyl-1,2,3,4-tetrahydro-isochinolin-6-carbonyl)-pyrrolidin-3-yl]-amid (als Trifluoracetat-Salz)

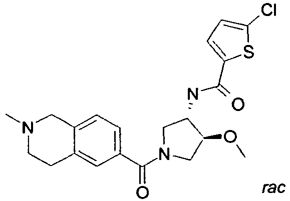

### (a) (3SR,4SR)-3-[(5-Chlor-thiophen-2-carbonyl)-amino]-4-methoxy-pyrrolidin-1-carbonsäure-tert.-butylester

Herstellung analog Beispiel 1a aus (3SR,4SR)-3-Amino-4-methoxy-pyrrolidin-1 - carbonsäure-tert.-butylester (hergestellt analog Y. Tsuzuki et al. Tetrahedron Asymm. 2001, 12, 2989) und 5-Chlorthiophen-2-carbonylchlorid.
Rₜ-Wert: 1.36 min (Methode B)
C₁₅H₂₁CIN₂O₄S (360.86)
Massenspektrum: (M+H)⁺ = 361/363 (Chlorisotope)

### (b) (3SR,4SR)-5-Chlor-thiophen-2-carbonsäure-[4-methoxy-1-(2-methyl-1,2,3,4-tetrahydro-isochinolin-6-carbonyl)-pyrrolidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 2-Methyl-1,2,3,4-tetrahydro-isochinolin-6-carbonsäure-Hydrochlorid.
Rₜ-Wert: 1.11 min (Methode B)
C₂₁H₂₄CIN₃O₃S (433.96)
Massenspektrum: (M+H)⁺ = 434/436 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **24** | 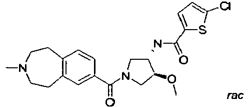 | (M+H)⁺ = 448/450 (Chlorisotope) | Rₜ-Wert: 1.12 min (Methode B) |
| | (3SR,4SR)-5-Chlor-thiophen-2-carbonsäure-[4-methoxy-1-(3-methyl-2,3,4,5-tetrahydro-1H-benzo[d]azepin-7-carbonyl)-pyrrolidin-3-yl]-amid (als Trifluoracetat-Salz) | | |

### Beispiel 26

### (2S,4R)-4-[(5-Chlor-thiophen-2-carbonyl)-amino]-1-[(3R)-2,3-dimethyl-1,2,3,4-tetrahydroisochinolin-6-carbonyl]-pyrrolidin-2-carbonsäure-dimethylamid (als Trifluoracetat-Salz)

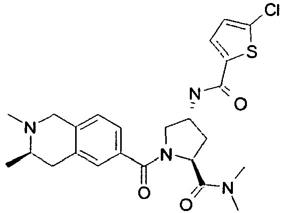

### (a) (Sₛ,R)-2-Methyl-propan-2-sulfinsäure-[2-(5-brom-2-cyano-phenyl)-1 - methyl-ethyl]-amid

6,0 ml (42,8 mmol) Diisopropylamin werden in 80 ml THF gelöst, langsam mit 26,7 ml (42,8 mmol) Butyllithium-Lösung (1.6 M in n-Hexan) bei 0°C versetzt, für 30 min nachgerührt. Anschließend wird diese Lösung auf -78°C abgekühlt und eine Lösung von 4,0 g (20.4 mmol) 4-Brom-2-methyl-benzonitril in 15 ml THF langsam zugetropft. Man rührt diese Mischung 70 Minuten bei -78°C und tropft dann eine Lösung von 1,5 g (10.2 mmol) (Sₛ)-Ethyliden-N-tert.-butyl-sulfinamid (hergestellt analog J. Ellman et al. J. Org. Chem. 2001, 66, 8772 aus Acetaldehyd und (Sₛ)-tert.-Butylsulfinamid) in 15 ml THF zu. Man rührt 2,5 Stunden bei -70 - -65°C. Die Reaktionsmischung wird mit 5 ml ges. Ammoniumchlorid-Lösung versetzt und nach dem Auftauen mit Wasser und Ethylacetat versetzt. Man extrahiert die wässrige Phase dreimal mit Ethylacetat, trocknet die vereinigten organischen Phasen über Natriumsulfat und engt i. Vak. zur Trockene ein. Der Rückstand wird säulenchromatographisch an Silicagel gereinigt (Fließmittel DCM/MeOH 100:3)
Rₜ-Wert: 1.45 min (Methode B)
C₁₄H₁₉BrN₂OS (343.28)
Massenspektrum: (M+H)⁺ = 343/345 (Bromisotope)

### (b) (R)-2-(2-Amino-propyl)-4-brom-benzonitril (als Hydrochlorid-Salz)

830 mg (2.4 mmol) (Sₛ,R)-2-Methyl-propan-2-sulfinsäure-[2-(5-brom-2-cyanophenyl)-1-methyl-ethyl]-amid werden in 10 ml ethanolischer Salzsäure (40%ig) gelöst und für 3 Stunden bei 60°C und dann 16 Stunden bei RT gerührt. Anschließend wird die Reaktionsmischung zur Trockene eingeengt.
Rₜ-Wert: 0.97 min (Methode B)
C₁₀H₁₁BrN₂ x HCl (239.12)
Massenspektrum: (M+H)⁺ = 239/241 (Bromisotope)

### (c) (R)-6-Brom-3-methyl-3,4-dihydro-2H-isochinolin-1-on

480 mg (1.7 mmol) (R)-2-(2-Amino-propyl)-4-brom-benzonitril (als Hydrochlorid-Salz) werden in 5 ml 10 N Natronlauge gelöst und für 16 Stunden bei 80°C gerührt. Anschließend wird die Reaktionsmischung mit Salzsäure sauer gestellt und dreimal mit Ethylacetat extrahiert. Die vereinigten ogranischen Phasen werden über Natriumsulfat getrocknet, filtriert und i. Vak. eingeengt. Der Rückstand wird mittels RP-HPLC gereinigt.
Rₜ-Wert: 1.31 min (Methode B)
C₁₀H₁₀BrNO (240.10)
Massenspektrum: (M+H)⁺ = 240/242 (Bromisotope)

### (d) (R)-6-Brom-2,3-dimethyl-3,4-dihydro-2H-isochinolin-1-on

426 mg (1.7 mmol) (R)-6-Brom-3-methyl-3,4-dihydro-2H-isochinolin-1-on werden in 3 ml DMF gelöst und bei 0°C mit 80 mg (2 mmol) Natriumhydrid (60%ig in Mineralöldispersion) versetzt. Nach 10 Minuten werden 122 µl (1.9 mmol) Methyliodid zugetropft, und die Mischung wird für 16 Stunden bei RT gerührt. Anschließend wird die Reaktionsmischung mit Wasser versetzt und dreimal mit Ethylacetat extrahiert. Die vereinigten organischen Phasen werden über Natriumsulfat getrocknet, filtriert und i. Vak. eingeengt. Der Rückstand wird mittels Flashchromatographie an Silicagel (Fließmittel DCM/MeOH 20:1) gereinigt.
Rₜ-Wert: 1.40 min (Methode B)
C₁₁H₁₂BrNO (254.12)
Massenspektrum: (M+H)⁺ = 254/256 (Bromisotope)

### (e) (R)-2,3-Dimethyl-1-oxo-1,2,3,4-tetrahydroisochinolin-6-carbonsäure-methylester

In einem inertisierten Autoklaven werden 100 mg (394 µmol) (R)-6-Brom-2,3-dimethyl-3,4-dihydro-2H-isochinolin-1-on in einer Mischung aus 20 ml MeOH und 5 ml DMF gelöst und mit 20 mg (89 µmol) Palladium(II)-acetat, 70 mg (86 µmol) 1,1'-Bis-(diphenylphospino)-ferrocen-dichlorpalladium(II)-Komplex mit DCM und mit 110 µl (08. mmol) TEA versetzt. Dann werden 2 bar Kohlenmonoxid aufgepresst und die Mischung für 16 Stunden geschüttelt. Anschließend wird noch zweimal die gleiche Menge Palladium(II)-acetat und 1,1'-Bis-(diphenylphospino)-ferrocen-dichlorpalladium(II)-Komplex mit DCM zugesetzt und jeweils weitere 24 Stunden bei gleicher Temperatur geschüttelt. Man lässt die Mischung abkühlen und filtriert vom Katalysatorgemisch ab. Das Filtrat wird i. Vak. eingeengt. Der so erhaltene Rückstand wird mittels Flashchromatographie an Silicagel (Fließmittel PE/EE 1:1) aufgereinigt. Die Produkt enthaltenden Fraktionen werden vereinigt und i. Vak. konzentriert. Das Rohprodukt wird mittel RP-HPLC gereinigt.
Rₜ-Wert: 1.22 min (Methode B)
C₁₃H₁₅NO₃ (233.26)
Massenspektrum: (M+H)⁺ = 234

### (f) (R)-2,3-Dimethyl-1,2,3,4-tetrahydroisochinolin-6-carbonsäure-methylester

60 mg (257 µmol) (R)-2,3-Dimethyl-1-oxo-1,2,3,4-tetrahydroisochinolin-6-carbonsäure-methylester werden unter Argon-Athmosphäre in 2 ml THF gelöst und bei RT mit 100 µl (542 µmol) Diphenylsilan versetzt. Anschließend werden 20 mg (21 µmol) Carbonylhydridotris(triphenylphosphin)rhodium(I) zugegeben und die Mischung für zwei Stunden gerührt. Man gibt weitere 50 µl Diphenylsilan und 10 mg Carbonylhydridotris(triphenylphosphin)rhodium(I) hinzu und rührt die Mischung weitere 2,5 Stunden. Anschließend wird das Reaktionsgemisch i. Vak. eingeengt und mittels Flashchromatographie an Silicagel (Fließmittel DCM/MeOH 95:5) gereinigt.
Rₜ-Wert: 0.93 min (Methode B)
C₁₃H₁₇NO2 (219.28)
Massenspektrum: (M+H)⁺ = 220

### (g) (R)-2,3-Dimethyl-1,2,3,4-tetrahydroisochinolin-6-carbonsäure (als Hydrochlorid-Salz)

44 mg (201 µmol) (R)-2,3-Dimethyl-1,2,3,4-tetrahydroisochinolin-6-carbonsäure-methylester werden in 2 ml 6 N Salzsäure gelöst und bei 60°C einen Tag gerührt. Anschließend wird das Reaktionsgemisch i. Vak. konzentriert und lyophilisiert.
Rₜ-Wert: 0.62 min (Methode B)
C₁₂H₁₅NO₂ x HCl (205.26)
Massenspektrum: (M+H)⁺ = 206

### (h) (2S,4R)-4-[(5-Chlor-thiophen-2-carbonyl)-amino]-1-[(3R)-2,3-dimethyl-1,2,3,4-tetrahydroisochinolin-6-carbonyl]-pyrrolidin-2-carbonsäure-dimethylamid (als Trifluoracetat-Salz)

Herstellung analog Beispiel 1 b aus (2S,4R)-4-[(5-Chlor-thiophen-2-carbonyl)-amino]-2-dimethylcarbamoyl-pyn-olidin-1-carbonsäure-tert.-butylester und (R)-2,3-Dimethyl-1,2,3,4-tetrahydroisochinolin-6-carbonsäure (als Hydrochlorid-Salz) mit HATU als Kupplungsreagenz.
Rₜ-Wert: 1.10 min (Methode B)
C₂₄H₂₉CIN₄O₃S x CF₃CO₂H (489.04)
Massenspektrum: (M+H)⁺ = 489/491 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **30** | 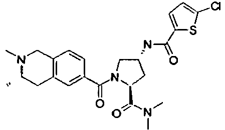 | (M+H)⁺ = 489/491 (Chlorisotope) | Rₜ-Wert: 1.16 min (Methode B) |
| | (2S,4R)-4-[(5-Chlor-thiophen-2-carbonyl)-amino]-1-[(3S)-2,3-dimethyl-1,2,3,4-tetrahydroisochinolin-6-carbonyl]-pyrrolidin-2-carbonsäure-dimethylamid (als Trifluoracetat-Salz) | | |

### Beispiel 32

### (3SR,4SR)-5-Chlor-thiophen-2-carbonsäure-[4-benzyloxy-1-(2-methyl-1,2,3,4-tetrahydro-isochinolin-6-carbonyl)-pyrrolidin-3-yl]-amid (als Trifluoracetat-Salz)

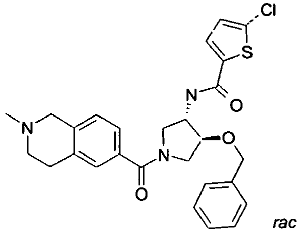

### (a) (3SR,4SR)-3-Azido-4-hydroxy-pyrrolidin-1-carbonsäure-tert.-butylester

1.7 g (9.2 mmol) rac-6-Oxa-3-aza-bicyclo[3.1.0]hexan-3-carbonsäure-tert.-butylester (hergestellt analog Y. Tsuzuki et al. Tetrahedron Asymm. 2001, 12, 2989) werden in einem Gemisch aus 16 ml 1,4-Dioxan und 3 ml Wasser gelöst, mit 1.8 g (27.5 mmol) Natriumazid versetzt und 20 Stunden bei 100°C gerührt. Anschließend wird die Reaktionsmischung abgekühlt, mit Wasser versetzt und dreimal mit Ethylacetat extrahiert. Die vereinigten organischen Phasen werden mit ges. Natriumchlorid-Lösung gewaschen und, mit Aktivkohle versetzt, über Natriumsulfat getrocknet und i. Vak. eingeengt.
R_{f}-Wert: 0.80 (Kieselgel, Fließmittel DCM/MeOH 10:1))
C₉H₁₆N₄O₃ (228.25)
Massenspektrum: (M+H)⁺ = 229

### (b) (3SR,4SR)-3-Amino-4-hydroxy-pyrrolidin-1-carbonsäure-tert.-butylester

Hergestellt analog Beispiel 14 c aus (3SR,4SR)-3-Azido-4-hydroxy-pyrrolidin-1-carbonsäure-tert.-butylester.
Rₜ-Wert: 0.42 min (Methode C)
C₉H₁₈N₂O₃ (202.25)
Massenspektrum: (M+H)⁺ = 203

### (c) (3SR,4SR)-3-[(5-Chlor-thiophen-2-carbonyl)-amino]-4-hydroxy-pyrrolidin-1-carbonsäure-tert.-butylester

201 mg (1.2 mmol) 5-Chlorthiophen-2-carbonsäure und 436 mg (1.4 mmol) TBTU werden in 5 ml DCM suspendiert und mit 260 µl (1.9 mmol) TEA versetzt. Man rührt die Mischung 30 Minuten und gibt dann eine Lösung von 250 mg (1.2 mmol) (3SR,4SR)-3-Amino-4-hydroxy-pyrrolidin-1-carbonsäure-tert.-butylester in 5 ml DCM hinzu und rührt 16 Stunden bei RT. Anschließend gießt man das Reaktionsgemisch in Wasser und extrahiert mit DCM. Die organische Phase wird mit ges. Natriumchlorid-Lösung gewaschen und, mit Aktivkohle versetzt, über Natriumsulfat getrocknet. Man filtriert und engt das Filtrat zur Trockene ein. Der so erhaltene Rückstand wird säulenchromatographisch gereinigt (Silicagel, Fließmittel DCM/MeOH 10:1).
Rₜ-Wert: 0.70 min (Methode C)
C₁₄H₁₉CIN₂O₄S (346.83)
Massenspektrum: (M+H)⁺ = 347/349 (Chlorisotope)

### (d) (3SR,4SR)-3-Benzyloxy-4-[(5-Chlor-thiophen-2-carbonyl)-amino]-pyrrolidin-1-carbonsäure-tert.-butylester

67 mg (193 µmol) (3SR,4SR)-3-[(5-Chlor-thiophen-2-carbonyl)-amino]-4-hydroxy-pyrrolidin-1-carbonsäure-tert.-butylester werden in 500 µl DMF gelöst und nacheinander mit 15 mg (367 µmol) Natriumhydrid (60%ige Dispersion in Mineralöl) und 25 µl (212 µmol) Benzylbromid versetzt. Nach zwei Stunden werden weitere 15 mg Natriumhydrid-Dispersion zugesetzt und die Reaktionsmischung 16 Stunden bei RT gerührt. Anschließend wird das Gemisch auf Wasser gegossen und mit DCM extrahiert. Die organische Phase wird über Natriumsulfat getrocknet, filtriert und i. Vak. eingeengt. Der so erhaltene Rückstand wird säulenchromatographisch an Silicagel (Fließmittel Petrolether/Ethylacetat 3:1) gereinigt
Rₜ-Wert: 1.80 min (Methode B)
C₂₁H₂₅CIN₂O₄S (436.95
Massenspektrum: (M+H)⁺ = 437/439 (Chlorisotope)

### (e) (3SR,4SR)-5-Chlor-thiophen-2-carbonsäure-[4-benzyloxy-1-(2-methyl-1,2,3,4-tetrahydro-isochinolin-6-carbonyl)-pyrrolidin-3-yl]-amid (als Trifluoracetat-Salz)

Hergestellt analog Beispiel 1 b aus (3SR,4SR)-3-[(5-Chlor-thiophen-2-carbonyl)-amino]-4-hydroxy-pyrrolidin-1-carbonsäure-tert.-butylester.
Rₜ-Wert: 1.42 min (Methode B)
C₂₇H₂₈CIN₃O₃S (510.06)
Massenspektrum: (M+H)⁺ = 510/512 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **27** | 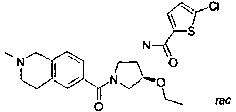 | (M+H)⁺ = 448/450 (Chlorisotope) | Rₜ-Wert: 1.21 min (Methode B) |
| | (3SR,4SR)-5-Chlor-thiophen-2-carbonsäure-[4-ethoxy-1-(3-methyl-1,2,3,4-tetrahydro-isochinolin-6-carbonyl)-pyrrolidin-3-yl]-amid (als Trifluoracetat-Salz) | | |
| **31** | 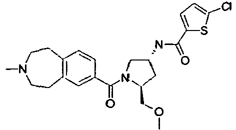 | (M+H)⁺ = 462/464 (Chlorisotope) | Rₜ-Wert: 1.16 min (Methode B) |
| | (2S,4R)-5-Chlor-thiophen-2-carbonsäure-[5-methoxymethyl-1-(3-methyl-2,3,4,5-tetrahydro-1H-benzo[d]azepin-7-carbonyl)-pyrrolidin-3-yl]-amid (als Trifluoracetat-Salz) | | |
| **74** | 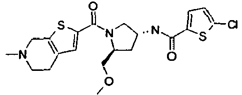 | (M+H)⁺ = 455/456 (Chlorisotope) | Rₜ-Wert = 1.14 min (Methode F) |
| | (3*R*,5*S*)-5-Chlor-thiophen-2-carbonsäure-{5-methoxymethyl-1-[6-methyl-4,5,6,7,-tetrahydro-thieno[2,3-c]pyridin-2-carbonyl]-pyrrolidin-3-yl}-amid (als Trifluoracetat-Salz) | | |

### Beispiel 39

### (R)-5-Ethinyl-thiophen-2-carbonsäure-[1-(2-methyl-1,2,3,4-tetrahydroisochinolin-6-carbonyl)-pyrrolidin-3-yl]-amid (als Trifluoracetat-Salz)

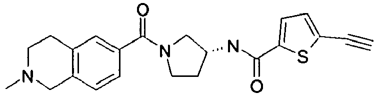

### (a) (R)-[1-(2-Methyl-1 ,2,3,4-tetrahydroisochinolin-6-carbonyl)-pyrrolidin-3-yl]-carbaminsäure-tert.-butylester

1.1 g (4.8 mmol) 2-Methyl-1,2,3,4-tetrahydroisochinolin-6-carbonsäure (als Hydrochlorid) werden in 12 ml DMF gelöst, mit 2.1 ml (19.3 mmol) NMM und 1.8 g (4.8 mmol) HATU versetzt und fünf Minuten bei RT gerührt. Dann werden 0.9 g (4.8 mmol) (R)-Pyrrolidin-3-yl-carbaminsäure-tert.-butylester zugegeben und die Mischung für 16 Stunden gerührt. Anschließend wird das Gemisch auf Wasser gegossen und mit DCM extrahiert. Man trennt die organische Phase über eine Phasentrennkartusche ab und engt i. Vak. zur Trockene ein. Der Rückstand wird mittels Flashchromatographie an Silicagel (Fließmittel DCM/MeOH 9:1 bis 8:2) gereinigt.
Rₜ-Wert: 1.00 min (Methode B)
C₂₀H₂₉N₃O₃ (330.83)
Massenspektrum: (M+H)⁺ =360

### (b) (R)-(3-Amino-pyrrolidin-1-yl)-(2-methyl-1,2,3,4-tetrahydroisochinolin-6-yl)-methanon

1.4 g (3.8 mmol) (R)-[1-(2-Methyl-1,2,3,4-tetrahydroisochinolin-6-carbonyl)-pyrrolidin-3-yl]-carbaminsäure-tert.-butylester werden in 5 ml THF gelöst und langsam mit 9.5 ml Salzsäure (4 M in 1,4-Dioxan) versetzt. Man rührt für zwei Stunden, engt dann die Mischung auf 2/3 des Volumens ein und filtriert das Rohprodukt als Niederschlag ab, das anschießend mittels RP-HPLC (Fließmittel: Gradient Ammoniak/Acetonitril) gereinigt wird.
Rₜ-Wert: 0.42 min (Methode C)
C₁₅H₂₁N₃O (259.35)
Massenspektrum: (M+H)⁺ = 260

### (c) (R)-5-Ethinyl-thiophen-2-carbonsäure-[1-(2-methyl-1 ,2,3,4-tetrahydroisochinolin-6-carbonyl)-pyrrolidin-3-yl]-amid (als Trifluoracetat-Salz)

Hergestellt analog Beispiel 39 a aus (R)-(3-Amino-pyrrolidin-1-yl)-(2-methyl-1,2,3,4-tetrahydroisochinolin-6-yl)-methanon und 5-Ethinyl-thiophen-2-carbonsäure.
Rₜ-Wert: 1.06 min (Methode B)
C₂₂H₂₃N₃O₂S x CF₃CO₂H (393.51)
Massenspektrum: (M+H)⁺ = 394

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **36** | 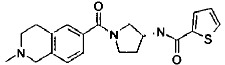 | (M+H)⁺ = 370 | Rₜ-Wert = 0.91 min (Methode B) |
| | (R)-Thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **37** | 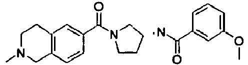 | (M+H)⁺ = 394 | Rₜ-Wert = 0.97 min (Methode B) |
| | (R)-3-Methoxy-N-[1-(2-methyl-1,2,3,4-tetrahydroisochinolin-6-carbonyl)-pyrrolidin-3-yl]-benzamid (als Trifluoracetat-Salz) | | |
| **38** | 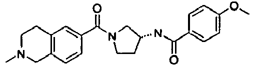 | (M+H)⁺ = 394 | Rₜ-Wert = 0.96 min (Methode B) |
| | (R)-4-Methoxy-N-[1-(2-methyl-1,2,3,4-tetrahydroisochinolin-6-carbonyl)-pyrrolidin-3-yl]-benzamid (als Trifluoracetat-Salz) | | |
| **40** | 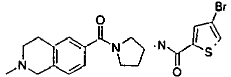 | (M+H)⁺ = 448/450 (Bromisotope) | Rₜ-Wert = 1.08 min (Methode B) |
| | (R)-4-Brom-thiophen-2-carbonsäure-[1-(2-methyl-1 ,2,3,4-tetrahydro-isochinolin-6-carbonyl)-pyrrolidin-3-yl]-amid (als Trifluoracetat-Salz) | | |
| **41** | 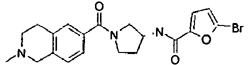 | (M+H)⁺ = 432/434 (Bromisotope) | Rₜ-Wert = 0.96 min (Methode B) |
| | (R)-5-Brom-furan-2-carbonsäure-[1-(2-methyl-1,2,3,4-tetrahydro-isochinolin-6-carbonyl)-pyrrolidin-3-yl]-amid (als Trifluoracetat-Salz) | | |
| **42** | 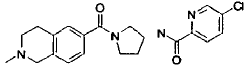 | (M+H)⁺ = 399/401 (Chlorisotope) | Rₜ-Wert = 1.01 min (Methode B) |
| | (R)-5-Chlor-pyridin-2-carbonsäure-[1-(2-methyl-1 ,2,3,4-tetrahydroisochinolin-6-carbonyl)-pyrrolidin-3-yl]-amid (als Bis-Trifluoracetat-Salz) | | |
| **43** | 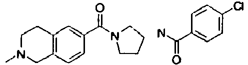 | (M+H)⁺ = 398/400 (Chlorisotope) | Rₜ-Wert = 1.01 min (Methode B) |
| | (R)-4-Chlor-N-[1-(2-methyl-1,2,3,4-tetrahydroisochinolin-6-carbonyl)-pyrrolidin-3-yl]-benzamid (als Trifluoracetat-Salz) | | |
| **44** | 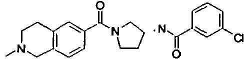 | (M+H)⁺ = 398/400 (Chlorisotope) | Rₜ-Wert = 1.01 min (Methode B) |
| | (R)-3-Chlor-N-[1-(2-methyl-1,2,3,4-tetrahydroisochinolin-6-carbonyl)-pyrrolidin-3-yl]-benzamid (als Trifluoracetat-Salz) | | |

### Beispiel 45

### (3SR,4SR)-5-Chlor-thiophen-2-carbonsäure-[4-methyl-1-(2-methyl-1,2,3,4-tetrahydroisochinolin-6-carbonyl)-pyn-olidin-3-yl]-amid (als Trifluoracetat-Salz)

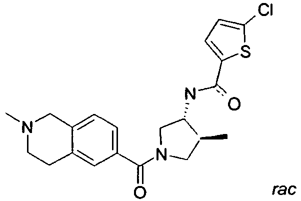

### (a) (3RS,4RS)-1-Benzyl-4-methyl-pyrrolidin-3-carbonsäure-methylester (als Trifluoracetat-Salz)

830 µl (7.8 mmol) Crotonsäuremethylester werden in 25 ml DCM gelöst und mit 61 µl (0.79 mmol) TFA versetzt. Dann wird eine Lösung aus 2,0 ml (7.8 mmol) N-Methoxymethyl-N-trimethylsilylmethyl-benzylamin in 5 ml DCM innerhalb von 20 Minuten zugetropft. Die Reaktionsmischung wird 16 Stunden gerührt und dann i. Vak. eingeengt. Der Rückstand wird mittels RP-HPLC gereinigt.
Rₜ-Wert: 0.94 min (Methode B)
C₁₄H₁₉NO₂ (233.31)
Massenspektrum: (M+H)⁺ = 234

### (b) (3RS,4RS)-1-Benzyl-4-methyl-pyrrolidin-3-carbonsäure (als Hydrochlorid-Salz)

2.0 g (5.7 mmol) (3RS,4RS)-1-Benzyl-4-methyl-pyn-olidin-3-carbonsäure-methylester (als Trifluoracetat-Salz) werden in 4 ml Methanol gelöst und mit 5 ml Lithiumhydroxid-Lösung (8%ig in Wasser) versetzt. Die Mischung wird fünf Stunden bei RT gerührt, dann mit 3.2 ml 4N Salzsäure versetzt und zur Trockene eingeengt.
Rₜ-Wert: 0.83 min (Methode B)
C₁₃H₁₇NO₂ (219.29)
Massenspektrum: (M+H)⁺ =220

### (c) (3RS,4RS)-1-Benzyl-4-methyl-pyrrolidin-3-carbonsäure-methylester

1.3 g (4.9 mmol) (3RS,4RS)-1-Benzyl-4-methyl-pyrrolidin-3-carbonsäure (als Hydrochlorid-Salz) werden in 10 ml Methanol gelöst und unter Eisbad-Kühlung mit 0.6 ml (8.5 mmol) Thionylchlorid versetzt. Das Eisbad wird entfernt und die Mischung für drei Stunden unter Rückfluß erhitzt. Anschließend wird die Mischung i. Vak. eingeengt und mit 1 N Natronlauge versetzt. Die wässrige Phase wird dreimal mit Ethylacetat extrahiert. Die vereinigten organischen Phasen werden über Natriumsulfat getrocknet, filtriert und i. Vak. eingeengt.
Rₜ-Wert: 1.00 min (Methode B)
C₁₄H₁₉NO₂ (233.31)
Massenspektrum: (M+H)⁺ =234

### (d) (3RS,4RS)-4-Methyl-pyrrolidin-3-carbonsäure-methylester (als Hydrochlorid-Salz)

927 mg (4 mmol) (3RS,4RS)-1-Benzyl-4-methyl-pyrrolidin-3-carbonsäure-methylester werden in 15 ml Methanol gelöst, mit 100 mg Palladium/Kohle (10%ig) und 2 ml 2N Salzsäure versetzt und 14 Stunden mit 3 bar Wasserstoff hydriert. Anschließend wird die Mischung filtriert und i. Vak. eingeengt.
Rₜ-Wert: 0.39 min (Methode B)
C₇H₁₃NO₂ x HCl (143.19)
Massenspektrum: (M+H)⁺ = 144

### (e) (3RS,4RS)-4-Methyl-1 -(2-methyl-1,2,3,4-tetrahydroisochinolin-6-carbonyl)-pyrrolidin-3-carbonsäure (als Trifluoracetat-Salz)

880 mg (3.9 mmol) 2-Methyl-1,2,3,4-tetrahydroisochinolin-6-carbonsäure (als Hydrochlorid-Salz) werden in 2 ml DMF gelöst und mit 1,5 g (3.9 mmol) HATU und 1,7 ml (15.6 mmol) NMM versetzt. Man rührt fünf Minuten bei RT und gibt dann eine Lösung von 700 mg (3.9 mmol) (3RS,4RS)-4-Methyl-pyrrolidin-3-carbonsäure-methylester (als Hydrochlorid-Salz) in 2 ml DMF zu. Die Reaktionsmischung wird 16 Stunden bei RT gerührt, dann mit 2N Natronlauge versetzt und dreimal mit Ethylacetat ausgeschüttelt. Die wässrige Phase wird mit TFA sauer gestellt und mittels RP-HPLC gereinigt. Man erhält die Carbonsäure als Produkt.
Rₜ-Wert: 0.74 min (Methode B)
C₁₇H₂₂N₂O₃ x CF₃CO₂H (302.38)
Massenspektrum: (M+H)⁺ = 303

### (f) (3RS,4SR)-(3-Amino-4-methyl-pyrrolidin-1 -yl)-(2-methyl-1,2,3,4-tetrahydroisochinolin-6-yl)-methanon

72 mg (173 µmol) (3RS,4RS)-4-Methyl-1-(2-methyl-1,2,3,4-tetrahydroisochinolin-6-carbonyl)-pyrrolidin-3-carbonsäure (als Trifluoracetat-Salz) werden in 4 ml tert.-Butanol gelöst und mit 95 µl (682 µmol) TEA versetzt. Dann gibt man 150 µl (675 µmol) Diphenylphosphorsäureazid hinzu und rührt für zwei Stunden unter Rückfluß. Die Reaktionsmischung wird dann mit 2N Natronlauge versetzt und dreimal mit Ethylacetat ausgeschüttelt. Die wässrige Phase wird i. Vak. auf 6 ml Volumen konzentriert und mittels RP-HPLC (Fließmittel: Ammoniak/Acetonitril) gereinigt. Man erhält das Amin als Produkt.
Rₜ-Wert: 0.46 min (Methode C)
C₁₆H₂₃N₃O (273.37)
Massenspektrum: (M+H)⁺ = 274

### (g) (3SR,4SR)-5-Chlor-thiophen-2-carbonsäure-[4-methyl-1-(2-methyl-1,2,3,4-tetrahydroisochinolin-6-carbonyl)-pyrrolidin-3-yl]-amid (als Trifluoracetat-Salz)

Hergestellt analog Beispiel 1 a aus (3RS,4RS)-(3-Amino-4-methyl-pyrrolidin-1 - yl)-(2-methyl-1,2,3,4-tetrahydroisochinolin-6-yl)-methanon.
Rₜ-Wert: 1.18 min (Methode B)
C₂₁H₂₄CIN₃O₂S x CF₃CO₂H (417.96)
Massenspektrum: (M+H)⁺ = 418/420 (Chlorisotope)

### Beispiel 46

### (2S,4R)-4-[(5-Chlor-thiophen-2-carbonyl)-amino]-1-(2-methyl-2,3-dihydro-1H-isoindol-5-carbonyl)-pyrrolidin-2-carbonsäure-methylester (als Trifluoracetat-Salz)

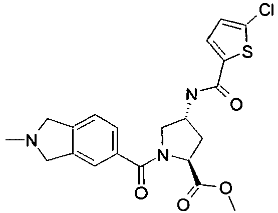

### (a) 2,3-Dihydro-1H-isoindol-5-carbonsäure-methylester

Herstellung analog Beispiel 45 c aus 2,3-Dihydro-1H-isoindol-5-carbonsäure (als Hydrochlorid-Salz; hergestellt analog EP 0 528 369).
Rₜ-Wert: 0.49 min (Methode D)
C₁₀H₁₁NO₂ (177.20)
Massenspektrum: (M+H)⁺ = 178

### (b) 2-Methyl-2,3-dihydro-1H-isoindol-5-carbonsäure-methylester

1,2 g (6.6 mmol) 2,3-Dihydro-1H-isoindol-5-carbonsäure-methylester werden in 5 ml Ameisensäure gelöst , mit 2 ml Formalin-Lösung (37%ige Lösung in Wasser) versetzt, für 3,5 Stunden auf 70°C erhitzt und für 16 Stunden bei RT gerührt. Das Reaktionsgemisch wird i. Vak. eingeengt und mit 0.1 N Natronlauge versetzt und dreimal mit Ethylacetat ausgeschüttelt. Die vereinigten organischen Phasen werden über Natriumsulfat getrocknet, filtriert und i. Vak. eingeengt.
Rₜ-Wert: 0.60 min (Methode E)
C₁₁H₁₃NO₂ (191.23)
Massenspektrum: (M+H)⁺ = 192

### (c) 2-Methyl-2,3-dihydro-1H-isoindol-5-carbonsäure (als Hydrochlorid-Salz)

Herstellung analog Beispiel 26 g aus 2-Methyl-2,3-dihydro-1H-isoindol-5-carbonsäure-methylester.
Rₜ-Wert: 0.25 min (Methode B)
C₁₀H₁₁NO₂ x HCl (177.20)
Massenspektrum: (M+H)⁺ = 178

### (d) (2S,4R)-4-[(5-Chlor-thiophen-2-carbonyl)-amino]-1-(2-methyl-2,3-dihydro-1H-isoindol-5-carbonyl)-pyrrolidin-2-carbonsäure-methylester (als Trifluoracetat-Salz)

Herstellung analog Beispiel 1 b aus (2S,4R)-4-[(5-Chlor-thiophen-2-carbonyl)-amino]-pyrrolidin-1,2-dicarbonsäure-1-tert.-butylester-2-methylester und 2-Methyl-2,3-dihydro-1H-isoindol-5-carbonsäure (als Hydrochlorid-Salz).
Rₜ-Wert: 1.14 min (Methode B)
C₂₁H₂₂ClN₃O₄S x CF₃CO₂H (447.94)
Massenspektrum: (M+H)⁺ = 448/450 (Chlorisotope)

### Beispiel 35

### (R)-5-Chlor-thiophen-2-carbonsäure-[1-(6-methyl-5,6,7,8-tetrahydro-4H-thieno[2,3-d]azepin-2-carbonyl)-pyrrolidin-3-yl]-amid (als Trifluoracetat-Salz)

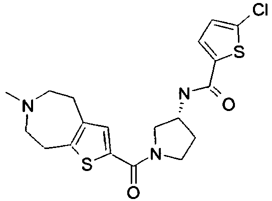

### (a) 5,6,7,8-Tetrahydro-4H-thieno[2,3-d]azepin-2-carbonsäure-methylester (als Hydrochlorid)

Herstellung analog DE 3105858 aus 6-(3-Chlor-benzyl)-5,6,7,8-tetrahydro-4H-thieno[2,3-d]azepin-2-carbonsäure-methylester.
Rₜ-Wert: 0.85 min (Methode B)
C₁₀H₁₃NO₂S x HCl (211.28)
Massenspektrum: (M+H)⁺ = 212

### (b) 6-Methyl-5,6,7,8-Tetrahydro-4H-thieno[2,3-d]azepin-2-carbonsäure-methylester

Herstellung analog Beispiel 46 b aus 5,6,7,8-Tetrahydro-4H-thieno[2,3-d]azepin-2-carbonsäure-methylester (als Hydrochlorid).
Rₜ-Wert: 0.64 min (Methode E)
C₁₁H₁₅NO₂S (225.31)
Massenspektrum: (M+H)⁺ = 226

### (c) 6-Methyl-5,6,7,8-Tetrahydro-4H-thieno[2,3-d]azepin-2-carbonsäure (als Hydrochlorid)

Herstellung analog Beispiel 45 b aus 5,6,7,8-Tetrahydro-4H-thieno[2,3-d]azepin-2-carbonsäure-methylester (als Hydrochlorid).
Ausbeute: quantitativ
C₁₀H₁₃NO₂S x HCl (211.28)
Massenspektrum: (M+H)⁺ = 212

### (d) (R)-5-Chlor-thiophen-2-carbonsäure-[1-(6-methyl-5,6,7,8-tetrahydro-4H-thieno[2,3-d]azepin-2-carbonyl)-pyrrolidin-3-yl]-amid (als Trifluoracetat-Salz)

Herstellung analog Beispiel 1 b aus(R)-3-[(5-Chlor-thiophen-2-carbonyl)-amino]-pyrrolidin-1-carbonsäure-tert.-butylester und 6-Methyl-5,6,7,8-Tetrahydro-4H-thieno[2,3-d]azepin-2-carbonsäure (als Hydrochlorid).
Rₜ-Wert: 1.15 min (Methode B)
C₁₉H₂₂ClN₃O₂S₂ x CF₃CO₂H (423.99)
Massenspektrum: (M+H)⁺ = 424/426 (Chlorisotope)

### Beispiel 47

### (3SR,4SR)-5-Chlor-thiophen-2-carbonsäure-[4-methoxy-1-(5-methyl-4,5,6,7-tetrahydro-thieno[3,2-c]pyridin-2-carbonyl)-pyrrolidin-3-yl]-amid (als Trifluoracetat-Salz)

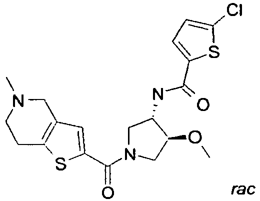

### (a) 6,7-Dihydro-4H-thieno[3,2-c]pyridin-2,5-dicarbonsäure-5-tert.-butylester-2-ethylester

1.5 ml (20.1 mmol) DMF bei 0°C langsam mit 1.5 ml (16.1 mmol) Phosphoroxychlorid versetzt. Anschließend nimmt man die Mischung in 10 ml DCM auf und rührt für 45 Minuten bei RT. Dann werden 2.2 g (9.8 mmol) 4-Oxo-piperidin-1-carbonsäure-tert.-butylester, gelöst in 10 ml DCM, zur Mischung bei 0-5°C zugetropft. Es werden weitere 10 ml DCM zugegeben und für eine Stunde bei RT gerührt. Die Reaktionsmischung wird dann auf ein Gemisch aus Eis und 20 ml gesättiger Natriumacetat-Lösung gegossen und für eine Stunde gerührt. Die organische Phase wird abgetrennt, mehrfach mit Wasser gewaschen und dann über Natriumsulfat getrocknet und i. Vak. eingeengt.

Das so erhaltene Rohprodukt wird in 15 ml DCM gelöst und mit einem Gemisch aus 1.8 ml (16.0 mmol) Mercaptoessigsäure-ethylester und 2.8 ml (19.9 mmol) TEA in 5 ml DCM versetzt. Anschließend wird das Reaktionsgemisch für 2,5 Stunden unter Rückfluß erhitzt und dann eine Stunde bei RT nachgerührt. Man gibt Wasser zu, trennt die organische Phase ab und wäscht mit viel Wasser nach. Die organische Phase wird über Natriumsulfat getrocknet, filtriert und i. Vak. eingeengt. Der Rückstand wird mittels Flashchromatographie an Silicagel (Fließmittelgemisch Cyclohexan/Ethylacetat 9:1 bis 8:2) gereinigt.
Rₜ-Wert: 1.71 min (Methode B)
C₁₅H₂₁NO₄S (311.40)
Massenspektrum: (M+H)⁺ = 312

### (b) 5-Methyl-4,5,6,7-tetrahydro-thieno[3,2-c]pyridin-2-carbonsäure-ethylester (als Trifluoracetat-Salz)

615 mg (987 µmol, 50%ige Reinheit) 6,7-Dihydro-4H-thieno[3,2-c]pyridin-2,5-dicarbonsäure-5-tert.-butylester-2-ethylester werden in 4 ml eines Gemisches aus TFA und DCM (v/v 1:1) gelöst und für 30 Minuten bei RT gerührt. Man neutralisiert das Reaktionsgemisch unter Zugabe von TEA und engt i. Vak. ein. Das so erhaltene Rohprodukt wird in 4 ml Ameisensäure gelöst und mit 0.5 ml (6.7 mmol) Formalin-Lösung (37%ig in Wasser) versetzt. Die Reaktionsmischung wird für 16 Stunden bei 70°C gerührt. Nach Abkühlen auf RT wird das Gemisch mit 50%iger wässriger Natronlauge und gesättigter Natriumhydrogencarbonat-Lösung basisch gestellt und mit Ethylacetat extrahiert. Die organische Phase wird dreimal mit Wasser gewaschen, über Natriumsulfat getrocknet, filtriert und i. Vak. eingeengt. Der Rückstand wird mittel RP-HPLC gereinigt.
Rₜ-Wert: 0.90 min (Methode B)
C₁₁H₁₅NO₂S x CF₃CO₂H (225.31)
Massenspektrum: (M+H)⁺ = 226

### (c) 5-Methyl-4,5,6,7-tetrahydro-thieno[3,2-c]pyridin-2-carbonsäure (als Hydrochlorid-Salz)

Herstellung analog Beispiel 45 b aus 5-Methyl-4,5,6,7-tetrahydro-thieno[3,2-c]pyridin-2-carbonsäure-ethylester (als Trifluoracetat-Salz).
Rₜ-Wert: 0.29 min (Methode B)
C₉H₁₁NO₂S x HCl (197.26)
Massenspektrum: (M+H)⁺ = 198

### (d) (3SR,4SR)-5-Chlor-thiophen-2-carbonsäure-[4-methoxy-1-(5-methyl-4,5,6,7-tetrahydro-thieno[3,2-c]pyridin-2-carbonyl)-pyrrolidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 5-Methyl-4,5,6,7-tetrahydro-thieno[3,2-c]pyridin-2-carbonsäure (als Hydrochlorid-Salz).
Rₜ-Wert: 1.17 min (Methode B)
C₁₉H₂₂ClN₃O₃S₂ x CF₃CO₂H (439.99)
Massenspektrum: (M+H)⁺ = 440/442 (Chlorisotope)

### Beispiel 48

### (3SR,4SR)-5-Chlor-thiophen-2-carbonsäure-[4-methoxy-1-(6-methyl-4,5,6,7-tetrahydro-thieno[2,3-c]pyridin-2-carbonyl)-pyrrolidin-3-yl]-amid (als Trifluoracetat-Salz)

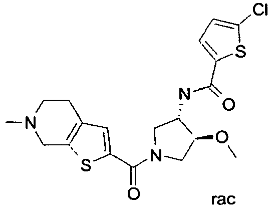

### (a) 6-Methyl-4,5,6,7-tetrahydro-thieno[2,3-c]pyridin-2-carbonsäure-methylester (als Trifluoracetat-Salz)

496 mg (2.1 mmol) 2-Brom-6-methyl-4,5,6,7-tetrahydro-thieno[2,3-c]pyridin (hergestellt analog EP 0314154 aus 6-Methyl-4,5,6,7-tetrahydro-thieno[2,3-c]pyridin) werden in 10 ml THF gelöst und auf -78°C gekühlt. Dann werden 2 ml (3.2 mmol) n-Butyllithium-Lösung (1.6 M in n-Hexan) langsam zugetropft. Das Reaktionsgemisch wird 30 Minuten bei -78°C gerührt und dann mit 1.0 ml (12.9 mmol) Chlorameisensäure-methylester versetzt. Man rührt fünf Minuten bei - 78°C, erwärmt auf RT und engt die Mischung i. Vak. ein. Der Rückstand wird mittels RP-HPLC gereinigt.
Rₜ-Wert: 0.79 min (Methode B)
C₁₀H₁₃NO₂S x CF₃CO₂H (211.28)
Massenspektrum: (M+H)⁺ = 212

### (b) 6-Methyl-4,5,6,7-tetrahydro-thieno[2,3-c]pyridin-2-carbonsäure (als Hydrochlorid-Salz)

Herstellung analog Beispiel 45 b aus 6-Methyl-4,5,6,7-tetrahydro-thieno[2,3-c]pyridin-2-carbonsäure-methylester (als Trifluoracetat-Salz).
Rₜ-Wert: 0.22 min (Methode B)
C₉H₁₁NO₂S x HCl (197.26)
Massenspektrum: (M+H)⁺ = 198

### (c) (3SR,4SR)-5-Chlor-thiophen-2-carbonsäure-[4-methoxy-1-(6-methyl-4,5,6,7-tetrahydro-thieno[2,3-c]pyridin-2-carbonyl)-pyn-olidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 6-Methyl-4,5,6,7-tetrahydro-thieno[2,3-c]pyridin-2-carbonsäure (als Hydrochlorid-Salz).
Rₜ-Wert: 1.16 min (Methode B)
C₁₉H₂₂ClN₃O₃S₂ x CF₃CO₂H (439.99)
Massenspektrum: (M+H)⁺ = 440/442 (Chlorisotope)

### Beispiel 49

### (3SR,4SR)-5-Chlor-thiophen-2-carbonsäure-[4-methoxy-1-(5-methyl-4,5,6,7-tetrahydro-thiazolo[5,4-c]pyridin-2-carbonyl)-pyrrolidin-3-yl]-amid, (als Trifluoracetat-Salz)

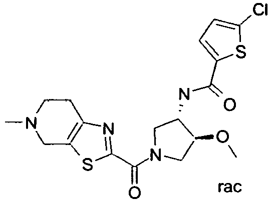

Herstellung analog Beispiel 26 h aus (3SR,4SR)-3-[(5-Chlor-thiophen-2-carbonyl)-amino]-4-methoxy-pyrrolidin und 5-Methyl-4,5,6,7-tetrahydrothiazolo[5,4-c]pyridin-2-carbonsäure (Heterocycles, 63, 2004, 1555-1562).
Rₜ-Wert: 1.17 min (Methode B)
C₁₈H₂₁ClN₄O₃S₂ x CF₃CO₂H (440.97)
Massenspektrum: (M+H)⁺ = 441/443 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **69** | 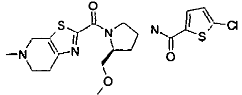 | (M+H)⁺ = 455/457 (Chlorisotope) | Rₜ-Wert = 1. 13 min (Methode F) |
| | (3*R*,5*S*)-5-Chlor-thiophen-2-carbonsäure-[5-methoxymethyl-1-(5-methyl-4,5,6,7-tetrahydro-thiazolo[5,4-c]pyridin-2-carbonyl)-5-hydroxymethyl-pyrrolidin-3-yl]-amid (als Trifluoracetat-Salz) | | |

### Beispiel 50

### 2-{(3S,4S)-3-[(5-Chloro-thiophen-2-carbonyl)-amino]-4-methoxy-pyrrolidin-1-carbonyl}-6,7-dihydro-4H-thieno[3,2-c]pyridine-5-carbonsäureethylester

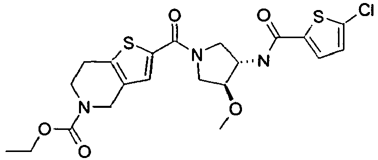

### (a) 6,7-Dihydro-4H-thieno[3,2-c]pyridin-2,5-dicarbonsäure-5-ethylester

1.00 g (4.18 mmol) 2-Formyl-6,7-dihydro-4H-thieno[3,2-c]pyridine-5-carbonsäureethylester werden mit 23 ml tert.Butanol und 1.44 g (25 mmol) im Gefrierschrank abgewogenem Isobutylen versetzt und anschließend werden 17 ml einer wäßrigen Lösung von 3.77 g (31 mmol) Natriumhydrogenphosphat und 3.78 g (41 mmol) Natriumchlorit zugegeben und 2 h gerührt. Das Reaktionsgemisch wird mit NaOH basisch gestellt und mit Essigester extrahiert. Anschließend wird die wäßrige Phase mit HCl acidifiziert und mit Essigester extrahiert. Die vereinigten Essigesterfraktionen werden mit Natriumsulfat getrocknet, konzentriert und der Rückstand wird mittels Flashchromatographie an Silicagel (Fließmittelgemisch Dichlormethan:Methanol 95:5 bis 8:2) gereinigt.
Rₜ-Wert: 1.23 min (Methode B)
C₁₁H₁₃NO₄S (255.29)
Massenspektrum: (M+H)⁺ = 256

### (b) 2-{(3S,4S)-3-[(5-Chloro-thiophen-2-carbonyl)-amino]-4-methoxy-pyrrolidin-1-carbonyl}-6,7-dihydro-4H-thieno[3,2-c]pyridine-5-carbonsäureethylester

Herstellung analog Beispiel 26 h aus (3SR,4SR)-3-[(5-Chlor-thiophen-2-carbonyl)-amino]-4-methoxy-pyrrolidin und 6,7-Dihydro-4H-thieno[3,2-c]pyridin-2,5-dicarbonsäure-5-ethylester.
Rₜ-Wert: 1.4 min (Methode B)
C₂₁H₂₄CIN₃O₅S₂ (498.02)
Massenspektrum: (M+H)⁺ = 4981 /500 (Chlorisotope)

### Beispiel 51

### (3SR,4SR)-5-Chlor-thiophen-2-carbonsäure-[4-methoxy-1-(6-methyl-5,6,7,8-tetrahydro-4H-thieno[2,3-d]azepin-2-carbonyl)-pyrrolidin-3-yi]-amid, (als Trifluoracetat-Salz)

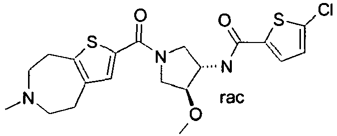

Herstellung analog Beispiel 1 b aus (3SR,4SR)-3-[(5-Chlor-thiophen-2-carbonyl)-amino]-4-methoxy-pyrrolidin und 6-Methyl-5,6,7,8-tetrahydro-4*H-*thieno[2,3-d]azepin-2-carbonsäure (WO2004058715) mit HATU als Kupplungsreagenz.
Rₜ-Wert: 1.09 min (Methode B)
C₂₀H₂₄ClN₃O₃S₂ x CF₃CO₂H (454.01)
Massenspektrum: (M+H)⁺ = 454/456 (Chlorisotope)

### Beispiel 52

### (3SR,4SR)-5-Chlor-th iophen-2-carbonsäure-[4-methoxy-1-(6-methyl-5,6,7,8-tetrahydro-4H-thiazolo[4,5-d]azepin-2-carbonyl)-pyrrolidin-3-yl]-amid

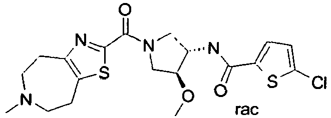

Herstellung analog Beispiel 1 b aus (3SR,4SR)-3-[(5-Chlor-thiophen-2-carbonyl)-amino]-4-methoxy-pyrrolidin und 6-Methyl-5,6,7,8-tetrahydro-4*H-*thiazolo[4,5-d]azepin-2-carbonsäure (WO2004058715) mit HATU als Kupplungsreagenz.
Rₜ-Wert: 1.09 min (Methode F)
C₁₉H₂₃ClN₄O₃S₂ (455.00)
Massenspektrum: (M+H)⁺ = 455/457 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **68** | 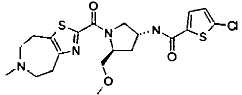 | (M+H)⁺ = 469/471 (Chlorisotope) | Rₜ-Wert = 1.14 min (Methode F) |
| | (3*R*,5*S*)-5-Chlor-thiophen-2-carbonsäure-[5-methoxymethyl-1-(6-methyl-5,6,7,8-tetrahydro-4H-thiazolo[4,5-d]azepin-2-carbonyl)-pyrrolidin-3-yl]-amid (als Trifluoracetat-Salz) | | |

### Beispiel 53

### (3SR,4SR)-5-Chlor-thiophen-2-carbonsäure-[4-methoxy-1-{(4RS)-4,5-dimethyl-4,5,6,7-tetrahydro-thiazolo[5,4-c]pyridin-2-carbonyl}-pyrrolidin-3-yl]-amid

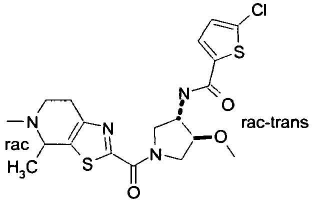

Herstellung analog Beispiel 1 b aus (3SR,4SR)-3-[(5-Chlor-thiophen-2-carbonyl)-amino]-4-methoxy-pyrrolidin und (RS)-4,5-Dimethyl-4,5,6,7-tetrahydro-thiazolo[5,4-c]pyridin-2-carbonsäure (Herstellung analog zu WO2004058728) mit HATU als Kupplungsreagenz.
Rₜ-Wert: 1.15 min (Methode F)
C₁₉H₂₃ClN₄O₃S₂ (455.00)
Massenspektrum: (M+H)⁺ = 455/457 (Chlorisotope)

### Beispiel 54

(3R,5S)-5-Chlor-thiophen-2-carbonsäure-[4-hydroxymethyl-1-(6-methyl-4,5,6,7-tetrahydro-thieno[2,3-c]pyridin-2-carbonyl)-pyn-olidin-3-yl]-amid (als Trifluoracetat-Salz)

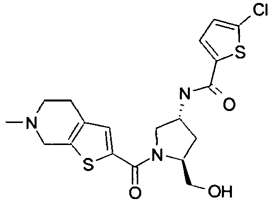

Herstellung analog Beispiel 1 b aus (3R,5S)-5-Chlor-thiophen-2-carbonsäure-(5-hydroxymethyl-pyrrolidin-3-yl)-amid und 6-Methyl-4,5,6,7-tetrahydrothieno[2,3-c]pyridin-2-carbonsäure mit HATU als Kupplungsreagenz.
Rₜ-Wert: 1.02 min (Methode B)
C₁₉H₂₂ClN₃O₃S₂ x CF₃CO₂H (439.99)
Massenspektrum: (M+H)⁺ = 440/442 (Chlorisotope)

### Beispiel 55

### (3R,5S)-5-Chlor-thiophen-2-carbonsäure-[5-methoxymethyl-1-(6-methyl-5,6,7,8-tetrahydro-4H-thieno[2,3-d]azepin-2-carbonyl)-pyrrolidin-3-yl]-amid, (als Trifluoracetat-Salz)

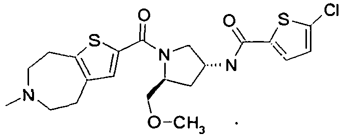

Herstellung analog Beispiel 1 b aus (3R,5S)-5-Chlor-thiophen-2-carbonsäure-(5-methoxymethyl-pyrrolidin-3-yl)-amid und 6-Methyl-5,6,7,8-tetrahydro-4*H-*thieno[2,3-d]azepin-2-carbonsäure (WO2004058715) mit HATU als Kupplungsreagenz.
Rt-Wert: 1.13 min (Methode B)
C₂₁H₂₆ClN₃O₃S₂ x CF₃CO₂H (468.04)
Massenspektrum: (M+H)⁺ = 468/470 (Chlorisotope)

### Beispiel 56

### (3SR,4SR)-5-Chlor-thiophen-2-carbonsäure-[4-methoxy-1-({RS}-6,7-dimethyl-4,5,6,7-tetrahydro-thieno[2,3-c]pyridin-2-carbonyl)-pyrrolidin-3-yl]-amid (als Trifluoracetat-Salz)

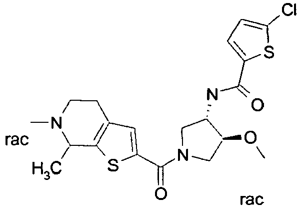

### (a) 6,7-Dimethyl-4,5-dihydro-thieno[2,3-c]pyridiniumiodid

Eine Mischung aus 4.64 g (31 mmol) 7-Methyl-4,5-dihydro-thieno[2,3-c]pyridin (hergestellt analog J.Am.Chem.Soc., 1951, 1257), 8.0 ml (128 mmol) Methyliodid und 20 ml Essigester wird 15 min gerührt. Der entstehende Niederschlag wird abgesaugt, mit Essigester gewaschen und im Vakuum getrocknet.
Rₜ-Wert: 0.47 min (Methode B)
C₉H₁₂NS x I (293.169)
Massenspektrum: (M-I)⁺ = 166

### (b) (RS)-6,7-Dimethyl-4,5,6,7-tetrahydro-thieno[2,3-c]pyridin

Eine Mischung aus 7.2 g (24 mmol) 6,7-Dimethyl-4,5-dihydro-thieno[2,3-c]pyridiniumiodid und 80 ml Methanol wird unter Eisbadkühlung portionsweise mit 1.87 g (49 mmol) NaBH₄ versetzt (starke Gasentwicklung). Es wird 1.5 h nachgerührt, konzentriert, mit ges. NaHCO₃-Lösung versetzt und 3x mit Methylenchlorid extrahiert. Die organischen Phasen werden mit NaSO₄ getrocknet, filtriert und konzentriert.
Rₜ-Wert: 0.66 min (Methode B)
C₉H₁₃NS (167.272)
Massenspektrum: (M+H)⁺ = 168

### (c) (RS)-2-Brom-6,7-Dimethyl-4,5,6,7-tetrahydro-thieno[2,3-c]pyridin

Eine Mischung aus 4.0 g (24 mmol) (RS)-6,7-Dimethyl-4,5,6,7-tetrahydrothieno[2,3-c]pyridin und 25 ml Wasser wird unter Eisbadkühlung portionsweise mit insgesamt 2.23 ml Brom/6.7 g KBr in 15 ml Wasser versetzt. Nach 30 min wird mit Methylenchlorid versetzt, die Wasserphase wird abgetrennt und die organische Phase wird konzentriert. Das Rohprodukt wird ohne weitere Reinigung weiter umgesetzt.
Rₜ-Wert: 0.94 min (Methode B)
C₉H₁₃BrNS x HBr (246.168)
Massenspektrum: (M+H)⁺ = 246/248

### (d) (RS)-6,7-Dimethyl-4,5,6,7-tetrahydro-thieno[2,3-c]pyridin-2-carbonsäure-methylester

Eine Mischung aus 2.0 g (6.1 mmol) (RS)-2-Brom-6,7-Dimethyl-4,5,6,7-tetrahydro-thieno[2,3-c]pyridinxHBr, 2.0 g (24 mmol) Natriumacetat und 100 ml Methanol wird mit 15 mg (0.067 mmol) Palladiumacetat, 150 mg (0.27 mmol) 1,1'-Bis(diphenylphosphino)ferrocen und 2.6 ml (18 mmol) Triethylamin versetzt und 3.5 h bei 80°C unter 5 bar CO-Atmosphäre carbonyliert. Anschließend wird abfiltriert, konzentriert mit ges. NaHCO₃-Lösung und Methylenchlorid versetzt und die resultierende Emulsion über Celite filtriert. Die wäßrige Phase wird abgetrennt und 2x mit Methylenchlorid extrahiert und die vereinigten ordganischen Phsen werden mit Na₂SO₄ getrocknet und konzentriert. Das Rohprodukt wird ohne weitere Reinigung weiter umgesetzt.
Rₜ-Wert: 0.84 min (Methode B)
C₁₁H₁₅NO₂S (225.308)
Massenspektrum: (M+H)⁺ = 226

### (e) (3SR,4SR)-5-Chlor-thiophen-2-carbonsäure-[4-methoxy-1-({RS}-6,7-dimethyl-4,5,6,7-tetrahydro-thieno[2,3-c]pyridin-2-carbonyl)-pyrrolidin-3-yl]-amid (als Trifluoracetat-Salz)

(RS)-6,7-Dimethyl-4,5,6,7-tetrahydro-thieno[2,3-c]pyridin-2-carbonsäure-methylester wird analog Beispiel 6a mit Lithiumhydroxid verseift und anschließend analog Beispiel 1 b mit (3SR,4SR)-3-[(5-Chlor-thiophen-2-carbonyl)-amino]-4-methoxy-pyrrolidin und HATU als Kupplungsreagenz zur Titelverbindung umgesetzt.
Rₜ-Wert: 1.01 min (Methode B)
C₂₀H₂₄ClN₃O₃S₂ x CF₃CO₂H (454.00)
Massenspektrum: (M+H)⁺ = 454/456 (Chlorisotope)

### Beispiel 60

### (3SR,4SR)-5-Chlor-thiophen-2-carbonsäure-{1-[(8SR)-6,8-dimethyl-5,6,7,8-tetrahydro-4H-thieno[2,3-d]azepin-2-carbonyl]-4-methoxy-pyrrolidin-3-yl}-amid (als Trifluoracetat-Salz)

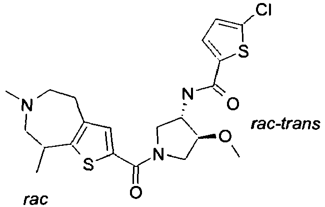

### (a) 6,8-Dimethyl-4,5,7,8-tetrahydro-4H-thieno[2,3-d]azepin

300 mg (7.9 mmol) Lithiumaluminumhydrid werden in 4 ml THF vorgelegt und langsam mit einer Lösung von 300 mg (0.9 mmol) 2-Brom-8-methyl-4,5,7,8-tetrahydro-thieno[2,3-d]azepin-6-carbonsäure-ethyl-ester (hergestellt analog US 2006/0003990) in 6 ml THF bei RT versetzt. Man rührt die Mischung bei RT, bis die Gasentwicklung stoppt und erhitzt dann für 1.5 Stunden auf Rückfluß. Anschließend wird die Mischung im Eis/Wasserbad abgekühlt und mit ges.Natriumsulfat-Lösung versetzt.

Es wird vom Ungelösten abfiltriert, das Filtrat mit 1 N Salzsäure sauer gestellt und zweimal mit Ethylacetat extrahiert. Diese organische Phase wird verworfen. Die wässrige Phase wird basisch gestellt und dreimal mit Ethylacetat extrahiert. Die vereinigten organischen Phasen werden mit Natriumsulfat getrocknet, filtriert und i. Vak. eingeengt.
Rₜ-Wert: 0.88 min (Methode F)
C₁₀H₁₅NS (181.30)
Massenspektrum: (M+H)⁺ = 182

### (b) 2-Brom-6,8-dimethyl-4,5,6,7-tetrahydro-4H-thieno[2,3-d]azepin

156 mg (0.9 mmol) 6,8-Dimethyl-4,5,7,8-tetrahydro-4H-thieno[2,3-d]azepin werden in einer Mischung aus 1.5 ml Eisessig und 1.5 ml Chloroform vorgelegt und mit 155 mg (0.9 mmol) N-Bromsuccinimid versetzt. Man rührt drei Stunden bei RT, gibt dann 20 mg N-Bromsuccinimid zu und rührt eine weitere Stunde bei RT. Anschließend wird die Mischung mit Wasser versetzt und zweimal mit Ethylacetat extrahiert. Das Extrakt wird verworfen. Die wässrige Phase wird alkalisch gestellt und dreimal mit Ethylacetat extrahiert. Die vereinigten organischen Phasen werden über Natriumsulfat getrocknet und i.Vak. eingeengt.
Rₜ-Wert: 1.12 min (Methode F)
C₁₀H₁₄BrNS (260.19)
Massenspektrum: (M+H)⁺ = 260/262 (Bromisotope)

### (c) 6,8-Dimethyl-4,5,6,7-tetrahydro-4H-thieno[2,3-d]azepin-2-carbonsäure-methylester

Hergestellt analog Beispiel 26 e aus 2-Brom-6,8-dimethyl-4,5,6,7-tetrahydro-4H-thieno[2,3-d]azepin.
Rₜ-Wert: 0.94 min (Methode F)
C₁₂H₁₇NO₂S (239.33)
Massenspektrum: (M+H)⁺ = 240

### (d) 6,8-Dimethyl-4,5,6,7-tetrahydro-4H-thieno[2,3-d]azepin-2-carbonsäure

Hergestellt analog Beispiel 45 b aus 6,8-Dimethyl-4,5,6,7-tetrahydro-4H-thieno[2,3-d]azepin-2-carbonsäure-methylester.
Rₜ-Wert: 0.66 min (Methode F)
C₁₁H₁₅NO₂S (225.31)
Massenspektrum: (M+H)⁺ = 226

### (e) (3SR,4SR)-5-Chlor-thiophen-2-carbonsäure-{1-[(8SR)-6,8-dimethyl-5,6,7,8-tetrahydro-4H-thieno[2,3-d]azepin-2-carbonyl]-4-methoxy-pyrrolidin-3-yl}-amid (als Trifluoracetat-Salz)

Hergestellt analog Beispiel 1 b aus 6,8-Dimethyl-4,5,6,7-tetrahydro-4H-thieno[2,3-d]azepin-2-carbonsäure und (3*SR*,4*SR*)-3-[(5-Chlor-thiophen-2-carbonyl)-amino]-4-methoxy-pyrrolidin mit HATU als Kupplungsreagenz.
Rₜ-Wert: 1.14 min (Methode F)
C₂₁H₂₆ClN₃O₂S₂ x CF₃CO₂H (468.04)
Massenspektrum: (M+H)⁺ = 468/470 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **61** | 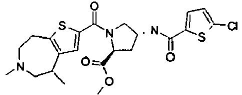 | (M+H)⁺ = 496/498 (Chlorisotope) | Rₜ-Wert = 1.17 min (Methode F) |
| | (2*S*,4*R*)-4-[(5-Chlor-thiophen-2-carbonyl)-amino]-1-(4,6-dimethyl-5,6,7,8-tetrahydro-4H-thieno[2,3-d]azepin-2-carbonyl)-pyrrolidin-2-carbonsäure-methylester (als Trifluoracetat-Salz) | | |
| **65** | 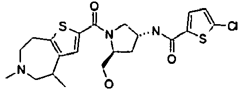 | (M+H)⁺ = 468/470 (Chlorisotope) | Rₜ-Wert = 1.42 min (Methode F) |
| | (3*R*,5*S*)-5-Chlor-thiophen-2-carbonsäure-[1-(4,6-dimethyl-5,6,7,8-tetrahydro-4H-thieno[2,3-d]azepin-2-carbonyl)-5-hydroxymethyl-pyrrolidin.3.yl]-amid (als Trifluoracetat-Salz) | | |
| **67** | 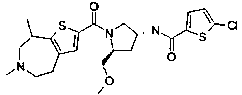 | (M+H)⁺ = 482/484 (Chlorisotope) | Rₜ-Wert = 0.72 min (Methode G) |
| | (3*R*,5*S*)-5-Chlor-thiophen-2-carbonsäure-[1-(6,8-dimethyl-5,6,7,8-tetrahydro-4H-thieno[2,3-d]azepin-2-carbonyl)-5-methoxymethyl-pyrrolidin-3-yl]-amid | | |

### Beispiel 70

### (3R,5S)-5-Chlor-thiophen-2-carbonsäure-{1-[(4SR)-4-methoxy-6-methyl-5,6,7,8-tetrahydro-4H-thieno[2,3-d]azepin-2-carbonyl]-4-methoxy-pyrrolidin-3-yl}-amid (als Trifluoracetat-Salz)

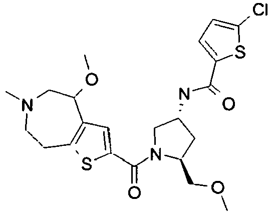

### (a) 4-Methoxy-6-methyl-5,6,7,8-tetrahydro-4H-thieno[2,3-d]azepin

Herstellung analog Beispiel 60 a aus 4-Methoxy-4,5,7,8-tetrahydro-thieno[2,3-d]azepin-6-carbonsäure-ethylester (US 2006/0003990) durch Reduktion mit Lithiumaluminumhydrid.
Rₜ-Wert: 0.97 min (Methode F)
C₁₀H₁₅NOS (197.30)
Massenspektrum: (M+H)⁺ = 198

### (b) 2-Brom-4-methoxy-6-methyl-5,6,7,8-tetrahydro-4H-thieno[2,3-d]azepin

Herstellung analog Beispiel 60 b aus 4-Methoxy-6-methyl-5,6,7,8-tetrahydro-4H-thieno[2,3-d]azepin.
Rₜ-Wert: 1.04 min (Methode F)
C₁₀H₁₄BrNOS (276.19)
Massenspektrum: (M+H)⁺ = 276/278 (Bromisotope)

### (c) 4-Methoxy-6-methyl-5,6,7,8-tetrahydro-4H-thieno[2,3-d]azepin-2-carbonsäuremethylester

Herstellung analog Beispiel 26 e aus 2-Brom-4-methoxy-6-methyl-5,6,7,8-tetrahydro-4H-thieno[2,3-d]azepin.
Rₜ-Wert: 0.87 min (Methode F)
C₁₂H₁₇NO₃S (255.33)
Massenspektrum: (M+H)⁺ = 256

### (d) 4-Methoxy-6-methyl-5,6,7,8-tetrahydro-4H-thieno[2,3-d]azepin-2-carbonsäure (als Hydrochlorid-Salz)

Herstellung analog Beispiel 45 b aus 4-Methoxy-6-methyl-5,6,7,8-tetrahydro-4H-thieno[2,3-d]azepin-2-carbonsäuremethylester.
Rₜ-Wert: 0.55 min (Methode F)
C₁₁H₁₅NO₃S (241.31)
Massenspektrum: (M+H)⁺ = 242

### (e) 4(3R,5S)-5-Chlor-thiophen-2-carbonsäure-{1-[(4SR)-4-methoxy-6-methyl-5,6,7,8-tetrahydro-4H-thieno[2,3-d]azepin-2-carbonyl]-4-methoxy-pyrrolidin-3-yl}-amid (als Trifluoracetat-Salz)

Herstellung analog Beispiel 1 b aus 4-Methoxy-6-methyl-5,6,7,8-tetrahydro-4H-thieno[2,3-d]azepin-2-carbonsäure (als Hydrochlorid-Salz).
Rₜ-Wert: 1.16 min (Methode F)
C₂₂H₂₈ClN₃O₄S₂ (498.07)
Massenspektrum: (M+H)⁺ = 498/500 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **71** | 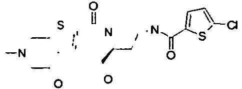 | (M+H)⁺ = 484/486 (Chlorisotope) | Rₜ-Wert = 1.14 min (Methode F) |
| | (3*R*,5*S*)-5-Chlor-thiophen-2-carbonsäure-{5-methoxymethyl-1-[(4*SR*)-4-methoxy-6-methyl-4,5,6,7,-tetrahydro-thieno[2,3-c]pyridin-2-carbonyl]-pyrrolidin-3-yl}-amid (als Trifluoracetat-Salz) | | |
| **72** | 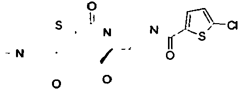 | (M+H)⁺ = 470/472 (Chlorisotope) | Rₜ-Wert = 1.02 min (Methode F) |
| | (3*R*,5*S*)-5-Chlor-thiophen-2-carbonsäure-{5-hydroxymethyl-1-[(4*SR*)-4-methoxy-6-methyl-4,5,6,7,-tetrahydro-thieno[2,3-c]pyridin-2-carbonyl]-pyrrolidin-3-yl}-amid | | |

### Beispiel 73

### (3S,4S)-5-Chlor-thiophen-2-carbonsäure-[1-(5,6-dimethyl-4,5,6,7-tetrahydrothieno[2,3-c]-pyridin-2-carbonyl)-4-methoxy-pyrrolidin-3-yl]-amid (als Trifluoracetat-Salz)

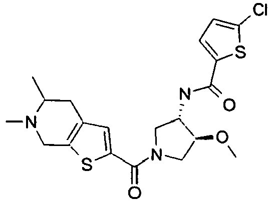

### (a) 2-(2-Nitro-propenyl)-thiophen

5.0 g (44.6 mmol) 3-Formylthiophen werden zusammen mit 8.9 ml (124.8 mmol) Nitroethan, 5.3 ml (53.5 mmol) Butylamin in 25.1 ml Eisessig (454.7 mmol) gelöst und auf 80°C erhitzt. Nach zwei Stunden wird die Mischung abgekühlt und der ausgefallene Niederschlag abgesaugt und mit reichlich Wasser gewaschen. Das so erhaltene Rohprodukt wird in Ethylacetat gelöst, mit Natriumsulfat getrocknet und i.Vak. eingeengt.
Rₜ-Wert: 1.48 min (Methode F)
C₇H₇NO₂S (169.20)
Massenspektrum: (M+H)⁺ = 170

### (b) 1-Methyl-2-thiophen-2-yl-ethylamin

Zu einer Suspension von 4.1 g (109.3 mmol) Lithiumaluminiumhydrid in 150 ml THF wird eine Lösung von 3.7 g (21.9 mmol) 2-(2-Nitro-propenyl)-thiophen in 50 ml THF zugetropft. Nach beendeter Zugabe wird die Mischung auf Rückfluß erhitzt. Nach 1,5 Stunden wird auf RT abgekühlt und für 16 Stunden weiter gerührt. Dann wird die Mischung langsam mit 10 ml ges. Natriumsulfat-Lösung versetzt und über Celite filtriert. Das so erhaltene Filtrat wird i. Vak. eingeengt.
Rₜ-Wert: 0.52 min (Methode G)
C₇H₁₁NS (141.23)
Massenspektrum: (M+H)⁺ = 142

### (c) N-(1-Methyl-2-thiphen-2-yl-ethyl)-formamid

Eine Lösung aus 5.9 g (33.4 mmol) 1-Methyl-2-thiophen-2-yl-ethylamin in 34.8 ml (417.7 mmol) Ameisensäureethylester wird für 16 Stunden auf Rückfluß erhitzt. Anschließend wird die Mischung abgekühlt und i.Vak. zur Trockene eingeengt.
Rₜ-Wert: 1.00 min (Methode F)
C₈H₁₁NOS (169.25)
Massenspektrum: (M+H)⁺ = 170

### (d) 5-Methyl-4,5-dihydro-thieno[2,3-c]-pyridin

Eine Lösung aus 6.6 g (33.1 mmol) N-(1-Methyl-2-thiphen-2-yl-ethyl)-formamid in 200 ml Acetonitril wird im Eisbad abgekühlt und langsam mit einer Lösung von 6.1 ml (66.3 mmol) Phosphoroxychlorid in 50 ml Acetonitril versetzt. Man rührt zunächst weitere drei Stunden im Eisbad und rührt die Mischung dann für 16 Stunden bei RT. Anschließend wird die Mischung auf 200 ml Wasser gegossen, mit 9 g festen Natriumhydroxid alkalisch gestellt und dreimal mit Ethylacetat extrahiert. Die vereinigten ogranischen Phasen werden mit Aktivkohle und Natriumsulfat versetzt, filtriert und i.Vak. eingeengt.
Rₜ-Wert: 0.84 min (Methode H)
C₈H₉NS(151.23)
Massenspektrum: (M+H)⁺ = 152

### (e) 5,6-Dimethyl-4,5-dihydro-thieno[2,3-c]pyridiniumiodid

Eine Lösung aus 4.0 g (26.5 mmol) 5-Methyl-4,5-dihydro-thieno[2,3-c]-pyridin in 250 ml Acetonitril wird bei RT mit 16.5 ml (264.5 mmol) Methyliodid versetzt und für 30 min gerührt. Der gebildete Niederschlag wird abfiltriert und mit Acetonitril gewaschen.
Rₜ-Wert: 0.29 min (Methode G)
C₉H₁₂NSI (293.17)
Massenspektrum: (M)⁺ = 166

### (f) 5,6-Dimethyl-4,5,6,7-tetrahydro-thieno[2,3-c]pyridin (als Trifluoracetat-Salz)

Eine Mischung aus 3.4 g (9.3 mmol) 5,6-Dimethyl-4,5-dihydro-thieno[2,3-c]pyridiniumiodid in 30 ml Methanol wird bei Eisbadtemperatur portionsweise mit 709 mg (18.6 mmol) Natriumborhydrid versetzt. Man erwärmt die Reaktionsmischung langsam auf RT und rührt für 16 Stunden. Anschließend wird die Mischung wieder abgekühlt und mit 5 ml Salzsäure in Dioxan (4N) versetzt. Das Gemisch wird i.Vak. eingeengt und mittels präparativer HPLC (Eluens Wasser/Acetonitril/TFA) gereinigt.
Rₜ-Wert: 0.69 min (Methode F)
C₉H₁₃NS x CF₃CO₂H (167.27)
Massenspektrum: (M)⁺ = 168

### (g) 2-Brom-5,6-dimethyl-4,5,6,7-tetrahydro-thieno[2,3-c]pyridin

1.0 g (3.6 mmol) 5,6-Dimethyl-4,5,6,7-tetrahydro-thieno[2,3-c]pyridin (als Trifluoracetat-Salz) werden in 60 ml Wasser gelöst und bei Eisbadtemperatur mit einer Lösung aus 1.0 g (8.5 mmol) Kaliumbromid und 182 µl (3.6 mmol) Brom in 20 ml Wasser versetzt. Man rührt die Mischung für 2,5 Stunden im Eisbad weiter und extrahiert dann zweimal mit 100 ml Ethylacetat. Die vereinigten organischen Phasen werden mit ges. Natriumchlorid-Lösung gewaschen, dann über Natriumsulfat getrocknet und i.Vak. eingeengt. Das so erhaltene Rohprodukt wird an Silicagel chromatographisch (Eluens DCM/Methanol 50:1) gereinigt.
Rₜ-Wert: 0.99 min (Methode F)
C₉H₁₂BrNS (246.17)
Massenspektrum: (M+H)⁺ = 246/248 (Bromisotope)

### (h) 5,6-Dimethyl-4,5,6,7-tetrahydro-thieno[2,3-c]pyridin-2-carbonsäure-methylester

Herstellung analog Beispiel 26 e aus 2-Brom-4-5,6-dimethyl-4,5,6,7-tetrahydrothieno[2,3-c]-pyridin.
Rₜ-Wert: 0.63 min (Methode G)
C₁₁H₁₅NO₂S (225.31)
Massenspektrum: (M+H)⁺ = 226

### (i) 5,6-Dimethyl-4,5,6,7-tetrahydro-thieno[2,3-c]pyridin-2-carbonsäure

Herstellung analog Beispiel 45 b aus 5,6-Dimethyl-4,5,6,7-tetrahydro-thieno[2,3-c]pyridin-2-carbonsäure-methylester.
Rₜ-Wert: 0.32 min (Methode F)
C₁₀H₁₃NO₂S (211.28)
Massenspektrum: (M+H)⁺ = 212

### (j) (3S,4S)-5-Chlor-thiophen-2-carbonsäure-[1-(5,6-dimethyl-4,5,6,7-tetrahydro-thieno[2,3-c]-pyridin-2-carbonyl)-4-methoxy-pyrrolidin-3-yl]-amid (als Trifluoracetat-Salz)

Herstellung analog Beispiel 1 b aus 5,6-Dimethyl-4,5,6,7-tetrahydro-thieno[2,3-c]pyridin-2-carbonsäure.
Rₜ-Wert: 1.17 min (Methode F)
C₂₀H₂₄ClNO₃S₂ x CF₃CO₂H (454.01)
Massenspektrum: (M+H)⁺ = 454/456 (Chlorisotope)

### Beispiel 76

### (3RS,4S)-5-Chlor-thiophen-2-carbonsäure-[4-methoxy-1-(7-methyl-5,6,7,8-tetrahydro-imidazo[1,2-a]pyrazin-2-carbonyl)-pyrrolidin-3-yl]-amid

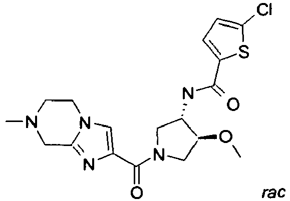

### (a) 7-Methyl-5,6,7,8-tetrahydro-imidazo[1,2-a]pyrazin-2-carbonsäure-ethylester (als Hydroformiat-Salz)

500,0 mg (2,2 mmol) 5,6,7,8-Tetrahydro-imidazo[1,2-a]pyrazin-2-carbonsäure-ethylester (hergestellt durch Freisetzung der Base ausgehend vom Hydrochlorid-Salz) werden in 0.8 ml (21.6 mmol) Ameisensäure gelöst und mit 0.6 ml (8.6 mmol) Formalin-Lösung (37% in Wasser) versetzt und für 5 Stunden bei 70°C Badtemperatur gerührt. Nach Abkühlen wird die Mischung zur Trockene i.Vak. eingeengt.
Rₜ-Wert: 1.48 min (Methode F)
C₁₀H₁₅N₃O₂ x HCO₂H (209.25)
Massenspektrum: (M+H)⁺ = 210

### (b) (3RS,4S)-5-Chlor-thiophen-2-carbonsäure-[4-methoxy-1-(7-methyl-5,6,7,8-tetrahydro-imidazo[1,2-a]pyrazin-2-carbonyl)-pyrrolidin-3-yl]-amid

Hergestellt analog zu Beispiel 1 b aus 7-Methyl5,6,7,8-Tetrahydro-imidazo[1,2-a]pyrazin-2-carbonsäure (Herstellung aus 7-Methyl-5,6,7,8-tetrahydro-imidazo[1,2-a]pyrazin-2-carbonsäure-ethylester (als Hydroformiat-Salz) analog Beispiel 45 b).
Rₜ-Wert: 1.02 min (Methode F)
C₁₁H₂₂ClN₅O₃S (423.92)
Massenspektrum: (M+H)⁺ = 424/426 (Chlorisotope)

### Beispiel 77

### (3R,5S)-5-Chlor-thiophen-2-carbonsäure-[5-methoxymethyl-1-(5-methyl-4,5,6,7-tetrahydro-pyrazolo[1,5-a]pyrarin-2-carbonyl)-pyrrolidin-3-yl]-amid

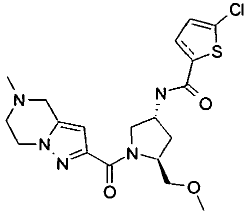

### (a) 6,7-Dihydro-4H-pyrazolo[1,5-a]pyrazin-2,5-dicarbonsäure-5-benzylester-2-ethylester

Hergestellt analog A. M. Venkatesan et al., J. Med. Chem. 2006, 49, 4623 ausgehend von Piperazin-1,3-dicarbonsäure-1-benzylester.
Rₜ-Wert: 1.41 min (Methode F)
C₁₇H₁₉N₃O₄ (329.35)
Massenspektrum: (M+H)⁺ = 330

### (b) 4,5,6,7-Tetrahydro-pyrazolo[1,5-a]pyrazin-2-carbonsäure-2-ethylester

200.0 mg (0.7 mmol) 6,7-Dihydro-4H-pyrazolo[1,5-a]pyrazin-2,5-dicarbonsäure-5-benzylester-2-ethylester werden in 20 ml Methanol gelöst, mit 100 mg Paladium/Kohle 10% versetzt und bei 3 bar Wasserstoffdruck und RT für 1.5 Stunden hydriert. Anschließend wird vom Katalysator abfiltriert und i.Vak. eingeengt.
Rₜ-Wert: 0.49 min (Methode F)
C₉H₁₃N₃O₂ (195.22)
Massenspektrum: (M+H)⁺ = 196

### (c) 5-Methyl-4,5,6,7-tetrahydro-pyrazolo[1,5-a]pyrazin-2-carbonsäure-2-ethylester (als Trifluoracetat-Salz)

Hergestellt analog Beispiel 76 a ausgehend von 4,5,6,7-Tetrahydropyrazolo[1,5-a]pyrazin-2-carbonsäure-2-ethylester mit anschließender Reinigung des Rohprodukts mittels präparativer HPLC (Eluens Wasser/Acetonitril/TFA).
Rₜ-Wert: 0.56 min (Methode F)
C₁₀H₁₅N₃O₂ x CF₃CO₂H (209.25)
Massenspektrum: (M+H)⁺ = 210

### (d) 5-Methyl-4,5,6,7-tetrahydro-pyrazolo[1,5-a]pyrazin-2-carbonsäure (als Hydrochlorid-Salz)

Hergestellt analog Beispiel 45 b ausgehend von 4,5,6,7-Tetrahydropyrazolo[1,5-a]pyrazin-2-carbonsäure-2-ethylester (als Trifluoracetat-Salz).
Rₜ-Wert: 0.24 min (Methode F)
C₈H₁₁N₃O₂ x HCl (181.19)
Massenspektrum: (M+H)⁺ = 182

### (e) (3R,5S)-5-Chlor-thiophen-2-carbonsäure-[5-methoxymethyl-1-(5-methyl-4,5,6,7-tetrahydro-pyrazolo[1,5-a]pyrarin-2-carbonyl)-pyrrolidin-3-yl]-amid

Hergestellt analog Beispiel 1 b ausgehend von 5-Methyl-4,5,6,7-tetrahydropyrazolo[1,5-a]pyrazin-2-carbonsäure (als Hydrochlorid-Salz).
Rₜ-Wert: 1.08 min (Methode F)
C₁₉H₂₄ClN₅O₃S (437.94)
Massenspektrum: (M+H)⁺ = 438/440 (Chlorisotope)

Analog kann die folgende Verbindung hergestellt werden:

| **Nr.** | **Strukturformel** | **Massenpeak(s)** | **R_{f}-Wert bzw. Rₜ** |
|---|---|---|---|
| | **Name** | | |
| **98** | 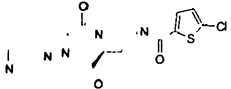 | (M+H)⁺ = 452/454 (Chlorisotope) | Rₜ-Wert = 0.59 min (Methode G) |
| | (3*R*,5*S*)-5-Chlor-thiophen-2-carbonsäure-[5-methoxymethyl-1-(6- | | |
| | methyl-5,6,7,8-tetrahydro-4H-1,6,8a-triaza-azulen-2-carbonyl)-pyrrol idin-3-yl]-amid | | |

### Beispiel 81

### (3R,5S)-5-Chlor-thiophen-2-carbonsäure-[5-methoxymethyl-1-(7-methyl-6,7,8,9-tetrahydro-5H-imidazo[1,2-a][1,4]diazepin-2-carbonyl)-pyrrolidin-3-yl]-amid

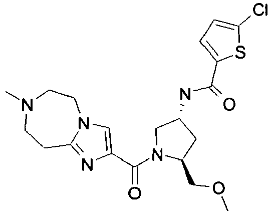

### (a) 5-Oxo-[1,4]diazepin-1-carbonsäure-benzylester

4.8 g (42.0 mmol) [1,4]Diazepin-5-on werden in 60 ml DCM gelöst und bei Eisbadtemperatur nacheinander mit 11.0 ml (83.9 mmol) Triethylamin und 6.8 ml (46.2 mmol) Chlorameisensäure-benzylester versetzt. Man erwärmt auf RT und drei Stunden bei dieser Temperatur. Anschließend konzentriert man die Mischung und versetzt mit Wasser. Die wässrige Phase wird dreimal mit Ethylacetat extrahiert. Die vereinigten organischen Phasen werden über Natriumsulfat getrocknet, filtriert und i.Vak. eingeengt. Das Rohprodukt wird mittels Säulenchromatographie an Silicagel (Eluens DCM/MeOH 95:5 -> 90:10).
Rₜ-Wert: 1.06 min (Methode F)
C₁₃H₁₆N₂O₃ (248.28)
Massenspektrum: (M+H)⁺ = 249

### (b) 5-Imino-[1,4]diazepin-1-carbonsäure-benzylester

1.0 g (4.0 mmol) 5-Oxo-[1,4]diazepin-1-carbonsäure-benzylester werden in 1.0 ml (10.6 mmol) Dimethylsulfat suspendiert, bei 90°C für zwei Stunden gerührt und nach dem Abkühlen in 10 ml (20 mmol) Ammoniak-Lösung (2N in Methanol) gelöst. Die Mischung wird für 3,5 Stunden bei RT gerührt und dann zur Trockene eingeengt.
Rₜ-Wert: 1.02 min (Methode F)
C₁₃H₁₇N₃O₂ (247.29)
Massenspektrum: (M+H)⁺ = 248

### (c) 2-Formyl-5,6,8,9-tetrahydro-imidazo[1,2-a][1,4]diazepin-7-carbonsäurebenzylester

1.0 g (4.0 mmol) 5-Imino-[1,4]diazepin-1-carbonsäure-benzylester werden in 3 ml Ethanol gelöst und mit 2.8 ml (8.1 mmol) Natriummethylat-Lösung (30%ig in Methanol) versetzt. Man gibt eine Lösung von 843 mg (4.4 mmol) 2-Brom-3-isopropoxy-propenal in 3 ml Ethanol zu und erhitzt nach beendeter Zugabe für 1,5 Stunden auf Rückfluß. Anschließend konzentriert man die Mischung, löst den Rückstand in 6 ml Chloroform und gibt 560 µl (4.0 mmol) Triethylamin zu. Die Reaktionsmischung wird 16 Stunden unter Rückfluß erhitzt, anschließend abegkühlt, eingeengt und mittels Flaschchromatographie an Silicagel (Eluens DCM/Methanol 20:1) gereinigt. Man erhält eine noch unreine Produktfraktion, die mittels präparativer HPLC (Eluens Wasser/Acetonitril/konz. Ammoniak) gereinigt wird.
Rₜ-Wert: 0.63 min (Methode G)
C₁₆H₁₇N₃O₃ (299.32)
Massenspektrum: (M+H)⁺ = 300

### (d) Ammnonium-5,6,8,9-tetrahydro-imidazo[1,2-a][1,4]diazepin-7-carbonsäure-benzylester-2-carboxylat

107,0 mg (0.36 mmol) 2-Formyl-5,6,8,9-tetrahydro-imidazo[1,2-a][1,4]diazepin-7-carbonsäure-benzylester werden in 1 ml DMSO gelöst und mit einer Lösung von 85 mg (0.55 mmol) Natriumdihydrogensulfat-dihydrat in 0.5 ml Wasser versetzt und im Eisbad abgekühlt. 160 mg (1.4 mmol) Natriumchlorat werden in 0.5 ml Wasser gelöst und langsam zur Eduktlösung getropft. Man rührt 30 min und filtriert vom Ungelösten. Das Filtrat wird mittels präparativer HPLC (Wasser/Acetonitril/konz. Ammoniak) gereinigt.
Rₜ-Wert: 0.41 min (Methode G)
C₁₆H₁₇N₃O₄ x NH₃ (315.33)
Massenspektrum: (M+H)⁺ = 316

### (e) 5,6,8,9-Tetrahydro-imidazo[1,2-a][1,4]diazepin-2,7-dicarbonsäure-7-benzylester-2-methylester

68.0 mg (0.21 mmol) Ammnonium-5,6,8,9-tetrahydro-imidazo[1,2-a][1,4]diazepin-7-carbonsäure-benzylester-2-carboxylat werden in 1.5 ml Methanol gelöst und im Eisbad mit 40 µl (0.6 mmol) Thionylchlorid versetzt. Man entfernt das Eisbad und erhitzt nach 30 Minuten für einen Tag auf Rückfluß. Es werden noch zweimal weitere 100 µl Thionylchlorid zugegeben und für einen weiteren Tag erhitzt. Anschließend wird das Reaktionsgemisch i.Vak. konzentriert und als Rohprodukt weiter umgesetzt.
Rₜ-Wert: 1.15 min (Methode F)
C₁₇H₁₉N₃O₄ (329.35)
Massenspektrum: (M+H)⁺ = 330

### (f) 6,7,8,9-Tetrahydro-5H-imidazo[1,2-a][1,4]diazepin-2-carbonsäure-methylester

Hergestellt analog Beispiel 77 b ausgehend von 5,6,8,9-Tetrahydro-imidazo[1,2-a][1,4]diazepin-2,7-dicarbonsäure-7-benzylester-2-methylester.
Rₜ-Wert: 0.36 min (Methode G)
C₉H₁₃N₃O₂ (195.22)
Massenspektrum: (M+H)⁺ = 196

### (g) 7-Methyl-6,7,8,9-Tetrahydro-5H-imidazo[11,2-a][1,4]diazepin-2-carbonsäure-methylester (als Trifluoracetat-Salz)

Hergestellt analog Beispiel 76 a ausgehend von 5,6,8,9-Tetrahydro-imidazo[1,2-a][1,4]diazepin-2-carbonsäure-methylester mit anschließender Reinigung über präparative HPLC (Eluens Wasser/Acetonitril/TFA).
Rₜ-Wert: 0.20 min (Methode F)
C₁₀H₁₅N₃O₂ x CF₃CO₂H (209.25)
Massenspektrum: (M+H)⁺ = 210

### (h) 7-Methyl-6,7,8,9-Tetrahydro-5H-imidazo[1,2-a][1,4]diazepin-2-carbonsäure (als Hydrochlorid-Salz)

Hergestellt analog Beispiel 45 b ausgehend von 4,5,6,7-Tetrahydropyrazolo[1,5-a]pyrazin-2-carbonsäure-2-ethylester (als Trifluoracetat-Salz).
Rₜ-Wert: 0.20 min (Methode F)
C₉H₁₃N₃O₂ (195.22)
Massenspektrum: (M+H)⁺ = 196

### (i) (3R,5S)-5-Chlor-thiophen-2-carbonsäure-[5-methoxymethyl-1-(7-methyl-6,7,8,9-tetrahydro-5H-imidazo[1,2-a][1,4]diazepin-2-carbonyl)-pyrrolidin-3-yl]-amid

Hergestellt analog Beispiel 1 b ausgehend von 7-Methyl-6,7,8,9-Tetrahydro-5H-imidazo[1,2-a][1,4]diazepin-2-carbonsäure (als Hydrochlorid-Salz).
Rₜ-Wert: 1.00 min (Methode F)
C₂₀H₂₆ClN₅O₃S (451.97)
Massenspektrum: (M+H)⁺ = 452/454 (Chlorisotope)

### Beispiel 82

### (3RS,4SR)-5-Chlor-thiophen-2-carbonsäure-{4-methoxy-1-[(7RS)-7-methoxymethyl-6-methyl-4,5,6,7-tetrahydro-thieno[2,3-c]pyridin-2-carbonyl]-pyrrolidin-3-yl}-amid (als Trifluoracetat-Salz)

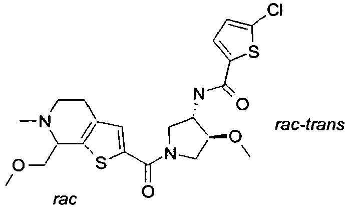

### (a) 2-Methoxy-N-(2-thiophen-3-yl-ethyl)-acetamid

10.0 g (78.6 mmol) 2-Thiophen-3-yl-ethylamin werden in 120 ml THF gelöst und bei -10°C nacheinander mit 7.9 ml (86.5 mmol) Methoxyessigsäurechlorid und einer Lösung von 21.9 ml (157.2 mmol) Triethylamin in 30 ml THF versetzt. Man erwärmt die Mischung auf RT und rührt für eine Stunde. Anschließend wird mit 2N Salzsäure angesäuert und die Mischung dreimal mit Ethylacetat extrahiert. Die vereinigten organischen Phasen werden über Natriumsulfat getrocknet, filtriert und i.Vak. eingeengt.
Rₜ-Wert: 1.01 min (Methode F)
C₉H₁₃NO₂S (199.27)
Massenspektrum: (M+H)⁺ = 200

### (b) 7-Methoxymethyl-4,5-dihydro-thieno[2,3-c]pyridin

15.6 g (78.3 mmol) 2-Methoxy-*N*-(2-thiophen-3-yl-ethyl)-acetamid werden in 350 ml Chloroform gelöst und unter Eisbadkühlung mit 35.8 ml (391.4 mmol) Phosphoroxychlorid versetzt. Man entfernt das Eisbad und erhitzt für 5 Stunden auf Rückfluß. Anschließend wird das Reaktionsgemisch vorsichtig in 600 ml warme Natronlauge (4N) eingetragen und die wässrige Phase dreimal mit Ethylacetat extrahiert. Die vereinigten organischen Phasen werden über Natriumsulfat getrocknet, filtriert und i.Vak. eingeengt. Das Rohprodukt wird mittels Flashchromatographie an Silicagel (Eluens DCM/Methanol 98:2 -> 95:5) gereinigt.
Rₜ-Wert: 0.59 min (Methode F)
C₉H₁₁NOS (181.26)
Massenspektrum: (M+H)⁺ = 182

### (c) 7-Methoxymethyl-4,5,6,7-tetrahydro-thieno[2,3-c]pyridin

1.0 g (5.5 mmol) 7-Methoxymethyl-4,5-dihydro-thieno[2,3-c]pyridin werden in 10 ml Methanol gelöst und unter Eisbadkühlung portionsweise mit 0.4 g (11.0 mmol) Natriumborhydrid versetzt. Man entfernt das Eisbad und rührt bei RT für zwei Stunden. Anschließend wird das Reaktionsgemisch eingeengt und als Rohprodukt weiter umgesetzt.
Rₜ-Wert: 0.75 min (Methode F)
C₉H₁₃NOS (183.27)
Massenspektrum: (M+H)⁺ = 184

### (d) 7-Methoxymethyl-6-methyl-4,5,6,7-tetrahydro-thieno[2,3-c]pyridin

Hergestellt analog Beispiel 76 a ausgehend von 7-Methoxymethyl-4,5,6,7-tetrahydro-thieno[2,3-c]pyridin.
Rₜ-Wert: 0.78 min (Methode F)
C₁₀H₁₅NOS (197.30)
Massenspektrum: (M+H)⁺ = 198

### (e) 2-Brom-7-methoxymethyl-6-methyl-4,5,6,7-tetrahydro-thieno[2,3-c]pyridin

Hergestellt analog Beispiel 73 g ausgehend von 7-Methoxymethyl-6-methyl-4,5,6,7-tetrahydro-thieno[2,3-c]pyridin.
Rₜ-Wert: 1.04 min (Methode F)
C₁₀H₁₄BrNOS (276.19)
Massenspektrum: (M+H)⁺ = 276/278 (Bromisotope)

### (f) 7-Methoxymethyl-6-methyl-4,5,6,7-tetrahydro-thieno[2,3-c]pyridin-2-carbonsäure-methylester

Hergestellt analog Beispiel 26 e ausgehend von 2-Brom-7-methoxymethyl-6-methyl-4,5,6,7-tetrahydro-thieno[2,3-c]pyridin.
Rₜ-Wert: 0.87 min (Methode F)
C₁₂H₁₇NO₃S (255.33)
Massenspektrum: (M+H)⁺ = 256

### (g) 7-Methoxymethyl-6-methyl-4,5,6,7-tetrahydro-thieno[2,3-c]pyridin-2-carbonsäure (als Hydrochlorid-Salz)

Hergestellt analog Beispiel 45 b ausgehend von 7-Methoxymethyl-6-methyl-4,5,6,7-tetrahydro-thieno[2,3-c]pyridin-2-carbonsäure-methylester.
Rₜ-Wert: 0.58 min (Methode F)
C₁₁H₁₅NO₃S x HCl (241.31)
Massenspektrum: (M+H)⁺ = 242

### (h) (3RS,4SR)-5-Chlor-thiophen-2-carbonsäure-{4-methoxy-1-[(7RS)-7-methoxymethyl-6-methyl-4,5,6,7-tetrahydro-thieno[2,3-c]pyridin-2-carbonyl]-pyrrolidin-3-yl}-amid (als Trifluoracet-Salz)

Hergestellt analog Beispiel 1 b ausgehend von 7-Methoxymethyl-6-methyl-4,5,6,7-tetrahydro-thieno[2,3-c]pyridin-2-carbonsäure (als Hydrochlorid-Salz).
Rₜ-Wert: 1.16 min (Methode F)
C₂₁H₂₆ClN₃O₄S₂ x CF₃CO₂H (241.31)
Massenspektrum: (M+H)⁺ = 484/486 (Chlorisotope)

### Beispiel 84

### (2S,4R)-Ethyl-carbaminsäure-4-[(5-chlor-thiophen-2-carbonyl)-amino]-1-(6-methyl-5,6,7,8-tetrahydro-4H-thieno[2,3-d]azepin-2-carbonyl)-pyrrolidin-2-yl-methylester (als Trifluoracetat-Salz)

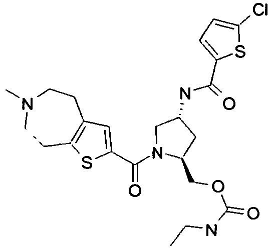

### (a) (2S,4R)-4-[(5-Chlor-thiophen-2-carbonyl)-amino]-2-ethylcarbamoyloxymethyl-pyrrolidin-1-carbonsäure-tert.-butylester

120.0 mg (0.3 mmol) (2*S*,4*R*)-4-[(5-Chlor-thiophen-2-carbonyl)-amino]-2-hydroxymethyl-pyrrolidin-1-carbonsäure-tert.-butylester werden in 5 ml Toluol mit 30 µl (0.38 mmol) Ethylisocyanat für drei Stunden unter Rückfluß erhitzt. Anschließend werden dreimal in Drei-Stunden-Intervallen je 100 µl Ethylisocyanat zugegeben und weitere 16 Stunden unter Rückfluß erhitzt. Anschließend wird die Mischung abgekühlt, zur Trockene eingeengt und mittels Flashchromatographie an Silicagel (Eluens DCM/Methanol 100:3) gereinigt.
Rₜ-Wert: 1.52 min (Methode F)
C₁₈H₂₆ClN₃O₅S (431.93)
Massenspektrum: (M+H)⁺ = 432/434 (Chlorisotope)

### (b) (2S,4R)-Ethyl-carbaminsäure-4-[(5-chlor-thiophen-2-carbonyl)-amino]-pyrrolidin-2-yl-methylester (als Trifluoracetat-Salz)

77.0 mg (0.3 mmol) (2*S*,4*R*)-4-[(5-Chlor-thiophen-2-carbonyl)-amino]-2-ethylcarbamoyloxymethyl-pyrrolidin-1-carbonsäure-tert.-butylester werden in 1.0 ml eines Gemisch aus TFA und DCM (v/v 1:1) gelöst und bei RT für drei Stunden gerührt. Anschließend wird das Reaktionsgemisch zur Trockene eingeengt.
Rₜ-Wert: 1.04 min (Methode F)
C₁₃H₁₈ClN₃O₃S x CF₃CO₂H (331.82)
Massenspektrum: (M+H)⁺ = 332/334 (Chlorisotope)

### (c) (2S,4R)-Ethyl-carbaminsäure-4-[(5-chlor-thiophen-2-carbonyl)-amino]-1-(6-methyl-5,6,7,8-tetrahydro-4H-thieno[2,3-d]azepin-2-carbonyl)-pyrrolidin-2-yl-methylester (als Trifluoracetat-Salz)

Hergestellt analog Beispiel 1 b aus (2*S*,4*R*)-Ethyl-carbaminsäure-4-[(5-chlorthiophen-2-carbonyl)-amino]-pyrrolidin-2-yl-methylester (als Trifluoracetat-Salz) Rₜ-Wert: 1.17 min (Methode F)
C₂₃H₂₉ClN₄O₄S₂ x CF₃CO₂H (331.82)
Massenspektrum: (M+H)⁺ = 525/527 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **85** | 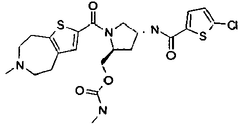 | (M+H)⁺ = 511/513 (Chlorisotope) | Rₜ-Wert = 1.14 min (Methode F) |
| | (2*S*,4*R*)-Methyl-carbaminsäure-4-[(5-chlor-thiophen-2-carbonyl)-amino]-1-(6-methyl-5,6,7,8-tetrahydro-4H-thieno[2,3-d]azepin-2-carbonyl)-pyrrolidin-2-yl-methylester (als Trifluoracetat-Salz) | | |
| **86** | 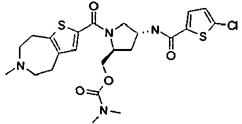 | (M+H)⁺ = 525/527 (Chlorisotope) | Rₜ-Wert = 1.16 min (Methode F) |
| | (2*S*,4*R*)-Dimethyl-carbaminsäure-4-[(5-chlor-thiophen-2-carbonyl)-amino]-1-(6-methyl-5,6,7,8-tetrahydro-4H-thieno[2,3-d]azepin-2-carbonyl)-pyrrolidin-2-yl-methylester (als Trifluoracetat-Salz) | | |

### Beispiel 90

### (3R,5S)-5-Chlor-thiophen-2-carbonsäure-[5-(acetylamino-methyl)-1-(6-methyl-5,6,7,8-tetrahydro-4H-thieno[2,3-d]azepin-2-carbonyl)-pyrrolidin-3-yl]-amid

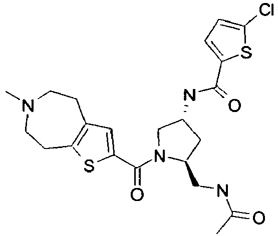

### (a) (2S,4R)-4-[(5-Chlor-thiophen-2-carbonyl)-amino]-2-methansulfonyloxymethyl-pyrrolidin-1-carbonsäure-tert.-butylester

278.0 mg (0.8 mmol) (2*S*,4*R*)-4-[(5-Chlor-thiophen-2-carbonyl)-amino]-2-hydroxymethyl-pyrrolidin-1-carbonsäure-tert.-butylester werden in 6 ml DCM gelöst und mit 216 µl (1.5 mmol) Triethylamin versetzt. Unter Eisbadkühlung werden 89 µl (1.2 mmol) Methansulfonsäurechlorid zugegeben. Man erwärmt auf RT und rührt für zwei Stunden. Anschließend wird das Reaktionsgemisch mit Wasser versetzt und dreimal mit Ethylacetat extrahiert. Die vereinigten organischen Phasen werden mit Natriumsulfat getrocknet, filtriert und i.Vak. eingeeengt.
Rₜ-Wert: 1.50 min (Methode F)
C₁₆H₂₃ClN₂O₆S₂ (438.95)
Massenspektrum: (M+H)⁺ = 439/441 (Chlorisotope)

### (b) (2S,4R)-2-Azidomethyl-4-[(5-Chlor-thiophen-2-carbonyl)-amino]-pyrrolidin-1-carbonsäure-tert.-butylester

330.0 mg (0.75 mmol) (2*S*,4*R*)-4-[(5-Chlor-thiophen-2-carbonyl)-amino]-2-methansulfonyloxymethyl-pyrrolidin-1-carbonsäure-tert.-butylester werden in 20 ml DMF gelöst und mit 146.6 mg (2.3 mmol) Natriumazid versetzt. Man rührt die Mischung bei 50°C für 16 Stunden. Danach werden weitere 73 mg (1.12 mmol) Natriumazid zugegeben und weitere zwei Stunden bei 50°C gerührt. Anschließend wird das Gemisch i.Vak. konzentriert. Der Rückstand wird mit Wasser/ges. Kochsalz-Lösung versetzt und dreimal mit Ethylacetat extrahiert. Die vereinigten organischen Phasen werden mit Natriumsulfat getrocknet, filtriert und i.Vak. eingeeengt.
Rₜ-Wert: 1.64 min (Methode F)
C₁₅H₂₀ClN₅O₃S (385.87)
Massenspektrum: (M+H-BOC)⁺ = 285/287 (Chlorisotope)

### (c) (2S,4R)-2-Aminomethyl-4-[(5-Chlor-thiophen-2-carbonyl)-amino]-pyrrolidin-1-carbonsäure-tert.-butylester

290.0 mg (0.75 mmol) (2*S*,4*R*)-2-Azidomethyl-4-[(5-Chlor-thiophen-2-carbonyl)-amino]-pyrrolidin-1-carbonsäure-tert.-butylester werden in einem Gemisch aus 4 ml THF und 0.4 ml Wasser gelöst und mit 0.3 g (1.1 mmol) Triphenylphosphin versetzt. Man rührt die Reaktionsmischung bei RT für 16 Stunden. Anschließend wird das Gemisch i.Vak. konzentriert, mit Wasser und verd. Natronlauge versetzt und dreimal mit DCM extrahiert. De vereinigten organischen Phasen werden über Natriumsulfat getrocknet, filtriert und i.Vak. eingeengt. Das Rohprodukt wird mittels Flashchromatographie an Silicagel (Eluens DCM/Methanol 9:1 -> 1:1) gereinigt.
Rₜ-Wert: 1.25 min (Methode F)
C₁₅H₂₂ClN₃O₃S (359.87)
Massenspektrum: (M+H)⁺ = 360/362 (Chlorisotope)

### (d) (2S,4R)-2-Acetylaminomethyl-4-[(5-Chlor-thiophen-2-carbonyl)-amino]-pyrrolidin-1-carbonsäure-tert.-butylester

70.0 mg (0.2 mmol) (2*S*,4*R*)-2-Aminomethyl-4-[(5-Chlor-thiophen-2-carbonyl)-amino]-pyrrolidin-1-carbonsäure-tert.-butylester werden in 5 ml DCM gelöst und bei -10°C nacheinander mit 15.4 µl (0.2 mmol) Acetylchlorid und mit 67 µl (0.4 mmol) DIPEA versetzt. Man rührt für zwei Stunden bei 0°C. Anschließend wird die Mischung durch Zugabe von verd. Salzsäure vorsichtig schwach sauer gestellt und dreimal mit Ethylacetat extrahiert. Die vereinigten organischen Phasen werden über Natriumsulfat getrocknet, filtriert und i.Vak. eingeengt.
Rₜ-Wert: 1.36 min (Methode F)
C₁₇H₂₄ClN₃O₄S (401.91)
Massenspektrum: (M+H)⁺ = 402/404 (Chlorisotope)

### (e) (3R,5S)-5-Chlor-thiophen-2-carbonsäure-[5-(acetylamino-methyl)-1-(6-methyl-5,6,7,8-tetrahydro-4H-thieno[2,3-d]azepin-2-carbonyl)-pyrrolidin-3-yl]-amid

Hergestellt analog Beispiel 1 b aus (2*S*,4*R*)-2-Acetylaminomethyl-4-[(5-Chlorthiophen-2-carbonyl)-amino]-pyrrolidin-1-carbonsäure-tert.-butylester.
Rₜ-Wert: 0.60 min (Methode G)
C₂₂H₂₇ClN₄O₃S₂ (495.06)
Massenspektrum: (M+H)⁺ = 495/497(Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **93** | 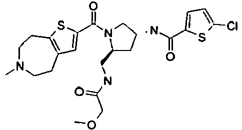 | (M+H)⁺ = 525/527 (Chlorisotope) | Rₜ-Wert = 1.08 min (Methode F) |
| | (3*R*,5*S*)-5-Chlor-thiophen-2-carbonsäure-[5-[(2-methoxy-acetylamino)-methyl]-1-(6-methyl-5,6,7,8-tetrahydro-4H-thieno{[2,3-d]azepin-2-carbonyl}-pyrrolidin-3-yl)-amid (als Trifluoracetat-Salz) | | |
| **94** | 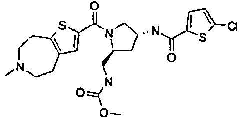 | (M+H)⁺ = 524/526 (Chlorisotope) | Rₜ-Wert = 1.08 min (Methode F) |
| | (2*S*,4*R*)- 4-[(5-Chlor-thiophen-2-carbonyl)-amino]-1-(6-methyl-5,6,7,8-tetrahydro-4H-thieno[2,3-d]azepin-2-carbonyl)-pyrrolidin-2-ylmethyl]-carbaminsäure-methylester (als Trifluoracetat-Salz) | | |
| **95** | 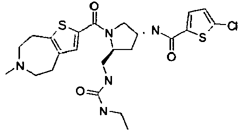 | (M+H)⁺ = 511/513 (Chlorisotope) | Rₜ-Wert = 1.12 min (Methode F) |
| | (3*R*,5*S*)- 5-Chlor-thiophen-2-carbonsäure-[5-[(3-ethyl-ureido)-methyl]-1-(6-methyl-5,6,7,8-tetrahydro-4H-thieno[2.3-d]azepin-2-carbonyl)-pyrrolidin-3-yl]-amid (als Trifluoracetat-Salz) | | |
| **96** | 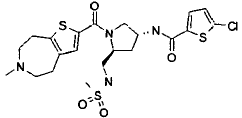 | (M+H)⁺ = 531/533 (Chlorisotope) | Rₜ-Wert = 1.38 min (Methode F) |
| | (3*R*,5*S*)- 5-Chlor-thiophen-2-carbonsäure-[5-(methansulfonylamino-methyl)-1-(6-methyl-5,6,7,8-tetrahydro-4H-thieno[2.3-d]azepin-2-carbonyl)-pyrrolidin-3-yl]-amid (als Trifluoracetat-Salz) | | |

### Beispiel 89

### (3R,5S)-5-Chlor-thiophen-2-carbonsäure-[5-dimethylaminomethyl-1-(6-methyl-5,6,7,8-tetrahydro-4H-thieno[2,3-d]azepin-2-carbonyl)-pyrrolidin-3-yl]-amid

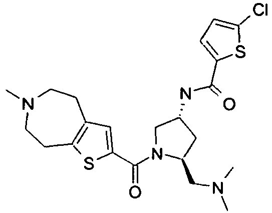

### (a) (2S,4R)-4-[(5-Chlor-thiophen-2-carbonyl)-amino]-2-dimethylaminomethylpyrrolidin-1-carbonsäure-tert.-butylester

70.0 mg (0.2 mmol) (2*S*,4*R*)-2-Aminomethyl-4-[(5-Chlor-thiophen-2-carbonyl)-amino]-pyrrolidin-1-carbonsäure-tert.-butylester werden in 4 ml Methanol suspendiert und mit Eisessig auf pH=6 gestellt. Dann gibt man 34 µl (0.45 mmol) Formaldehyd-Lösung (37%ig in Wasser) zu und rühtr für 30 min bei RT. Anschließend wird portionsweise 95 mg (0.45 mmol)
Natriumtriacetoxyborhydrid zugegeben und für 16 Stunden bei RT gerührt. Dann wird die Reaktionsmischung auf ges. Natriumhydrogencarbonat-Lösung gegossen und dreimal mit Ethylacetat extrahiert. Die vereinigten organischen Phasen werden über Natriumsulfat getrocknet, filtriert und i.Vak. eingeengt.
Rₜ-Wert: 1.35 min (Methode F)
C₁₇H₂₆ClN₃O₃S (387.93)
Massenspektrum: (M+H)⁺ = 388/390 (Chlorisotope)

### (b) (3R,5S)-5-Chlor-thiophen-2-carbonsäure-[5-dimethylaminomethyl-1-(6-methyl-5,6,7,8-tetrahydro-4H-thieno[2,3-d]azepin-2-carbonyl)-pyrrolidin-3-yl]-amid

Hergestellt analog Beispiel 1 b aus (2*S*,4*R*)-4-[(5-Chlor-thiophen-2-carbonyl)-amino]-2-dimethylaminomethyl-pyrrolidin-1-carbonsäure-tert.-butylester.
Rₜ-Wert: 0.64 min (Methode G)
C₂₂H₂₉ClN₄O₂S₂ (481.08)
Massenspektrum: (M+H)⁺ = 481/483(Chlorisotope)

### Beispiel 91

### (3R,5S)-5-Chlor-thiophen-2-carbonsäure-[5-methoxymethyl-1-((4S)-6-methyl-5,6,7,8-tetrahydro-4H-4-methoxy-thieno[2,3-d]azepin-2-carbonyl]-pyrrolidin-3-yl]-amid

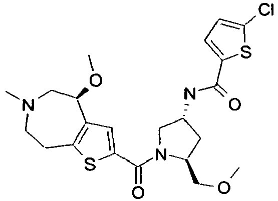

### (a) (S)-4-Hydroxy-4,5,7,8-tetrahydro-thieno[2,3-d]azepin-6-carbonsäureethyl-ester

3.86 g (16.1 mmol) 4-Oxo-4,5,7,8-tetrahydro-thieno[2,3-d]azepin-6-carbonsäureethyl-ester (hergestellt analog zu WO2007/84622) in 50 ml Methylenchlorid werden unter Argon bei -25°C erst mit 1.92 ml einer 1.0 M-Toluol-Lösung von (S)-3,3-Diphenyl-1-methyl-tetrahydro-1H,3H-pyrrolo[1,2-c][1,3,2]oxazaborolidin und anschließend mit 20.2 ml einer 2 M Boran-Dimethylsulfidkomplex-Toluol-Lösung in 70 ml Methylenchlorid versetzt und 3 Tage bei -18°C kaltgestellt. Anschließend wird ges. NH3Cl-Lösung zugegeben und 3 x mit Methylenchlorid extrahiert. Die vereinigten organischen Phasen werden mit NaSO4 getrocknet, konzentriert und das Rohprodukt wird mittels Flashchromatographie an Silicagel (Eluens DCM/Methanol 95:5) gereinigt.
Rₜ-Wert: 1.15 min (Methode F)
C₁₁H₁₅NO₃S (241.31)
Massenspektrum: (M+H)⁺ = 242

### (b) (S)- 4-Methoxy-4,5,7,8-tetrahydro-thieno[2,3-d]azepin-6-carbonsäureethyl-ester

3.39 g (14.0 mmol) (S)-4-Hydroxy-4,5,7,8-tetrahydro-thieno[2,3-d]azepin-6-carbonsäureethyl-ester in 40 ml THF werden unter Eisbadkühlung portionsweise mit 0.86 g 60% NaH-Mineralöldispersion versetzt, 15 min gerührt und anschließend werden 1.07 ml Methyliodid landsam zugetropft. Es wird 1 h nachgerührt, auf Eiswasser gegossen und 3 x mit Essigester extrahiert. Die vereinigten organischen Phasen werden mit NaSO4 getrocknet und konzentriert.
Rₜ-Wert: 1.38 min (Methode F)
C₁₂H₁₇NO₃S (241.31)
Massenspektrum: (M+H)⁺ = 256

### (c) (3R,5S)-5-Chlor-thiophen-2-carbonsäure-[5-methoxymethyl-1-[(4S)-6-methyl-5,6,7,8-tetrahydro-4H-4-methoxy-thieno[2,3-d]azepin-2-carbonyl]-pyrrolidin-3-yl]-amid

Hergestellt aus (S)- 4-Methoxy-4,5,7,8-tetrahydro-thieno[2,3-d]azepin-6-carbonsäureethyl-ester in analogie zu der folgenden Synthesesequenz: 60a, 60b, 26e, 45b, 53.
Rₜ-Wert: 0.67 min (Methode G)
C₂₂H₂₈ClN₃O₃S₂ (498.06)
Massenspektrum: (M+H)⁺ = 498/500 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **92** | 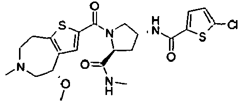 | (M+H)⁺ = 511/513 (Chlorisotope) | Rₜ-Wert = 0.60 min (Methode G) |
| | (2S,4R)-4-[(5-Chlor-thiophen-2-carbonyl)-amino]-1-((S)-4-methoxy-6-methyl-5,6,7,8-tetrahydro-4H-thieno[2,3-d]azepin-2-carbonyl)- | | |
| | pyrrolidin-2-carbonsäuremethylamid | | |
| **97** | 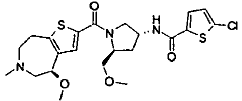 | (M+H)⁺ = 498/500 (Chlorisotope) | Rₜ-Wert = 0.67 min (Methode F) |
| | 5-Chlor-thiophen-2-carbonsäure[(3R,5S)-5-methoxymethyl-1-((R)-4-methoxy-6-methyl-5,6,7,8-tetrahydro-4H-thieno[2,3-d]azepin-2-carbonyl)-pyrrolidin-3-yl]-amid | | |

Die nachfolgenden Beispiele beschreiben die Herstellung pharmazeutischer Anwendungsformen, die als Wirkstoff eine beliebige Verbindung der allgemeinen Formel I enthalten.

### Beispiel A

Trockenampulle mit 75 mg Wirkstoff pro 10 ml

### Zusammensetzung:

Wirkstoff 75.0 mg
Mannitol 50.0 mg
Wasser für Injektionszwecke ad 10.0 ml

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

Trockenampulle mit 35 mg Wirkstoff pro 2 ml

### Zusammensetzung:

Wirkstoff 35.0 mg
Mannitol 100.0 mg
Wasser für Injektionszwecke ad 2.0 ml

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel C

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel D

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel E

Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel F

Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 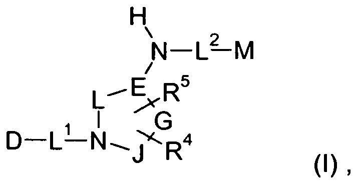 in denen
D ein substituiertes bicyclisches Ringsystem der Formel (IIa) , (IIb) oder (IIc) 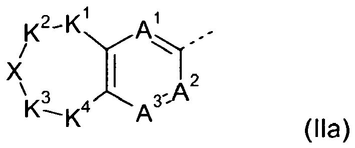 oder 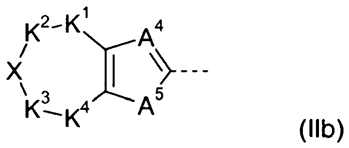 oder 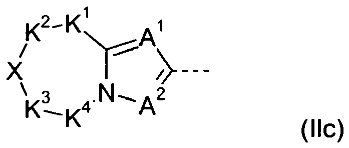 darstellt, in dem
K¹ und K⁴
jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem N-Atom durch eine -CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³
jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c}
jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Al kyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (IIa) oder (IIb) oder (IIc) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonylgruppe substituiert sein können, oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
A⁴ entweder N oder CR¹² bedeutet,
A⁵ NH, Schwefel oder Sauerstoff bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Phenyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
-L-E-G-J- eine -C-C-C-C-Gruppe bedeutet, die durch R⁴ und R⁵ substituiert sein kann, und
L¹ eine -C(O)-Gruppe bedeutet, und
R⁴ ein Wasserstoffatom oder
eine geradkettige oder verzweigte C₁₋₆-Alkyl-, C₂₋₆-Alkenyl- oder C₂-₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkylaminocarbonyloxy-, Di-(C₁₋₅-alkyl)-aminocarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₃-Alkyloxy-C_{1- 2}alkylcarbonylamino-, C₁₋₃-Alkyloxycarbonylamino-, C₁₋₃-Alkylaminocarbonylamino-, C₁₋₅-Alkylsulfonylamino-, N-(C₁₋₅-Alkylsulfonyl)-C₁₋₅-alkylamino-, C₃₋₆-Cycloalkylcarbonyl-aminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und lodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet,
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₅-Alkyloxycarbonyl-, Carboxy-, Methyl-, Hydroxy-, Methoxy- oder Aminosubstituiert sein können, und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O- Bindung bilden, ausgeschlossen ist, und
R⁵ ein Wasserstoffatom, eine C₁₋₅Alkyl-, C₂₋₅Alkenyl- oder C₂₋₅Alkinyl- oder eine Phenyl-C₀₋₅Alkylgruppe bedeutet, wobei die Alkylgruppe durch eine Hydroxy-, Methoxy-, Hydroxycarbonyl- oder C₁₋₅Alkoxycarbonyl-gruppe substituiert sein kann,
oder sofern R⁵ mit G verknüpft ist auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O Gruppe, oder eine -CF₂ Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O- Bindung bilden,
ausgeschlossen ist, und
L² eine -C(O)-Gruppe bedeutet, und
M einen gegebenenfalls durch R² und R³ substituierten Phenyl-, Pyridyl-, Thienyl- oder Furyl-Ring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R³ ein Wasserstoff-, Fluor-, Chlor-, Brom- oder Jod-atom oder eine Hydroxy-, Methoxy-, Trifluormethoxy-gruppe, oder eine gegebenenfalls durch Fluoratome substituierte C₁₋₃-Alkyl-gruppe, oder eine Cyano-, Amino- oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und lod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen D, E, G, J, L, L¹, L² und M wie in Anspruch 1 beschrieben definiert sind, und in der
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, C₁₋₅-Alkylaminocarbonyloxy-, Di-(C₁₋₅-alkyl)-aminocarbonyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₃-Alkyloxy-C₁₋₂alkylcarbonylamino-, C₁₋₃-Alkyloxycarbonylamino-, C₁₋₃-Alkylaminocarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋6-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅-Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkyl-, Allyl-, Propargyl- oder Benzylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder in der zwei Atome im Ring eine -O-O- oder -S-O-Bindung bilden,
ausgeschlossen ist,
deren Tautomere, deren Enantiomere, deren Diastereomere, deren Gemische und deren Salze.

3. Verbindungen der allgemeinen Formel (I) nach Anspruch 1 oder 2, in denen E, G, J, L, L¹, L², M, R⁴ und R⁵ wie in Anspruch 1 oder 2 beschrieben definiert sind, und in der
D ein substituiertes bicyclisches Ringsystem der Formel (IIa) oder (IIb) 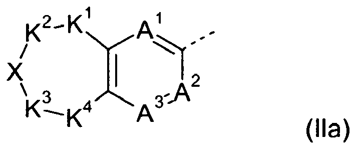 oder 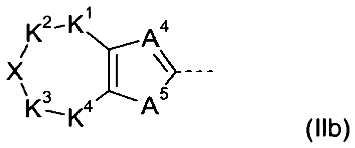 darstellt, in dem K¹ und K⁴
jeweils unabhängig voneinander eine Bindung, eine -CH₂-, -CHR^{7a}--CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe, oder
zwei Reste R^{7b}/R^{7c} zusammen mit dem Ringkohlenstoffatom einen 3-gliedrigen Carbocyclus bilden können,
mit der Maßgabe, dass K¹ und K⁴ gleichzeitig eine Bindung bedeuten, ausgeschlossen ist, und
K² und K³
jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c}
jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt in den Formeln (IIa) oder (IIb) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine -CF₂- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
A⁴ entweder N oder CR¹² bedeutet,
A⁵ NH, Schwefel oder Sauerstoff bedeutet,
wobei R¹⁰, R¹¹ und R¹² jeweils unabhängig voneinander
ein Wasserstoff-, Fluor-, Chlor-, Brom- oder Iodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten,
deren Tautomere, deren Enantiomere, deren Diastereomere, deren Gemische und deren Salze.

4. Verbindungen der allgemeinen Formel (I) nach einem der Ansprüche 1 bis 3, in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
A⁴ entweder N oder CR¹² bedeutet,
A⁵ Schwefel bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

5. Verbindungen der allgemeinen Formel (I) nach einem der Ansprüche 1 bis 4, in denen
D ein substituiertes bicyclisches Ringsystem der Formel 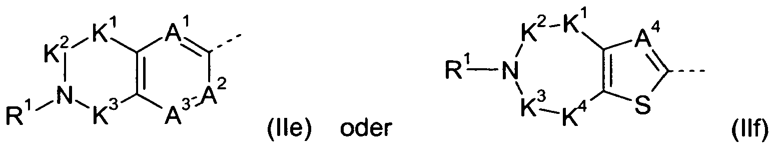 darstellt, in dem
K¹ eine -CH₂-, -CHR^{7a}-, oder eine -CR^{7b}R^{7c}- Gruppe bedeutet, und K² und K³
jeweils unabhängig voneinander eine -CH₂-, -CHR^{8a}-, oder eine - CR^{8b}R^{8c}- Gruppe bedeuten, wobei
R⁸a/R^{8b}/R^{8c}
jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe bedeutet,
wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können,und
insgesamt in den Formeln (IIe) oder (IIf) maximal vier Reste ausgewählt aus R^{7a}, R^{7b}, R^{7c}, R^{8a}, R^{8b} und R^{8c} vorhanden sein dürfen, und
R¹ ein Wasserstoffatom oder eine C₁₋₃-Alkyl-, Allyl- oder Cyclopropylgruppe bedeutet, und in denen
A¹ CR¹⁰ bedeutet,
A² CR¹¹ bedeutet,
A³ CR¹² bedeutet,
A⁴ entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine -C-C-C-C-Gruppe bedeutet, die durch R⁴ und R⁵ substituiert sein kann, und
R⁴ ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, C₁₋₅-Alkylaminocarbonyloxy-, Di-(C₁₋₅-alkyl)-aminocarbonyloxy-, Carboxy-, C₁₋₅-Alkyloxycarbonyl-, C₁₋₃-Alkyloxy-C₁₋₂alkylcarbonylamino-, C₁₋₃-Alkyloxycarbonylamino-, C₁₋₃-Alkylaminocarbonylamino-, C₁₋₅-Alkylcarbonylamino-, C₁₋₅-Alkylsulfonylamino-gruppe substituiert sein können, oder
sofern R⁴ mit G verknüpft ist, auch ein Fluoratom oder eine Hydroxy-, Methoxy-, C₃₋₅-Alkenyl-oxy-, C₂₋₅-Alkyl-oxy-, C₃₋₆-Cycloalkyl-oxy-, Benzyloxy-, C₁₋₅-Alkylaminocarbonyloxy-, Di(C₁₋₅-alkyl)aminocarbonyloxy- oder eine C₄₋₇-Cycloalkyleniminocarbonyloxygruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
R⁵ ein Wasserstoffatom oder eine C₁₋₅ Alkyl-, Allyl-, Benzyl- oder Phenylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
deren Tautomere, deren Enantiomere, deren Diastereomere, deren Gemische und deren Salze.

6. Verbindungen der allgemeinen Formel (I) nach einem der Ansprüche 1 bis 5, in denen
D ein substituiertes bicyclisches Ringsystem der Formel 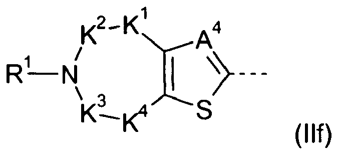 darstellt, in dem
K¹ eine -CH₂-, -CHR^{7a}-, oder eine -CR^{7b}R^{7c}- Gruppe bedeutet, und
K² und K³
jeweils unabhängig voneinander eine -CH₂-, -CHR^{8a}-, oder eine - CR^{8b}R^{8c}- Gruppe bedeuten, wobei
R^{8a}/R^{8b}/R^{8c}
jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe bedeutet,
wobei
R^{7a} eine C₁₋₅-Alkylgruppe bedeutet, und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können,
und
insgesamt in der Formel (IIf) maximal vier Reste ausgewählt aus R^{7a}, R^{7b}, R^{7c}, R^{8a}, R^{8b} und R^{8c} vorhanden sein dürfen, und
R¹ ein Wasserstoffatom oder eine C₁₋₃-Alkyl- oder Cyclopropylgruppe
bedeutet, und in denen
A¹ CR¹⁰ bedeutet,
A² CR¹¹ bedeutet,
A³ CR¹² bedeutet,
A⁴ entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

7. Verbindungen der allgemeinen Formel (I) nach einem der Ansprüche 1 bis 6, in denen
M einen Thiophen-2-yl-Ring der Formel 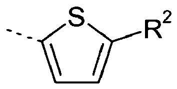 bedeutet, in dem
R² ein Chlor-, Bromatom oder eine Ethinyl-Gruppe darstellt,
deren Tautomere, deren Enantiomere, deren Diastereomere, deren Gemische und deren Salze.

8. Physiologisch verträgliche Salze der Verbindungen gemäß einem der Ansprüche 1 bis 7.

9. Arzneimittel, enthaltend eine Verbindung nach mindestens einem der Ansprüche 1 bis 7 oder ein physiologisch verträgliches Salz gemäß Anspruch 8, neben gegebenenfalls einem oder mehreren inerten Trägerstoffen und/oder Verdünnungsmitteln.

10. Verwendung einer Verbindung nach mindestens einem der Ansprüche 1 bis 7 oder eines physiologisch verträglichen Salzes gemäß Anspruch 8 zur Herstellung eines Arzneimittels zur Vorbeugung und Behandlung venöser und arterieller thrombotischer Erkrankungen.

11. Verfahren zur Herstellung eines Arzneimittels gemäß Anspruch 9, **dadurch gekennzeichnet, dass** auf nichtchemischem Wege eine Verbindung nach mindestens einem der Ansprüche 1 bis 7 oder ein physiologisch verträgliches Salz gemäß Anspruch 8 in einen oder mehrere inerte Trägerstoffe und/oder Verdünnungsmittel eingearbeitet wird.

## Claims

1. Compounds of general formula (I) 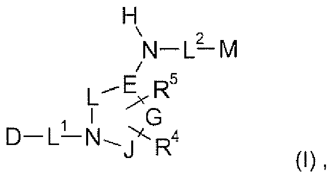 wherein denotes a substituted bicyclic ring system of formula (IIa), (IIb) or (IIc) 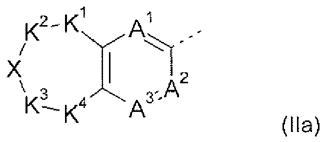 or 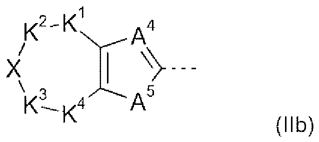 or 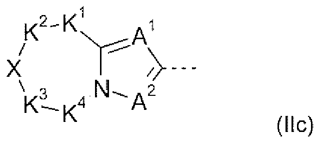 wherein
K¹ and K⁴
each independently denote a bond, a -CH₂, -CHR^{7a}, -CR^{7b}R^{7c} or a -C(O) group, and wherein
R^{7a}/R^{7b}/R^{7c}
each independently denote a fluorine atom, a hydroxy, C₁₋₅-alkyloxy, amino, C₁₋₅-alkylamino, di-(C₁₋₅-alkyl)-amino, C₃₋₅-cycloalkyleneimino, C₁₋₅-alkylcarbonylamino group,
a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇-cycloalkyleneiminocarbonyl-C₀₋₅-alkyl group,
wherein the two groups R^{7b}/R^{7c} may not simultaneously be bound to the cyclic carbon atom via a heteroatom, except that -C(R^{7b}R^{7C})- corresponds to a -CF₂ group, or
R^{7a} denotes a phenyl or monocyclic heteroaryl group substituted by fluorine, chlorine, bromine, methyl, methoxy, amino or nitro, or
two groups R^{7b}/R^{7c} together with the cyclic carbon atom may form a 3-, 4-, 5-, 6- or 7-membered saturated carbocyclic group or a cyclopentene, cyclohexene, oxetan, azetidine, thietan, tetrahydrofuran, pyrrolidine, tetrahydrothiophene, tetrahydropyran, piperidine, pentamethylene sulphide, hexamethyleneimine, 1,3-dioxolan, 1,4-dioxane, hexahydropyridazine, piperazine, thiomorpholine, morpholine, 2-imidazolidinone, 2-oxazolidinone, tetrahydro-2(1H)-pyrimidinone or [1,3]oxazinan-2-one ring,
wherein the methylene groups thereof may be substituted by 1-2 C₁₋₃-alkyl or CF₃- groups, and/or
the methylene groups thereof, if they are not bound to a heteroatom, may be substituted by 1-2 fluorine atoms, and/or wherein a -CH₂ group, besides being replaced by an N atom, may be replaced by a -CO group, and/or
the imino groups thereof may each be substituted by a C₁₋₃-alkyl or C₁₋₃-alkylcarbanyl group, and/or
wherein the sulphur atom may be oxidised to form a sulphoxide or sulphone group,
K² and K³
each independently denote a -CH₂, -CHR^{8a}, -CR^{8b}R^{8c} or a -C(O) group, wherein
R^{8a}/R^{8b}/R^{8c}
each independently denote a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl , C₁₋₅-alkylamino-C₁₋₅-alkyl , di-(C₁₋₅-alkyl)-amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇-cycloalkyleneiminocarbonyl-C₀₋₅-alkyl group,
or two groups R^{8b}/R^{8c} together with the cyclic carbon atom may form a 3-, 4-, 5-, 6- or 7-membered saturated carbocyclic group or a cyclopentene, cyclohexene, oxetan, azetidine, thietan, tetrahydrofuran, pyrrolidine, tetrahydrothiophene, tetrahydropyran, piperidine, pentamethylene sulphide, hexamethyleneimine, hexahydropyridazine, tetrahydro-2(1H)-pyrimidinone, [1,3]oxazinan-2-one ring,
wherein the methylene groups thereof may be substituted by 1-2 C₁₋₃-alkyl or CF₃- groups, and/or
the methylene groups thereof, if they are not bound to a heteroatom, may be substituted by 1-2 fluorine atoms, and/or wherein a -CH₂ group besides being replaced by a nitrogen atom may be replaced by a -CO group, and/or
the imino groups thereof may each be substituted by a C₁₋₃-alkyl or C₁₋₃-alkylcarbonyl group, and/or
wherein the sulphur atom may be oxidised to form a sulphoxide or sulphone group,
with the proviso that a heteroatom introduced by R^{8b} or R^{8c} must not be only one carbon atom away from X in formula (I), and
in all, in formula (IIa) or (IIb) or (IIc) a maximum of four groups selected from among R^{7a}, R^{7b}, R^{7c}, R^{8a}, R^{8b} and R^{8c} may be present, and
X denotes an oxygen or sulphur atom, a CF₂, sulphene, sulphone
or a NR¹ group, wherein
R¹ denotes a hydrogen atom or a hydroxy, C₁₋₃-alkyloxy, amino,
C₁₋₃-alkylamino, di-(C₁₋₃-alkyl)-amino , a C₁₋₅-alkyl, C₂₋₅-alkenyl-CH₂, C₂₋₅-alkynyl-CH₂, C₃₋₆-cycloalkyl, C₄₋₆-cycloalkenyl, oxetan-3-yl, tetrahydrofuran-3-yl, benzyl, C₁₋₅-alkyl-carbonyl, trifluoromethylcarbonyl, C₃₋₆-cycloalkyl-carbonyl, C₁₋₅-alkylsulphonyl, C₃₋₆-cycloalkyl-sulphonyl, aminocarbonyl, C₁₋₅-alkylaminocarbonyl, di-(C₁₋₅-alkyl)-aminocarbonyl, C₁₋₅-alkyloxycarbonyl, C₄₋₇-cycloalkyleneiminocarbonyl group,
wherein the methylene and methyl groups present in the groups mentioned previously may additionally be substituted by a C₁₋₃alkyl, carboxy, C₁₋₅-alkoxycarbonyl group, or by a hydroxy, C₁₋₅-alkyloxy, amino, C₁₋₅-alkylamino, C₁₋₅-dialkylamino or C₄₋₇-cycloalkyleneimino group, provided that the methylene or methyl groups are not directly bound to a heteroatom selected from among O, N and S, and/or one to three hydrogen atoms may be replaced by fluorine atoms, provided that the methylene or methyl groups are not directly bound to a heteroatom selected from among O, N and S,
and wherein
A¹ denotes either N or CR¹⁰,
A² denotes either N or CR¹¹,
A³ denotes either N or CR¹²,
A⁴ denotes either N or CR¹²,
A⁵ denotes NH, sulphur or oxygen,
while R¹⁰, R¹¹ and R¹² each independently denote
a hydrogen, fluorine, chlorine, bromine or iodine atom, or a C₁₋₅-alkyl, CF₃, C₂₋₅ -alkenyl, C₂₋₅-alkynyl , a phenyl, a cyano, carboxy, C₁₋₅-alkyloxycarbonyl, hydroxy, C₁₋₃-alkyloxy, CF₃O, CHF₂O, CH₂FO, amino, C₁₋₅-alkylamino, di-(C₁₋₅-alkyl)-amino or C₄₋₇-cycloalkyleneimino group, and
-L-E-G-J- denotes a -C-C-C-C group which may be substituted by R⁴ and R⁵, and
L¹ denotes a -C(O) group, and
R⁴ denotes a hydrogen atom or
a straight-chain or branched C₁₋₆-alkyl, C₂₋₆-alkenyl or C₂₋₆-alkynyl group, wherein the hydrogen atoms of the methylene and/or methyl fragments of the straight-chain or branched C₁₋₆-alkyl, C₂₋₆-alkenyl or C₂₋₆-alkynyl group may optionally be wholly or partly replaced by fluorine atoms, and/or
wherein the hydrogen atoms of the methylene and/or methyl fragments of the straight-chain or branched C₁₋₆-alkyl, C₂₋₆-alkenyl or C₂₋₆-alkynyl group may optionally each be substituted independently by one to two substituents selected from a C₃₋₅-cycloalkyl group, a nitrile, hydroxy or C₁₋₅-alkyloxy group, wherein the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₃₋₅-alkylaminocarbonyloxy, di-(C₁₋₅-alkyl)-aminocarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl, aminosulphonyl, C₁₋₅-alkylaminosulphonyl, di-(C₁₋₅-alkyl)-aminosulphonyl, C₄₋₇-cycloalkyleneiminosulphonyl, amino, C₁₋₅-alkylamino, di-(C₁₋₅-alkyl)-amino, C₁₋₅-alkylcarbonylamino, C₁₋₃-alkyloxy-C₁₋₂alkylcarbonylamino, C₁₋₃-alkyloxycarbonylamino, C₁₋₃-alkylaminocarbonylamino, C₁₋₅-alkylsulphonylamino, N-(C₁₋₅-alkylsulphonyl)-C₁₋₅-alkylamino, C₃₋₆-cycloalkylcarbonylamino group, or a morpholinyl, thiomorpholinyl, pyrrolidinyl, piperidinyl, piperazinyl, tetrahydrofuranyl, tetrahydropyranyl group, while the above-mentioned carbocyclic and heterocyclic groups in the ring may each be substituted by 1-4 C₁₋₃-alkyl or C₁₋₃-alkylcarbonyl groups or may each be substituted by 1-2 oxo groups, and/or
wherein the hydrogen atoms of the sp²-hybridise carbon atoms of the straight-chain or branched C₂₋₆-alkenyl group may optionally be wholly or partly replaced by fluorine atoms, or
a nitrile, carboxy, aminocarbonyl, C₁₋₅-alkylaminocarbonyl, C₃₋₆-cycloalkylaminocarbonyl, di-(C₁₋₅-alkyl)-aminocarbonyl, C₁₋₅-alkyloxycarbonyl or a C₄₋₇-cycloalkyleneiminocarbonyl group wherein a methylene group may optionally be replaced by an oxygen, sulphur or C₀₋₃-alkyl-substituted nitrogen atom, or
a phenyl, mono- or bicyclic heteroaryl, phenyl-C₁₋₅-alkyl or mono- or bicyclic heteroaryl-C₁₋₅-alkyl group,
which may optionally be mono- to trisubstituted in the phenyl or heteroaryl moiety by identical or different substituents selected from among fluorine, chlorine, bromine and iodine atoms, and C₁₋₅-alkyl, trifluoromethyl, amino, C₁₋₅-alkyl-amino, di-(C₁₋₅-alkyl)-amino, hydroxy, C₁₋₅-alkyloxy, mono-, di- or trifluoromethoxy, carboxy- and C₁₋₅-alkyloxycarbonyl group,
or if R⁴ is linked to G it may also denote a fluorine atom or a hydroxy, C₁₋₅-alkyl-oxy, C₂₋₅-alkenyl-oxy, C₂₋₅-alkynyl-oxy, C₃₋₆-cycloalkyl-oxy, C₁₋₅-alkylaminocarbonyloxy, di(C₁₋₅-alkyl)aminocarbonyloxy or C₄₋₇-cycloalkyleneiminocarbonyloxy, phenyl-C₀₋₃-alkyloxy, heteroaryl-C₀₋₃-alkyloxy, amino, C₁₋₅-alkylamino, di-(C₁₋₅-alkyl)-amino, C₄₋₇-cycloalkyleneimino, C₁₋₃-acylamino, (C₁₋₃-acyl)C₁₋₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonylamino-group,
wherein the methyl or methylene groups present in the above-mentioned alkyl or cycloalkyl groups may each independently be substituted by a substituent selected from among morpholinyl, thiomorpholinyl, pyrrolidinyl, piperidinyl, piperazinyl, tetrahydrofuranyl, tetrahydropyranyl, dimethylaminocarbonyl, C₁₋₅-alkyloxycarbonyl, carboxy, methyl, hydroxy, methoxy or amino, and
the above-mentioned phenyl or heteroaryl groups may optionally be mono- to trisubstituted by identical or different substituents selected from among fluorine, chlorine, bromine and iodine atoms, and C₁₋₅-alkyl, trifluoromethyl, amino, C₁₋₅-alkyl-amino, di-(C₁₋₅-alkyl)-amino, hydroxy, C₁₋₅-alkyloxy, mono-, di- or trifluoromethoxy, carboxy- and C₁₋₅-alkyloxycarbonyl group,
with the proviso that two heteroatoms selected from among oxygen and nitrogen are separated from one another by precisely one optionally substituted -CH₂ group, and/or
that two atoms form an -O-O or -S-O bond,
is excluded, and
R⁵ denotes a hydrogen atom, a C₁₋₅ alkyl, C₂₋₅alkenyl or C₂₋₅ alkynyl or a phenyl-C₀₋₅alkyl group, wherein the alkyl group may be substituted by a hydroxy, methoxy, hydroxycarbonyl or C₁₋₅alkoxycarbonyl group,
or if R⁵ is linked to G it may also denote a hydroxy or methoxy group, or
R⁴ and R⁵ provided that they are bound to the same carbon atom, may form, together with the carbon atom, a -C=O group or a -CF₂ group, or
R⁴ and R⁵ provided that they are bound to the same carbon atom or to two adjacent carbon atoms, may form, together with the carbon atom or atoms a 3-7-membered carbocyclic group or a monounsaturated 5-7 membered carbocyclic group
wherein one of the carbon chain members of this cyclic group may be replaced by an oxygen or sulphur atom or an -NH, -N(C₁₋₅-alkyl), -N(C₁₋₄-alkylcarbonyl) or a carbonyl, sulphinyl or sulphonyl group, and/or
wherein two directly adjacent carbon chain members of these C₄₋₇-carbocyclic groups may together be replaced by a -C(O)NH, -C(O)N(C₁₋₅-alkyl), -S(O)₂NH or -S(O)₂N(C₁₋₅-alkyl) group, and/or
wherein four directly adjacent carbon chain members of these C₅₋₇-carbocyclic groups may together be replaced by a -O-CH₂-CH₂-O group, and/or
wherein 1 to 3 carbon atoms of these 3-7-membered cyclic groups may optionally each be substituted independently of one another by one or two fluorine atoms or one or two C₁₋₅-alkyl groups or a hydroxy, C₁₋₅-alkyloxy, C₁₋₅-alkylcarbonyloxy, amino, C₁₋₅-alkylamino, di-(C₁₋₅-alkyl)-amino, C₄₋₇-cycloalkyleneimino, C₁₋₅-alkylcarbonylamino, C₃₋₆-cycloalkylcarbonylamino, nitrile, carboxy-C₁₋₅-alkyl, C₁₋₅-alkyloxycarbonyl-C₁₋₅-alkyl, carboxy, C₁₋₅-alkyloxycarbonyl, aminocarbonyl, C₁₋₅-alkylaminocarbonyl, di-(C₁₋₅-alkyl)-aminocarbonyl or C₄₋₇-cycloalkyleneiminocarbonyl group,
with the proviso that a cyclic group formed from R⁴ and R⁵ together,
wherein two nitrogen atoms or one nitrogen and one oxygen atom in the cyclic group are separated from one another by precisely one optionally substituted -CH₂ group, and/or
wherein two atoms in the ring form an -O-O or -S-O- bond,
is excluded, and
L² denotes a -C(O) group, and
M denotes a phenyl, pyridyl, thienyl or furyl ring optionally substituted by R² and R³, wherein
R² denotes a fluorine, chlorine, bromine or iodine atom or a methyl, ethyl, vinyl, methoxy, ethynyl, cyano or -C(O)NH₂ group, and
R³ denotes a hydrogen, fluorine, chlorine, bromine or iodine atom or a hydroxy, methoxy, trifluoromethoxy group, or a C₁₋₃-alkyl group optionally substituted by fluorine atoms, or a cyano, amino or NH₂C(O) group,
while, unless stated otherwise, by the term "heteroaryl group" mentioned in the definitions hereinbefore is meant a monocyclic 5- or 6-membered heteroaryl group,
wherein
the 6-membered heteroaryl group contains one, two or three nitrogen atoms, and
the 5-membered heteroaryl group contains an imino group optionally substituted by a C₁₋₃-alkyl group or an oxygen or sulphur atom, or
an imino group optionally substituted by a C₁₋₃-alkyl group or an oxygen or sulphur atom and additionally one or two nitrogen atoms, or
an imino group optionally substituted by a C₁₋₃-alkyl group and three nitrogen atoms,
and moreover a phenyl ring optionally substituted by a fluorine, chlorine or bromine atom or by a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms,
and the bond is effected via a nitrogen atom or a carbon atom of the heterocyclic moiety or a fused-on phenyl ring,
and wherein, unless stated otherwise, by the term "halogen atom" mentioned in the definitions hereinbefore is meant an atom selected from among fluorine, chlorine, bromine and iodine,
and wherein, unless stated otherwise, the alkyl, alkenyl, alkynyl and alkyloxy groups contained in the definitions mentioned previously which have more than two carbon atoms may be straight-chain or branched and the alkyl groups in the previously mentioned dialkylated groups, for example the dialkylamino groups, may be identical or different,
and the hydrogen atoms of the methyl or ethyl groups contained in the foregoing definitions, unless otherwise stated, may be wholly or partly replaced by fluorine atoms,
the tautomers, enantiomers, diastereomers, mixtures and salts thereof.

2. Compounds of general formula (I) according to claim 1, wherein D, E, G, J, L, L¹, L² and M are defined as in claim 1, and wherein
R⁴ denotes a hydrogen atom or
a straight-chain or branched C₁₋₆-alkyl group,
wherein the hydrogen atoms of the methylene and/or methyl fragments of the straight-chain or branched C₁₋₆-alkyl group may optionally be wholly or partly replaced by fluorine atoms, and/or wherein the hydrogen atoms of the methylene and/or methyl fragments of the straight-chain or branched C₁₋₆-alkyl group may optionally each independently be substituted by a substituent selected from a hydroxy, C₁₋₅-alkyloxy, C₁₋₅-alkylaminocarbonyloxy, di-(C₁₋₅-alkyl)-aminocarbonyloxy, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl , amino, C₁₋₅-alkylamino, di-(C₁₋₅-alkyl)-amino, C₁₋₅-alkylcarbonylamino, C₁₋₃-alkyloxy-C₁₋₂alkylcarbonylamino, C₁₋₃-alkyloxycarbonylamino, C₁₋₃-alkylaminocarbonylamino, C₁₋₅-alkylsulphonylamino, N-(C₁₋₅-alkylsulphonyl)-C₁₋₅-alkylamino, C₃₋₆-cycloalkylcarbonylamino group, or
a nitrile, carboxy, aminocarbonyl, C₁₋₅-alkylaminocarbonyl, C₃₋₆-cycloalkylaminocarbonyl, di-(C₁₋₅-alkyl)-aminocarbonyl, C₁₋₅-alkyloxycarbonyl or a C₄₋₇-cycloalkyleneiminocarbonyl group wherein a methylene group may optionally be replaced by an oxygen, sulphur or C₀₋₃-alkyl-substituted nitrogen atom, and
or if R⁴ is linked to G it may also denote a fluorine atom or a hydroxy, C₁₋₅-alkyl-oxy, C₂₋₅-alkenyl-oxy, C₂₋₅-alkynyl-oxy, C₃₋₆-cycloalkyl-oxy, C₁₋₅-alkylaminocarbonyloxy, di(C₁₋₅-alkyl)aminocarbonyloxy or C₄₋₇-cycloalkyleneiminocarbonyloxy, phenyl-C₀₋₂-alkyloxy group, amino, C₁₋₅-alkylamino, di-(C₁₋₅-alkyl)-amino, C₄₋₇-cycloalkyleneimino, C₁₋₃-acylamino, (C₁₋₃-acyl)C₁₋₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonylamino group,
wherein the methyl or methylene groups present in the above-mentioned alkyl or cycloalkyl groups may each independently be substituted by a substituent selected from among dimethylaminocarbonyl, C₁₋₅-alkyloxycarbonyl, carboxy, methyl, hydroxy, methoxy or amino,
with the proviso that two heteroatoms selected from among oxygen and nitrogen are separated from one another by precisely one optionally substituted -CH₂ group, and/or
that two atoms form an -O-O or -S-O- bond, is excluded, and
R⁵ denotes a hydrogen atom or a C₁₋₅ alkyl, allyl, propargyl or benzyl group, or if R⁵ is linked to G, it may also denote a hydroxy or methoxy group, or
R⁴ and R⁵ if they are bound to the same carbon atom, may form, together with the carbon atom, a -C=O group or a -CF₂- group, or
R⁴ and R⁵ if they are bound to the same carbon atom or to two adjacent carbon atoms, may form, together with the carbon atom or atoms, a 3-7-membered carbocyclic group,
wherein one of the carbon chain members of this cyclic group may be replaced by an oxygen or sulphur atom or a -NH, -N(C₁₋₅-alkyl), -N(C₁₋₄-alkylcarbonyl) or a carbonyl, sulphinyl or sulphonyl group, and/or
wherein two directly adjacent carbon chain members of these C₄₋₇-carbocyclic groups may together be replaced by a -C(O)NH, -C(O)N(C₁₋₅-alkyl), -S(O)₂NH or -S(O)₂N(C₁₋₅-alkyl) group, and/or
wherein four directly adjacent carbon chain members of these C₅₋₇-carbocyclic groups may together be replaced by a -O-CH₂-CH₂O group,
with the proviso that a cyclic group formed from R⁴ and R⁵ together,
wherein two nitrogen atoms or one nitrogen and one oxygen atom in the cyclic group are separated from one another by precisely one optionally substituted -CH₂ group, and/or
wherein two atoms in the ring form an -O-O or -S-O bond,
is excluded,
the tautomers, enantiomers, diastereomers, mixtures and salts thereof.

3. Compounds of general formula (I) according to claim 1 or 2, wherein E, G, J, L, L¹, L², M, R⁴ and R⁵ are defined as in claim 1 or 2, and wherein
D denotes a substituted bicyclic ring system of formula (IIa) or (IIb) 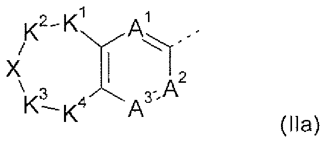 or 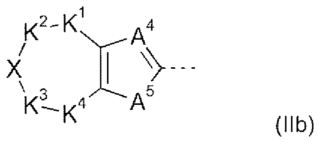 wherein
K¹ and K⁴
each independently denote a bond, a -CH₂, -CHR^{7a}, -CR^{7b}R^{7c} or a -C(O) group, and wherein
R^{7a}/R^{7b}/R^{7c}
each independently denote a fluorine atom, a hydroxy, C₁₋₅-alkyloxy or a C₁₋₅-alkyl group,
wherein the two groups R^{7b}/R^{7c} may not simultaneously be bound to the cyclic carbon atom via a heteroatom, except where -C(R^{7b}R^{7c})-corresponds to a -CF₂ group, or
two groups R^{7b}/R^{7c} together with the cyclic carbon atom may form a 3-membered carbocyclic group,
with the proviso that K¹ and K⁴ simultaneously denote a bond, is excluded, and
K² and K³
each independently denote a -CH₂, -CHR^{8a}, -CR^{8b}R^{8c} or a -C(O)-group, wherein
R^{8a}/R^{8b}/R^{8c}
each independently denote a C₁₋₅-alkyl group,
and/or
two groups R^{8b}/R^{8c} together with the cyclic carbon atom may form a 3-membered saturated carbocyclic group
and
in all in formulae (IIa) or (IIb) not more than four groups selected from among R^{7a}, R^{7b}, R^{7c}, R^{8a}, R^{8b} and R^{8c} may be present, and
X denotes an oxygen or sulphur atom, a -CF₂-
or a NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂ or a C₃₋₆-cycloalkyl group,
and wherein
A¹ denotes either N or CR¹⁰,
A² denotes either N or CR¹¹,
A³ denotes either N or CR¹²,
A⁴ denotes either N or CR¹²,
A⁵ denotes NH, sulphur or oxygen,
wherein R¹⁰, R¹¹ and R¹² each independently denote
a hydrogen, fluorine, chlorine, bromine or iodine atom, or a C₁₋₅-alkyl, CF₃, a cyano, carboxy, C₁₋₅-alkyloxycarbonyl, hydroxy, C₁₋₃-alkyloxy, CF₃O, CF₂O, CH₂FO, amino, C₁₋₅-alkylamino, di-(C₁₋₅-alkyl)-amino or C₄₋₇-cycloalkyleneimino group,
the tautomers, enantiomers, diastereomers, mixtures and salts thereof.

4. Compounds of general formula (I) according to one of claims 1 to 3, wherein
X denotes a NR¹ group, wherein
R¹ denotes a hydrogen atom or a C₁₋₅-alkyl, allyl or cyclopropyl group, and
A¹ denotes CR¹⁰,
**A²** denotes CR¹¹,
A³ denotes CR¹²,
A⁴ denotes either N or CR¹²,
A⁵ denotes sulphur,
while R¹⁰, R¹¹ and R¹² each independently denote
a hydrogen, fluorine or chlorine atom, or a methyl, CF₃, hydroxy, methoxy, CF₃O, CHF₂O, CH₂FO group,
the tautomers, enantiomers, diastereomers, mixtures and salts thereof.

5. Compounds of general formula (I) according to one of claims 1 to 4, wherein
D denotes a substituted bicyclic ring system of formula 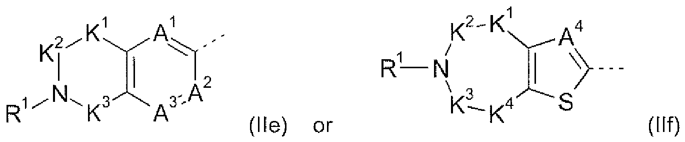 wherein
K¹ denotes a -CH₂, -CHR^{7a}, or a -CR^{7b}R^{7c}- group, and
K² and K³
each independently denote a -CH₂, -CHR^{8a} or a -CR^{8b}R^{8c}- group, wherein
R^{8a}/R^{8b}/R^{8c} each independently denote a C₁₋₅-alkyl group, and
K⁴ denotes a bond, a -CH₂, -CHR^{7a} or a -CR^{7b}R^{7c} group,
wherein
R^{7a} denotes a C₁₋₅-alkyl group and
R^{7b}/R^{7c} each independently denote a hydroxy, C₁₋₅-alkyloxy or a C₁₋₅-alkyl group,
wherein the two groups R^{7b}/R^{7c} may not simultaneously be bound to the cyclic carbon atom via an oxygen atom, and
in all, in formulae (IIe) or (IIf) not more than four groups selected from among R^{7a}, R^{7b}, R^{7c}, R^{8a}, R^{8b} and R^{8c} may be present, and
R¹ denotes a hydrogen atom or a C₁₋₃-alkyl, allyl or cyclopropyl group,
and wherein
A¹ denotes CR¹⁰,
A² denotes CR¹¹,
A³ denotes CR¹²,
A⁴ denotes either N or CR¹²,
while R¹⁰, R¹¹ and R¹² each independently denote
a hydrogen, fluorine or chlorine atom, or a methyl, CF₃, hydroxy, methoxy, CF₃O, CHF₂O, CH₂FO group, and
-L-E-G-J- denotes a -C-C-C-C group which may be substituted by R⁴ and R⁵, and
R⁴ denotes a hydrogen atom or
a straight-chain or branched C₁₋₃-alkyl group,
wherein the hydrogen atoms of the methylene and/or methyl fragments of the straight-chain or branched C₁₋₆-alkyl group may optionally be substituted independently of one another by a substituent selected from a hydroxy, C₁₋₅-alkyloxy, C₁₋₅-alkylaminocarbonyloxy, di-(C₁₋₅-alkyl)-aminocarbonyloxy, carboxy, C₁₋₅-alkyloxycarbonyl, C₁₋₃-alkyloxy-C₁₋₂alkylcarbonylamino, C₁₋₃-alkyloxycarbonylamino, C₁₋₃-alkylaminocarbonylamino, C₁₋₅-alkylcarbonylamino, C₁₋₅-alkylsulphonylamino group, or
if R⁴ is linked to G, it may also denote a fluorine atom or a hydroxy, methoxy, C₃₋₅-alkenyl-oxy, C₂₋₅-alkyl-oxy, C₃₋₆-cycloalkyl-oxy, benzyloxy, C₁₋₅-alkylaminocarbonyloxy, di(C₁₋₅-alkyl)aminocarbonyloxy or a C₄₋₇-cycloalkyleneiminocarbonyloxy group,
with the proviso that
two heteroatoms selected from among oxygen and nitrogen are separated from one another by precisely one optionally substituted -CH₂ group,
is excluded, and
R⁵ denotes a hydrogen atom or a C₁₋₅ alkyl, allyl, benzyl or phenyl group, or if R⁵ is linked to G, it may also denote a hydroxy or methoxy group, or
R⁴ and R⁵ if they are bound to the same carbon atom, may form together with the carbon atom a -C=O group, or a -CF₂- group, or
R⁴ and R⁵ if they are bound to the same carbon atom or to two adjacent carbon atoms, may form together with the carbon atom or atoms a 3-6-membered carbocyclic group,
wherein four directly adjacent carbon chain members of these C₅₋₆-carbocyclic groups may together be replaced by a -O-CH₂-CH₂O group,
the tautomers, enantiomers, diastereomers, mixtures and salts thereof.

6. Compounds of general formula (I) according to one of claims 1 to 5, wherein
D denotes a substituted bicyclic ring system of formula 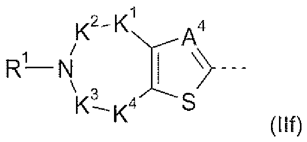 wherein
K¹ denotes a -CH₂, -CHR^{7a}, or a -CR^{7b}R^{7c}- group, and
K² and K³
each independently denote a -CH₂, -CHR^{8a}, or a -CR^{8b}R^{8c}- group, wherein
R^{8a}/R^{8b}/R^{8c} each independently denote a C₁₋₅-alkyl group, and
K⁴ denotes a bond, a -CH₂, -CHR^{7a}, or a -CR^{7b}R^{7c}- group, wherein
R^{7a} denotes a C₁₋₅-alkyl group, and
R^{7b}/R^{7c} each independently denote a hydroxy, C₃₋₅-alkyloxy or a C₁₋₅-alkyl group,
wherein the two groups R^{7b}/R^{7c} may not simultaneously be bound to the cyclic carbon atom via an oxygen atom,
and
in all, in formula (IIf) not more than four groups selected from among R^{7a}, R^{7b}, R^{7c}, R^{8a}, R^{8b} and R^{8c} may be present, and
R¹ denotes a hydrogen atom or a C₁₋₃-alkyl or cyclopropyl group, and wherein
A¹ denotes CR¹⁰,
A² denotes CR¹¹,
A³ denotes CR¹²,
A⁴ denotes either N or CR¹²,
wherein R¹⁰, R¹¹ and R¹² each independently denote
a hydrogen, fluorine or chlorine atom, or a methyl, CF₃, hydroxy, methoxy, CF₃O, CHF₂O, CH₂FO group,
the tautomers, enantiomers, diastereomers, mixtures and salts thereof.

7. Compounds of general formula (I) according to one of claims 1 to 6, wherein M denotes a thiophen-2-yl ring of formula 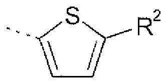 wherein
R² denotes a chlorine or bromine atom or an ethynyl group,
the tautomers, enantiomers, diastereomers, mixtures and salts thereof.

8. Physiologically acceptable salts of the compounds according to one of claims 1 to 7.

9. Medicaments, containing a compound according to at least one of claims 1 to 7 or a physiologically acceptable salt according to claim 8, optionally in addition to one or more inert carriers and/or diluents.

10. Use of a compound according to at least one of claims 1 to 7 or a physiologically acceptable salt according to claim 8 for preparing a medicament for the prevention and treatment of venous and arterial thrombotic diseases.

11. Method of preparing a medicament according to claim 9, **characterised in that** a compound according to at least one of claims 1 to 7 or a physiologically acceptable salt according to claim 8 is incorporated in one or more inert carriers and/or diluents by a non-chemical method.

## Revendications

1. Composés de formule générale (I) 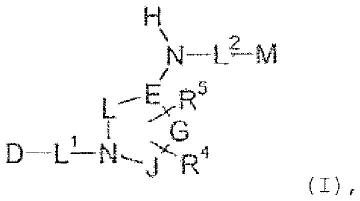 dans lesquels
D représente un système cyclique bicyclique substitué de formule (IIa), (IIb) ou (IIc) 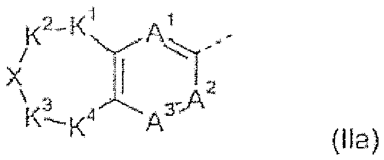 ou 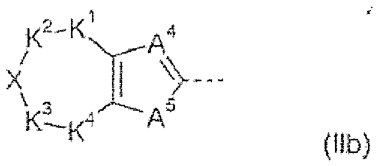 ou 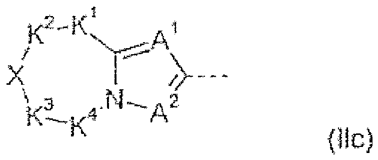 dans lequel
K¹ et K⁴
représentent respectivement, indépendamment les uns des autres, une liaison, une groupe -CH₂-, -CHR^{7a}-, CR^{7b}R^{7c}- ou un groupe -C(O)- et où
R^{7a}/R^{7b}/R^{7c}
représentent respectivement indépendamment les uns des autres, un atome de fluor, un groupe hydroxy, alkyloxy en C₁ à C₅, amino, alkylamino en C₁ à C₅, di-(alkyle en C₁ à C₅)-amino, cycloalkylène-imino en C₃ à C₅, alkylcarbonylamino en C₁ à C₅,
un groupe alkyle en C₁ à C₅ qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇-alkyle en C₁ à C₅, carboxy-alkyle en C₀ à C₅, alkyloxycarbonyle en C₁ à C5-alkyle en C₀ à C₅, aminocarbonyl-al kyle en C₀ à C₅, alkylaminocarbonyle en C₁ à C₅-alkyle en C₀ à C₅, di(alkyle en C₁ à C₅)-aminocarbonyl-alkyle en C₀ à C₅ ou cycloalkylène-iminocarbonyle en C₄ à C₇-alkyle en C₀ à C₅,
où les deux radicaux R^{7b}/R^{7c} ne peuvent pas être liés en même temps par un hétéroatome à l'atome de carbone cyclique, sauf si -C(R^{7b}R^{7c})- correspond à un groupe -CF₂-,
ou
R^{7a} désigne un groupe phényle substitué par un atome de fluor, de chlore, de brome, un groupe méthyle, methoxy, amino ou nitro, ou un groupe hétéroaryle monocyclique, ou
deux radicaux R^{7b}/R^{7c} pouvant former conjointement avec l'atome de carbone cyclique, un carbocycle saturé de 3, 4, 5, 6 ou 7 chaînons ou un groupe cyclopentène, cyclohexène, oxétane, azétidine, thiétane, tétrahydrofurane, pyrrolidine, tétrahydrothiophène, tétrahydropyrane, pipéridine, pentaméthylènesulfure, hexaméthylène-imine, 1,3-dioxolane, 1,4-dioxane, hexahydropyridazine, pipérazine, thiomorpholine, morpholine, 2-imidazolidinone, 2-oxazolidinone, tétrahydro-2(1H)-pyrimidinone ou [1,3]oxazinan-2-one
leur groupe méthylène pouvant être substitués par 1 à 2 groupes alkyle en C₁ à C₃ ou CF₃, et/ou leurs groupes méthylènes, dans la mesure où ils ne sont pas liés à un hétéroatome, peuvent être substitués par 1 à 2 atomes de fluor, et/ou
dans lequel un groupe -CH₂- à côté d'un atome de N peut être substitué par un groupe -CO-, et/ou leurs groupes imino pouvant être substitués respectivement par un groupe alkyle en C₁ à C₃ ou alkylcarbonyle en C₁ à C₃, et/ou
où l'atome de soufre peut être oxydé en un sulfoxyde ou un groupe sulfone,
K² et K³
désignent respectivement, indépendamment l'un de l'autre, un groupe -CH₂-, -CHR^{8a}-, -CR^{8b}R^{8c}- ou un groupe -C(O)-, dans lequel
R^{8a}/R^{8b}/R^{8c}
représentent respectivement, indépendamment les uns des autres, un groupe alkyle en C₁ à C₅ qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇-alkyle en C₁ à C₅, carboxy-alkyle en C₀ à C₅, alkyloxycarbonyle en C₁ à C₅-alkyle en C₀ à C₅, aminocarbonyl-alkyle en C₀ à C₅, alkylaminocarbonyle en C₁ à C₅-alkyle en C₀ à C₅, di-(alkyle en C₁ à C₅)-aminocarbonyl-alkyle en C₀ à C₅ ou cycloalkylène-iminocarbonyl-alkyle en C₀ à C₅ ou cycloalkylène-iminocarbonyle en C₄ à C₇-alkyle en C₀ à C₅,
ou deux radicaux R^{8b}/R^{8c} peuvent former conjointement avec l'atome de carbone cyclique, un carbocycle saturé de 3, 4, 5, 6 ou 7 chaînons ou un groupe cyclopentène, cyclohexène, oxétane, azétidine, thiétane, tétrahydrofurane, pyrrolidine, tétrahydrothiophène, tétrahydropyrane, pipéridine, pentaméthylènesulfure, hexaméthylène-imine, hexahydropyridazine, tétrahydro-2(1H)-pyrimidinone, [1,3]oxazinan-2-one,
leurs groupes méthylène pouvant être substitués par 1 à 2 groupes alkyle en C₁ à C₃ ou CF₃, et/ou leurs groupes méthylène, dans la mesure où ils ne sont pas liés à un hétéroatome, peuvent être substitués par 1 à 2 atomes de fluor, et/ou
dans lequel un groupe -CH₂- à côté d'un atome de N peut être substitué par un groupe -CO-, et/ou leurs groupes imino pouvant être substitués respectivement par un groupe alkyle en C₁ à C₃ ou alkylcarbonyle en C₁ à C₃, et/ou dans lequel l'atome de soufre peut être oxydé en un sulfoxyde ou un groupe sulfone,
à condition qu'un hétéroatome apporté par R^{8b} ou R^{8c} ne puisse pas être éliminé par uniquement un atome de carbone de X dans la formule (I), et
au total dans la formule (IIa) ou (IIb) ou (IIc), au maximum, quatre radicaux choisis parmi R^{7a}, R^{7b}, R^{7c}, R^{8a}, R^{8b} et R^{8c} pouvant être présents,
et
X désigne un atome d'oxygène ou de soufre, un groupe CF₂, sulfène, sulfone ou NR¹, dans lequel
R¹ désigne un atome d'hydrogène ou un groupe hydroxy, alkyloxy en C₁ à C₃, amino, alkylamino en C₁ à C₃, di-(alkyle en C₁ à C₃)-amino, un groupe alkyle en C₁ à C₅, alcényle en C₂ à C₅-CH₂-, alcinyle en C₂ à C₅-CH₂, cycloalkyle en C₃ à C₆, cycloalcényle en C₄ à C₆, oxétan-3-yle, tétrahydrofuran-3-yle, benzyle, alkyle en C₁ à C₅-carbonyle, trifluorométhylcarbonyle, cycloalkyle en C₃ à C₆-carbonyle, alkyle en C₁ à C₅-sulfonyle, cycloalkyle en C₃ à C₆-sulfonyle, aminocarbonyle, alkylaminocarbonyle en C₁ à C₅, di-(alkyle en C₁ à C₅) -aminocarbonyle, alkyloxycarbonyle en C₁ à C₅, cycloalkylène-iminocarbonyle en C₄ à C₇,
où les groupes méthylène ou méthyle se trouvant dans les groupes précédemment cités peuvent être en outre substitués par un groupe alkyl en C₁ à C₃, carboxy, alcoxycarbonyle en C₁ à C₅, ou par un groupe hydroxy, alkyloxy en C₁ à C₅, amino, alkylamino en C₁ à C₅, dialkylamino en C₁ à C₅ ou cyloalkylène-imino en C₄ à C₇, dans la mesure où les groupes méthylène ou méthyle ne sont pas directement liés à un hétéroatome du groupe de O, N ou S, et/ou un à trois atomes d'hydrogène peuvent être remplacés par des atomes de fluor, dans la mesure où les groupes méthylène ou méthyle ne sont pas liés directement à un hétéroatome du groupe O, N ou S,
et dans lequel
A¹ désigne soit N soit CR¹⁰,
A² désigne soit N soit CR¹¹,
A³ désigne soit N soit CR¹²,
A⁴ désigne soit N soit CR¹²,
A⁵ désigne NH, un atome de soufre ou d'oxygène,
où R¹⁰, R¹¹ et R¹² désignent respectivement, indépendamment les uns des autres
un atome d'hydrogène, de fluor, de chlore, de brome ou d'iode ou un groupe alkyle en C₁ à C₅, CF₃, alcényle en C₂ à C₅, alcinyle en C₂ à C₅, un groupe phényle, cyano, carboxy, alkyloxycarbonyle en C₁ à C₅, hydroxy, alkyloxy en C₁ à C₃, CF₃O, CHF₂O, CH₂FO, amino, alkylamino en C₁ à C₅, di-(alkyle en C₁ à C₅)-amino ou cycloalkylène-imino en C₄ à C₇, et
-L-E-G-J- désigne un groupe -C-C-C-C- qui peut être substitué par R⁴ ou R⁵, et
L¹ représente un groupe -C(O)-, et
R⁴ représente un atome d'hydrogène ou
un groupe alkyle en C₁ à C₆, alcènyle en C₂ à C₆ ou alcinyle en C₂ à C₆ linéaire ou ramifié,
où les atomes d'hydrogène des fragments méthylène et/ou méthyle du groupe alkyle en C₁ à C₆, alcényle en C₂ à C₆ ou alcinyle en C₂ à C₆ à chaîne linéaire ou ramifiée peuvent être remplacés éventuellement, en totalité ou en partie par des atomes de fluor, et/ou
où les atomes d'hydrogène des fragments méthylène et/ou méthyle du groupe alkyle en C₁ à C₆, alcényle en C₂ à C₆ ou alcinyle en C₂ à C₆ à chaîne linéaire ou ramifiée peuvent être substitués éventuellement, respectivement, indépendamment les uns des autres, par un à deux substituants choisis dans un groupe cycloalkyle en C₃ à C₅, un groupe nitrile, hydroxy
ou alkyloxy en C₁ à C₅, les atomes d'hydrogène du groupe alkyloxy en C₁ à C₅ pouvant éventuellement être remplacés totalement ou en partie par des atomes de fluor, un groupe allyloxy, propargyloxy, benzyloxy, alkylcarbonyloxy en C₁ à C₅, alkylaminocarbonyloxy en C₁ à C₅, di-(alkyle en C₁ à C₅)-aminocarbonyloxy, alkyloxycarbonyloxy en C₁ à C₅, carboxyalkyloxy en C₁ à C₅, alkyloxycarbonyle en C₁ à C₅-alkyloxy en C₁ à C₅, mercapto, alkylsulfanyle en C₁ à C₅, alkylsulfinyle en C₁ à C₅, alkylsulfonyle en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle, cycloalkylène-iminocarbonyle en C₄ à C₇, aminosulfonyle, alkylaminosulfonyle en C₁ à C₅, di-(alkyle en C₁ à C₅)-aminosulfonyle, cycloalkylène-iminosulfonyle en C₄ à C₇, amino, alkylamino en C₁ à C₅, di- (alkyle en C₁ à C₅)-amino, alkylcarbonylamino en C₁ à C₅, alkyloxy en C₁ à C₃-alkylcarbonylamino en C₁ à C₂, alkyloxycarbonylamino en C₁ à C₃, alkylaminocarbonylamino en C₁ à C₃, alkylsulfonylamino en C₁ à C₅, N-(alkylsulfonyle en C₁ à C₅)-alkylamino en C₁ à C₅, cycloalkylcarbonyle-amino en C₃ à C₆, ou un groupe morpholinyle, thiomorpholinyle, pyrrolidinyle, pipéridinyle, pipérazinyle, tétrahydrofuranyle, tétrahydropyranyle, tétrahydropyranyle, les carbocycles et hétérocycles précédemment cités pouvant être substitués dans le cycle, respectivement par 1 à 4 groupes alkyle en C₁ à C₃ ou alkylcarbonyle en C₁ à C₃ ou respectivement par 1 à 2 groupes oxo, et/ou
où les atomes d'hydrogènes des atomes de carbone hybridées par sp² du groupe alcényle en C₂ à C₆ à chaîne linéaire ou ramifiée peuvent éventuellement être remplacés totalement ou en partie par des atomes de fluor, ou
un groupe nitrite, carboxy-, aminocarbonyle, alkylaminocarbonyle en C₁ à C₇, cycloalkylaminocarbonyle en C₃ à C₆, di-(alkyle en C₁ à C₅)-aminocarbonyle, alkyloxycarbonyle en C₁ à C₅ ou un groupe cycloalkylène-iminocarbonyle en C₄ à C₇ pouvant être substitué éventuellement dans le groupe méthylène, par un atome d'oxygène, de soufre ou un groupe alkyle en C₀ à C₃,
ou
un groupe phényle, hétéroaryle mono- ou bicyclique, phényl-alkyle en C₁ à C₅ ou hétéroaryl-alkyle en C₁ à C₅ mono- ou bicyclique,
qui peut être substitué dans la partie phényle ou hétéroaryle, éventuellement une à trois fois par des substituants identiques ou différents choisis dans le groupe comprenant des atomes de fluor, de chlore, de brome, d'iode, et un groupe alkyle en C₁ à C₅, trifluorométhyle, amino, alkyle en C₁ à C₅-amino-, di-(alkyle en C₁ à C₅)-amino, hydroxy, alkyloxy en C₁ à C₅, mono-, di- ou trifluorométhoxy, carboxy et alkyloxycarbonyle en C₁ à C₅,
ou, dans la mesure où R⁴ est relié à G, peut représenter également un atome de fluor, ou un groupe hydroxy, alkyle en C₁ à C₅-oxy, alcényle en C₂ à C₅-oxy, alcinyle en C₂ à C₅-oxy, cycloalkyle en C₃ à C₆-oxy, alkylaminocarbonyloxy en C₁ à C₅, di(alkyle en C₁ à C₅)aminocarbonyloxy ou cycloalkylène-iminocarbonyloxy en C₄ à C₇, phénylalkyloxy en C₀ à C₃, hétéroaryl-alkyle en C₀ à C₃-oxy, amino, alkylamino en C₁ à C₅, di-(alkyle en C₁ à C₅)-amino, cycloalkylène-imino en C₄ à C₇, acylamino en C₁ à C₃, (acyle en C₁ à C₃)-alkylamino en C₁ à C₃, alkyloxycarbonylamino en C₁ à C₅, alkylaminocarbonylamino en C₁ à C₅, di(alkyle en C₁ à C₅)aminocarbonylamino ou cycloalkylène-iminocarbonylamino en C₄ à C₇,
où les groupes méthyle ou méthylène présents dans les radicaux alkyle ou cycloalkyle précédemment cités, peuvent être substitués respectivement indépendamment les uns des autres par un substituant choisi dans le groupe comprenant les groupes morpholinyle, thiomorpholinyle, pyrrolidinyle, pipéridinyle, pipérazinyle, tétrahydrofuranyle, tétrahydropyranyle, diméthylaminocarbonyle, alkyloxycarbonyle en C₁ à C₅, carboxy, méthyle, hydroxy, methoxy ou amino, et
les radicaux phényle ou hétéroaryle précédemment cités pouvant être substitués éventuellement une à trois fois par des substituants identiques ou différents choisis dans le groupe comprenant des atomes de fluor, de chlore, de brome et d'iode, et un groupe alkyle en C₁ à C₅, trifluorométhyle, amino, alkyle en C₁ à C₅-amino, di-(alkyle en C₁ à C₅)amino, hydroxy, alkyloxy en C₁ à C₅, mono-, di ou trifluorométhoxy, carboxy et alkyloxycarbonyle en C₁ à C₅,
à condition que deux hétéroatomes du groupe comprenant oxygène et l'azote séparés l'un de l'autre par exactement un groupe -CH₂- éventuellement substitué et/ou
que deux atomes formant une liaison -O-O- ou -S-O-, soient exclus, et
R⁵ représente un atome d'hydrogène, un groupe alkyle en C₁ à C₅, alcényle en C₂ à C₅ ou alcinyle en C₂ à C₅ ou
un groupe phényl-alkyle en C₀ à C₅, le groupe alkyle pouvant être substitué par un groupe hydroxy, méthoxy, hydroxycarbonyle ou alcoxycarbonyle en C₁ à C₅,
ou, dans la mesure où R⁵ est relié à G, peut également représenter un groupe hydroxy ou méthoxy, ou
R⁴ et R⁵, dans la mesure où ils sont liés au même atome de carbone, peuvent former conjointement avec l'atome de carbone, un groupe -C=O ou un groupe -CF₂, ou
R⁴ et R⁵, dans la mesure où ils sont liés au même atome de carbone ou à deux atomes de carbone voisins, peuvent former conjointement avec le ou les atome (s) de carbone, un carbocycle de 3 à 7 chaînons ou un carbocycle de 5 à 7 chaînons mono-insaturé,
où l'un des chaînons de carbone de ce cycle peut être remplacé par un atome d'oxygène ou de soufre ou un groupe -NH-, -N(alkyle en C₁ à C₅), N(alkylcarbonyle en C₁ à C₄) ou un groupe carbonyle, sulfinyle ou sulfonyle, et/ou
où deux chaînons de carbone directement voisins de ces carbocycles en C₄ à C₇ peuvent être remplacés conjointement par un groupe - C(O)NH-, -C(O)N(alkyle en C₁ à C₅), -S(O)₂NH- ou -S(O)₂N(alkyle en C₁ à C₅), et/ou
où quatre chaînons de carbone directement voisins de ces carbocycles en C₅ à C₇ peuvent être remplacés conjointement par un groupe - O-CH₂-CH₂-O, et/ou
où 1 à 3 atomes de carbone de ces cycles de 3 à 7 chaînons peuvent être substitués éventuellement indépendamment les uns des autres respectivement par un ou deux atomes de fluor ou un ou deux groupes alkyle en C₁ à C₅ ou un groupe hydroxy, alkyloxy en C₁ à C₅, alkylcarbonyloxy en C₁ à C₅, amino, alkylamino en C₁ à C₅, di-(alkyle en C₁ à C₅)-amine, cycloalkylène-imino en C₄ à C₇, alkylcarbonylamino en C₁ à C₅, cycloalkylcarbonylamino en C₃ à C₆, nitrile, carboxy-alkyle en C₁ à C₅, alkyloxycarbnyle en C₁ à C₅-alkyle en C₁ à C₅, carboxy-alkyloxycarbonyle en C₁ à C₅, aminocarbonyle, alkylaminocarbonyle en C₁ à C₅, di-(alkyle en C₁ à C₅)-aminocarbonyle ou cycloalkylène-iminocarbonyle en C₄ à C₇,
à condition que l'un de ces cycles formé conjointement de R⁴ et R⁵
dans lequel deux atomes d'azote ou un atome d'azote et un atome d'oxygène dans le cycle sont séparés par exactement un groupe -CH₂- éventuellement substitué, et/ou
dans lequel deux atomes dans le cycle forment une liaison -O-O- ou -S-O-
soit exclu, et
L2 désigne un groupe -C(O)-, et
M désigne un groupe phényle, pyridyle, thiényle ou furyle éventuellement substitué par R² et R³, dans lequel
R² représente un atome de fluor, de chlore, de brome ou d'iode ou un groupe méthyle, méthyle, vinyle, méthoxy, éthinyle, cyano ou -C(O)NH₂, et
R³ représente un atome d'hydrogène, de fluor, de chlore ou d'iode ou un groupe hydroxy, méthoxy, trifluorométhoxy ou un groupe alkyle en C₁ à C₃ éventuellement substitué par un atome de fluor, ou un groupe cyano, amino ou NH₂C(O),
où, sauf indication contraire, l'expression « hétéroaryle » mentionnée précédemment dans les définitions désigne un groupe hétéroaryle de 5 ou 6 chaînons monocyclique, où
le groupe hétéroaryle à 6 chaînons contient un, deux ou trois atomes d'azote, et
le groupe hétéroaryle à 5 chaînons contient un groupe imino éventuellement substitué par un groupe alkyle en C₁ à C₃, un atome d'oxygène ou un atome de soufre, ou
un groupe imino éventuellement substitué par un groupe alkyle en C₁ à C₃ ou un atome d'oxygène ou de soufre et en outre un ou deux atomes d'azote, ou
un groupe amino éventuellement substitué par un groupe alkyle en C₁ à C₃ et trois atomes d'azote,
et en outre, un groupe phényle substitué par un atome de fluor, de chlore ou de brome, un groupe alkyle en C₁ à C₃-hydroxy, alkyloxy en C₁ à C₃, amino, alkylamino en C₁ à C₃, di-(alkyle en C₁ à C₃)amino ou cycloalkylène-imino en C₃ à C₆ peut être condensé aux groupes hétéroaryle monocycliques mentionnées précédemment par deux atomes de carbone voisin,
et la liaison s'effectue par un atome d'azote ou par un atome de carbone de la partie hétérocyclique ou d'un cycle phényle condensé,
et où les groupes alkyle, alcényle, alcinyle et alkyloxy contenus dans les définitions mentionnées précédemment, qui présentent plus de deux atomes de carbone, sauf indication contraire, peuvent être à chaîne linéaire ou ramifiée et les groupes alkyle dans les radicaux dialkylés mentionnés précédemment, par exemple les groupes dialkylamino, peuvent être identiques ou différents,
et où les atomes d'hydrogène des groupes méthyle ou méthyle contenus dans les définitions mentionnées précédemment, sauf indication contraire, peuvent être remplacés en totalité ou en partie par des atomes de fluor,
leurs tautomères, leurs énantiomères, leurs diastéréomères, leurs mélanges et leurs sels.

2. Composés de formule générale (I) selon la revendication 1, dans lesquels D, E, G, J, L, L¹, L² et M sont tels que définis comme décrit dans la revendication 1 et dans lesquels
R⁴ représente un atome d'hydrogène ou
un groupe alkyle en C₁ à C₆ à chaîne linéaire ou ramifiée,
où les atomes d'hydrogène des fragments méthylène et/ou méthyle du groupe alkyle en C₁ à C₅ à chaîne linéaire ou ramifiée peuvent être remplacés éventuellement en totalité ou en partie par des atomes de fluor, et/ou
où les atomes d'hydrogène des fragments méthylène et/ou méthyle du groupe alkyle en C₁ à C₆ à chaîne linéaire ou ramifiée peuvent être substitués éventuellement respectivement, indépendamment les uns des autres par un substituants choisi parmi un groupe hydroxy,
et ou, sauf indication contraire, l'expression « halogénoatome » mentionnée précédemment dans la définition désigne un atome du groupe comprenant le fluor, le chlore, le brome et l'iode,
alkyloxy en C₁ à C₅, alkylaminocarbonyloxy en C₁ à C₅, di-(alkyle en C₁ à C₅)-aminocarbonyloxy, carboxy, alkyloxycarbonyle en C₁ à C₅, aminocarbonyle, alkylaminocarbonyle en C₁ à C₅, di-(alkyle en C₁ à C₅), aminocarbonyle, cycloalkylène-iminocarbonyle en C₄ à C₇, amino, alkylamino en C₁ à C₅, di-(alkyle en C₁ à C₅) -amino, alkylcarbonylamino en C₁ à C₅, alkyloxy en C₁ à C₃-alkylcarbonylamino en C₁ à C₂, alkyloxycarbonylamino en C₁ à C₃, alkylaminocarbonylamino en C₁ à C₃, alkylsulfonylamino en C₁ à C₅, N-(alkylsulfonyle en C₁ à C₅)-alkylamino en C₁ à C₅, cycloalkylcarbonylamino en C₃ à C₆, ou
un groupe nitrile, carboxy, aminocarbonyle, alkylaminocarbonyle en C₁ à C₅, cycloalkylaminocarbonyle en C₃ à C₆, di-(alkyle en C₁ à C₅)-aminocarbonyle, alkylcarbonyle en C₁ à C₅ ou un groupe cycloalkylène-iminocarbonyle en C₄ à C₇ dans lequel éventuellement un groupe méthylène peut être remplacé par un atome d'oxygène, de soufre ou d'azote substitué par un groupe alkyle en C₀ à C₃, et
où, dans la mesure où R⁴ est relié à G, peut représenter également un atome de fluor ou un groupe hydroxy, alkyle en C₁ à C₅-oxy, alcényle en C₂ à C₅-oxy, alcinyle en C₂ à C₅-oxy, cycloalkyle en C₃ à C₆-oxy, alkylaminocarbonyloxy en C₁ à C₅, di (alkyle en C₁ à C₅)aminocarbonyloxy ou cycloalkylène-iminocarbonyloxy en C₄ à C₇, phényl-alkyloxy en C₀ à C₂, amino, alkylamino en C₁ à C₅, di-(alkyle en C₁ à C₅)-amino, cycloalkylène-imino en C₄ à C₇, acylamino en C₁ à C₃, (acyle en C₁ à C₃)-alkylamino en C₁ à C₃, alkyloxycarbonylamino en C₁ à C₅, alkylaminocarbonylamino en C₁ à C₅, di(alkyle en C₁ à C₅)aminocarbonylamino ou cycloalkylène-iminocarbonylamino en C₄ à C₇,
où les groupes méthyle ou éthylène présents dans les radicaux alkyle ou cycloalkyle précédemment cités, peuvent être substitués respectivement indépendamment les uns des autres par un substituant choisi dans le groupe comprenant les groupes diméthylaminocarbonyle, alkyloxycarbonyle en C₁ à C₅, carboxy, méthyle, hydroxy, méthoxy ou amino,
à condition que deux hétéroatomes du groupe comprenant l'oxygène et l'azote séparés l'un de l'autre par exactement une groupe -CH₂- éventuellement substitué et/ou
que deux atomes formant une liaison -O-O- ou -S-O-, soient exclus, et
R⁵ représente un atome d'hydrogène, un groupe alkyle en C₁ à C₅, allyle, propargyle ou benzoyle ou, dans la mesure où R₅ est relié à G, peut également représenter un groupe hydroxy ou méthoxy, ou
R⁴ et R⁵, dans la mesure où ils sont liés au même atome de carbone, peuvent former conjointement avec l'atome de carbone, un groupe -C=O ou un groupe -CF₂, ou
R⁴ et R⁵, dans la mesure ou ils sont liés au même atome de carbone ou à deux atomes de carbone voisins, peuvent former conjointement avec le ou les atome (s) de carbone, un carbocycle de 3 à 7 chaînons,
où l'un des chaînons de carbone de ce cycle peut être remplacé par un atome d'oxygène ou de soufre ou un groupe -NH-, -N(alkyle en C₁ à C₅), N(alkylcarbonyle en C₁ à C₄) ou un groupe carbonyle, sulfinyle ou sulfonyle, et/ou
où deux chaînons de carbone directement voisins de ces carbocycles en C₄ à C₇ peuvent être remplacés conjointement par un groupe - C(O)NH-, -C(O)N(alkyle en C₁ à C₅), -S(O)₂NH- ou -S(O)₂N(alkyle en C₁ à C₅), et /ou
où quatre chaînons de carbone directement voisins de ces carbocycles en C₅ à C₇ peuvent être remplacés conjointement par un groupe - O-CH₂-CH₂-O,
à condition que l'un de ces cycles formé conjointement de R⁴ et R⁵
dans lequel deux atomes d'azote ou un atome d'azote et un atome d'oxygène dans le cycle sont séparés par exactement un groupe -CH₂- éventuellement substitué, et/ou
dans lequel deux atomes dans le cycle forment une liaison -O-O- ou -S-O-
soient exclus,
leurs tautomères, leurs énantiomères, leurs diastéréomères, leurs mélanges et leurs sels.

3. Composés de formule générale (I) selon la revendication 1 ou 2, dans lesquels E, G, J, L, L¹, L² et M, R⁴ et R⁵ sont tels que définis comme décrit dans la revendication 1 ou 2 et dans lesquels
D représente un système cyclique bicyclique substitué de formule (IIa) ou (IIb) 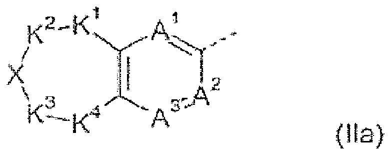 ou 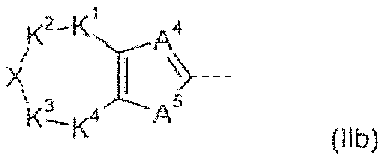 dans lequel
K¹ et K⁴ représentent respectivement, indépendamment les uns des autres, une liaison, un groupe -CH₂-, -CHR^{7a}-, -CR^{7b}R^{7c}- ou un groupe -C(O)- et où
R^{7a}/R^{7b}/R^{7c}
représentent respectivement indépendamment les uns des autres, un atome de fluor, un groupe hydroxy, alkyloxy en C₁ à C₅, où les deux radicaux P^{7b}/R^{7c} ne peuvent pas être liés en même temps par un hétéroatome à l'atome de carbone cyclique, sauf si - C(R^{7b}R^{7c})- correspond à un groupe -CF₂-, ou deux radicaux R^{7b}/R^{7c} pouvant former conjointement avec l'atome de carbone cyclique, un carbocycle de 3 chaînons,
à condition que K¹ et K⁴ désignant en même temps une liaison, soient exclus, et
K² et K³
désignant respectivement, indépendamment l'un de l'autre, un groupe -CH₂-, -CHR^{8a}-, CR^{8b}R^{8c}- ou un groupe -C(O)-, dans lequel
R^{8a}/R^{8b}/R^{8c}
représentent respectivement, indépendamment les uns des autres, un groupe alkyle en C₁ à C₅
et/ou
deux radicaux R^{6b}/^{p8c} peuvent former conjointement avec l'atome de carbone cyclique, un carbocycle saturé de 3 chaînons
et
au total, dans les formules (IIa) ou (IIb), au maximum quatre radicaux choisis parmi R^{7a}, R^{7b}, R^{7c}, R^{8a}, R^{8b} et R^{8c} peuvent être présents, et
X désigne un atome d'oxygène ou de soufre, un groupe CF₂ ou NR¹, dans lequel
R¹ désigne un atome d'hydrogène ou un groupe hydroxy, alkyloxy en C₁ à C₃, amino, alkylamino en C₁ à C₃, di-(alkyle en C₁ à C₃)-amino, un groupe alkyle en C₁ à C₅, alcényle en C₂ à C₅-CH₂-, alcinyle en C₂ à C₅-CH₂ ou un groupe cycloalkyle en C₃ à C₆,
et dans lequel
A¹ désigne soit N soit CR¹⁰,
A² désigne soit N soit CR¹¹,
A⁷ désigne soit N soit CR¹²,
A⁴ désigne soit N soit CR¹²,
A⁵ désigne NH, un atome de soufre ou d'oxygéne,
où R¹⁰, R¹¹ et R¹² désignent respectivement, indépendamment les uns des autres
un atome d'hydrogène, de fluor, de chlore, de brome ou d'iode ou un groupe alkyle en C₁ à C₅, CF₃, cyano, carboxy, alkyloxycarbonyle en C₁ à C₅, hydroxy, alkyloxy en C₁ à C₃-, CF₃O-, CHF₂O-, CH₂FO-, amino, alkylamino en C₁ à C₅, di-(alkyle en C₁ à C₅)-amino ou cycloalkylène-imino en C₄ à C₇,
leurs tautomères, leurs énantiomères, leurs diastéréomères, leurs mélanges et leurs sels.

4. Composés de formule générale (I) selon l'une des revendications 1 à 3, dans lesquels
X désigne un groupe NR¹, dans lequel
R¹ désigne un atome d'hydrogène ou un groupe alkyle en C₁ à C₅, allyle ou cyclopropyle, et
A¹ désigne CR¹⁰,
A² désigne CR¹¹,
A³ désigne CR¹²,
A⁴ désigne soit N soit CR¹²,
A⁵ désigne un atome de soufre,
où R¹⁰, R¹¹ et R¹² désignent respectivement, indépendamment les uns des autres
un atome d'hydrogène, de fluor ou de chlore, ou un groupe méthyle, CF₃-, hydroxy, méthoxy, CF₃O-, CHF₂O-, CH₂FO,
leurs tautomères, leurs énantiomères, leurs diastéréomères, leurs mélanges et leurs sels.

5. Composés de formule générale (I) selon l'une des revendications 1 à 4, dans lesquels
D représente un système cyclique bicyclique substitué de formule 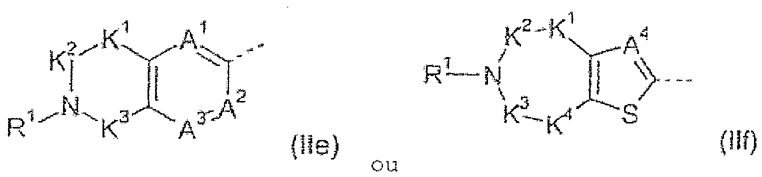 dans lequel
K¹ représente -CH₂-, -CHR^{7a} ou un groupe CR^{7b}R^{7c} et K² et K³
représentent Indépendamment l'un de l'autre, un groupe -CH₂-, -CHR^{8a} ou CR^{8b}R^{8c}, où
R^{8a}/R^{8b}/R^{8c}
représentent indépendamment les uns des autres, un groupe alkyle en C₁ à C₅, et
K⁴ représente une liaison, un groupe -CH₂-, - CHR^{7a} ou -CR^{7b}R^{7c},
où
R^{7a} représente un groupe alkyle en C₁ à C₅ et R^{7b}/R^{7c} représentent indépendamment l'un de
l'autre, un groupe hydroxy, alkyloxy en C₁ à C₅ ou alkyle en C₁ à C₅,
où les deux radicaux R^{7b}/R^{7c} ne peuvent pas être liés en même temps par un atome d'oxygène à l'atome de carbone cyclique, et
au total dans les formules (IIe) ou (IIf) au maximum quatre radicaux choisis parmi R^{7a}, R^{7b}, R^{7c}, R^{8a}, R^{ab} et R^{8c} peuvent être présents, et
R¹ représente un atome d'hydrogène ou un groupe alkyle en C₃ à C₃, allyl ou cyclopropyle, et dans lesquels
A¹ désigne CR¹⁰,
A² désigne CR¹¹,
A³ désigne CR¹²,
A⁴ désigne soit N soit CR¹²,
où R¹⁰, R¹¹ et R¹² désignent respectivement, indépendamment les uns des autres
un atome d'hydrogène, de fluor ou de chlore ou un groupe méthyle, CF₃-, hydroxy, méthoxy, CF₃O-, CHF₂O-, CH₂FO et
-L-E-G-J- représente un groupe -C-C-C-C- qui peut être substitué par R⁴ et R⁵, et
R⁴ représente un atome d'hydrogène ou
un groupe alkyle en C¹ à C³ à chaîne linéaire ou ramifiée,
où les atomes d'hydrogène des fragments méthylène et/ou méthyle du groupe alkyle en C¹ à C⁶ à chaîne linéaire ou ramifiée peuvent être remplacés éventuellement, indépendamment les uns des autres par un substituant choisi parmi un groupe hydroxy, alkyloxy en C¹ à C⁵, alkylaminocarbonyloxy en C¹ à C⁵, di-(alkyle en C¹ à C⁵), aminocarbonyloxy, carboxy, alkyloxycarbonyle en C¹ à C⁵, alkyloxy en C¹ à C³-alkylcarbonylamino en C¹ à C², alkyloxycarbonylamino en C¹ à C³, alkylaminocarbonylamino en C¹ à C³, alkylcarbonylamino en C¹ à C⁵, alkyle en C¹ à C⁵, sulfonylamino, ou
dans la mesure où dans la mesure où R⁴ est relié à G, peut représenter également un atome de fluor ou un groupe hydroxy, méthoxy, alcényle en C₃ à C₅-oxy, alkyle en C₂ à C₅-oxy, cycloalkyle en C₃ à C₆-oxy, benzyloxy, alkylaminocarbonyloxy en C₁ à C₅, di(alkyle en C₁ à C₅)aminocarbonyloxy ou cycloalkylène-iminocarbonyloxy en C₄ à C₇,
à condition que
deux hétéroatomes du groupe comprenant l'oxygène et l'azote séparés l'un de l'autre par exactement un groupe -CH₂- éventuellement substitué
soit exclu, en
R⁵ représente un atome d'hydrogène ou un groupe alkyle en C₁ à C₅, allyle, benzyle ou phényle ou, dans la mesure où R₅ est relié à G, peut également représenter un groupe hydroxy, ou methoxy, ou
R⁴ et R⁵, dans la mesure où ils sont liés au même atome de carbone, peuvent former conjointement avec l'atome de carbone, un groupe -C=O ou un groupe -CF₂, ou
R⁴ et R⁵, dans la mesure où ils sont liés au même atome de carbone ou à deux atomes de carbone voisins, peuvent former conjointement avec le ou les atome(s) de carbone, un carbocycle de 3 à 6 chaînons,
où quatre chaînons de carbone directement voisins de ces carbocycles en C₅ à C₆ peuvent être remplacés conjointement par un groupe -O-CH₂-CH₂-O,
leurs tautomères, leurs énantiomères, leurs diastéréomères, leurs mélanges et leurs sels.

6. Composés de formule générale (I) selon l'une des revendications 1 à 5, dans lesquels
D représente un système cyclique bicyclique substitué de formule 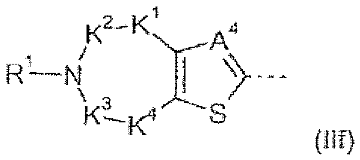 dans lequel
K¹ représente -CH₂-, -CHR^{7a} ou un groupe CR^{7b}R^{7c} et
K² et K³
représentent indépendamment l'un de l'autre, un groupe -CH₂-, -CHR^{8a} ou CR^{8b}R^{8c}, où
R^{8a}/R^{8b}/R^{8e}
représentent indépendamment les uns des autres, un groupe alkyle en C₁ à C₅, et
K4 représente une liaison, un groupe -CH₂-, - CHR^{7a} ou -CR^{7b}R^{7c},
où
R^{7a} représente un groupe alkyle en C₁ à C₅ et R^{7b}/R^{7c} représentent indépendamment l'un de l'autre, un groupe hydroxy, alkyloxy en C₁ à C₅ ou alkyle en C₁ à C₅,
où les deux radicaux R^{7b}/R^{7c} ne peuvent pas être liés en même temps par un atome d'oxygène à l'atome de carbone cyclique,
et
au total dans la formule (IIf), au maximum quatre radicaux choisis parmi R^{7a}, R^{7b}, R^{7c}, R^{8a}, R^{8b} et R^{8c} peuvent être présents, et
R¹ représente un atome d'hydrogène ou un groupe alkyle en C₁ à C₃ ou cyclopropyle, et dans lesquels
A¹ désigne CR¹⁰,
A² désigne CR¹¹,
A³ désigne CR¹²,
A⁴ désigne soit N soit CR¹²,
où R¹⁰, R¹¹ et R¹² désignant respectivement, indépendamment les uns des autres
un atome d'hydrogène, de fluor ou de chlore ou un groupe méthyle, CF₃-, hydroxy, méthoxy, CF₃O-, CHF₂O-, CH₂FO,
leurs tautomères, leurs énantiomères, leurs diastéréomères, leurs mélanges et leurs sels.

7. Composés de formule générale (I) selon l'une des revendications 1 à 6, dans lesquels
M représente un cycle thiophén-2-yle de formule 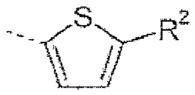 dans lesquels
R² représente un atome de chlore, de brome ou un groupe éthinyle,
leurs tautomères, leurs énantiomères, leurs diastéréomères, leurs mélanges et leurs sels.

8. Sels physiologiquement acceptables des composés selon l'une des revendications 1 à 7.

9. Médicament contenant un composé selon au moins l'une des revendications 1 à 7, ou un sel physiologiquement acceptable selon la revendication 8, parallèlement à éventuellement un ou plusieurs véhicules inertes et/ou diluants.

10. Utilisation d'un composé selon au moins l'une des revendications 1 à 7, ou un sel physiologiquement acceptable selon la revendication 8, pour la production d'un médicament pour la prévention et le traitement des maladies thrombotiques veineuses et artérielles.

11. Procédé de production d'un médicament selon la revendication 9, **caractérisé en ce que**, de façon non chimique un composé selon au moins l'une des revendications 1 à 7 ou un sel physiologiquement acceptable selon la revendication 8, est incorporé dans un ou plusieurs véhicule inertes et/ou diluants .
